(19) Europäisches Patentamt
European Patent Office
Office européen des brevets

(11) Publication number: 0 307 513 A2

(12) EUROPEAN PATENT APPLICATION

(21) Application number: 87117967.7

(22) Date of filing: 04.12.87

(51) Int. Cl.4: C07K 7/06 , C07K 7/08 , C07K 7/10 , C07K 13/00 , C12N 15/00 , C12P 21/02 , A61K 37/02 , G01N 33/53 , A61K 39/395 , //A61K35/42

(30) Priority: 08.12.86 US 939206
10.06.87 US 60719
01.10.87 US 101680

(43) Date of publication of application:
22.03.89 Bulletin 89/12

(84) Designated Contracting States:
ES GR

(71) Applicant: ABBOTT LABORATORIES
14th Street and Sheridan Road North St
North Chicago Illinois 60064(US)

(72) Inventor: Fox, Lawrence J.,
634, Rockland Avenue,
Lake Bluff, Illinois 60044,(US)
Inventor: Pilot-Matias, Tami J.,
205, Appley Avenue,
Libertyville, Illinois 60048,(US)
Inventor: Meuth, Joseph L.,
310, Charlotte Mundelein,
Illinois 60060,(US)
Inventor: Sarin, Virender K.,
516, Fairlawn Avenue,
Libertyville, Illinois 60048,(US)
Inventor: Whitsett, Jeffrey A.,
5565 Salem Road,
Cincinnati, Ohio 45229,(US)

(74) Representative: Brown, John David et al
FORRESTER & BOEHMERT
Widenmayerstrasse 4/I
D-8000 München 22(DE)

# (54) Pulmonary hydrophobic surfactant-associated proteins.

(57) Novel, isolated, substantially pure, hydrophobic surfactant-associated proteins of 4,000-7,000 dalton simple molecular weight, SAP(Val) and SAP(Phe); multimers thereof, and substitution, addition and deletion analogs and fragments thereof are disclosed. When SAP(Val) and/or SAP(Phe) is combined with lipids, its surfactant-like property imparts to the combination significant pulmonary biophysical activity. Such a combination results in enhanced adsorption of the lipids with properties similar to that of natural pulmonary surfactant material. SAP-(Val) and/or SAP(Phe) in combination with lipids is highly useful for replacing or supplementing natural pulmonary surfactant material for reducing or maintaining normal surface tension in the lungs, especially in the lungs of patients suffering from hyaline membrane disease, HMD, or other syndromes associated with the lack or insufficient amounts of natural pulmonary surfactant material. A combination of SAP(Val) and/or SAP(Phe) and lipids may be administered as an aerosol spray or in aqueous normal saline with or without calcium chloride for treating or preventing HMD and other surfactant deficiency states. Also disclosed are methods of isolating the SAP(Val) from animal tissue and methods for making SAP(Val) and SAP(Phe) by recombinant DNA techniques

EP 0 307 513 A2

and direct peptide synthesis; genomic clones of SAP(Val) and SAP(Phe); expression systems and products for SAP(Val) DNA; and polyclonal and monoclonal antibodies to SAP(Val) and SAP(Phe).

# PULMONARY HYDROPHOBIC SURFACTANT-ASSOCIATED PROTEINS

## Background

The present application relates to pulmonary hydrophobic surfaction associated proteins ("SAPs") and methods for the isolation and use thereof. In particular, the present application relates to SAP(Val), methods for isolation thereof and methods for the use thereof.

Hyaline membrane disease ("HMD") is a common disorder of premature infants and is related to diffuse atelectesis, hypoxia and resultant respiratory impairment. More particularly, HMD relates to the lack of vital pulmonary materials necessary for reducing surface tension in the airways of the alveoli. As a result, the alveoli or terminal respiratory sacs of patients suffering from HMD normally collapse. And, because the surface tension at the gas-liquid interface in HMD patients is elevated, their alveoli or terminal respiratory sacs are very difficult to reinflate. Consequently, HMD may be associated with significant morbidity and mortality, especially in premature infants.

Present treatments for HMD employ high concentrations of oxygen, positive pressure and/or mechanical ventilation to maintain adequate oxygenation. Such treatments are complicated by oxygen- and pressure-related injuries as well as by injuries resulting from the need to mechanically access the airway via endotracheal tubes.

Another approch to treatment of HMD and other syndromes associated with a lack of pulmonary surfactant material involves the use of replacement pulmonary surfactant material. Other syndromes associated with a lack of pulmonary surfactant material include adult respiratory distress syndrome ("ARDS") caused by, for example, trauma, sepsis, smoke inhalation and myocardial infarction. In addition, syndromes associated with a lack of pulmonary surfactant material include any chronic obstructive pulmonary diseases, pneumonia or other conditions resulting in damage to pulmonary type II cells.

Therapy for syndromes associated with a lack of pulmonary surfactant material may include the use of aerosolized or liquid synthetic phospholipid mixtures, natural pulmonary surfactant material and various preparations of surfactant material prepared from animal lung. The surface tension lowering ability of naturally derived preparations is in general better than that of synthetic lipid preparations. Modified bovine surfactants are also proposed for use in such preparations. However, problems with human and animal pulmonary preparations include batch-to-batch variability and possible infection and immunologic risks.

When treating patients for HMD, it is important to employ only the required active substances in order to minimize possible adverse immunologic consequences of therapy. Unfortunately, because the available natural pulmonary surfactant material and preparations are in crude form, they are less specific and are possibly associated with greater immunologic risks.

Natural pulmonary surfactant is a complex material composed primarily of phospholipids and surfactant-associated proteins or apolipoproteins. The phospholipids, mainly phosphatidycholine ("PC"), disaturated phosphatidycholine ("DSPC") and phosphatidylglycerol ("PG"), are of paramount importance for the physiological role of natural pulmonary surfactant material in reducing surface tension in the alveoli. Phospholipids, of which DSPC is the principal component, are synthesized in the endoplasmic reticulum of Type II epithelial cells, packaged into lamellar bodies, then secreted into the alveolar space by an exocytotic process. It is believed that several of the phospholipids are not catabolized and resynthesized, but that they are reutilized, primarily as intact molecules, and that they constitute the major components of the natural pulmonary surfactant material.

With respect to the surfactant-associated proteins or apolipoproteins, there is considerable disagreement as to their identity and utility. Nonetheless, there is an increasing agreement that, in addition to the lung surfactant phospholipids, at least some apolipoproteins are vital for the full biological activity of the natural pulmonary surfactant material in reducing surface tension in the alveoli.

Surfactant-associated proteins or apolipoproteins include both serum- and lung-specific proteins. The major lung specific surfactant-associated protein of $M_r = 30\text{-}40{,}000$ daltons is identified in lung surfactant by King et al., Am. J. Physiol., 244, 788-795 (1973), is a glycoprotein which is rich in glycine and which contains a collagen-like region rich in hydroxyproline. This protein, herein called SAP-35, is synthesized from $M_r = 28\text{-}30{,}000$ dalton translation products which undergo glycosylation, hydroxylation of proline residues and sulfhydryl-dependent cross-linking to form large oligomers which may be detected in the airway. Proteolytic fragments of SAP-35 are found in protein preparations isolated from lavage of patients with alveolar proteinosis and from other mammalian surfactants migrating as proteins of small molecular weight [Whitsett et al., Pediatr. Res., 19, 501-508 (1985)]. While the glycoprotein SAP-35 binds phospholipids and may confer the structural organization of tubular myelin to surfactant lipids, SAP-35 may not be required for the biophysical activity of surfactants. See King et al., J. Appl. Physiol., 42, 483-491 (1977).

Smaller lung specific surfactant-associated proteins are also identified from a variety of mammalian surfactants. A 10,000-12,000 dalton protein may be present in pulmonary surfactant material [King et al., Am. J. Physiol, 223, 715-726 (1972)]. This protein is now believed to be a fragment of the major glycoprotein SAP-35. Smaller surfactant-associated proteins may be present in alveolar lavage material from a number of species and may have molecular weights of approximately 10,000 daltons in the dog and the rabbit, 10,500-14,000 daltons in the rat, 11,500-16,500 daltons in the pig, and 10,000 daltons in the cow.

The. nature of and the relationships among these various smaller surfactant-associated proteins and the larger protein, SAP-35 or its fragments, have not been established. Nevertheless, Suzuki et al., J. Lipid. Res., 23, 53-61 (1982), suggests that a small 15,000 dalton protein in pig alveolar lavage may have a greater affinity for lipid than SAP-35. However, Suzuki et al. does not distinguish this protein from SAP-35 or its fragments and does not demonstrate surfactant properties in a purified state. Rather, Suzuki et al. only suggests that this 15,000 daltons protein is possibly a physiological regulator for the clearance of alveolar phospholipid. A small SAP protein is reported to be isolated from rat alveolar lavage by isolation methods involving ether/ethanol treatment, chloroform/methanol extraction, and chloroform/methanol elution from a silicic acid column, and is reported to increase the uptake of liposome by cultured Type II epithelial cells [Claypool et al., J. Clin. Invest., 74, 677-684 (1984)].

Wang et al., Fed. Proc., 44, 1024 (Abstract) (1985), describes two distinct small molecular weight proteins in rat lung surfactant one of which is soluble in ethanol, the other of which is soluble in ether/ethanol. However, these proteins are not reported as purified or characterized, and surfactant-like activity is not identified. Wang et al. indicate that these small molecular weight proteins may be involved with surfactant recycling. The smaller molecular weight proteins, such as those discussed above, may arise as proteolytic fragments of the larger SAP-35 molecule. However, it is unclear whether SAP-35, one or more of the smaller proteins, or all proteins together are active components imparting biophysical activity to natural mammalian pulmonary surfactant.

Tanaka et al., Chem. Pharm. Bull., 31, 4100-4109 (1983) describes a 10,000 molecular weight protein isolated by chloroform extraction of a fraction of minced bovine lung.

Fujiwara et al., Biochem., Biophys. Res. Comm., 135, 527-532 (1986), reports the isolation of a 5,000 dalton molecular weight proteolipid which, based on biophysical testing, may have surface activity when mixed with phospholipids. However, this protein is not well-characterized in Fujiwara et al. The reported amino acid composition of a hydrophobic, small molecular weight protein of $M_r = 5{,}000$ isolated from bovine surfactant extracts appears to be similar to, but is distinguished by a paucity of valine residues in a reported amino acid composition from, a surfactant-associated protein according to the present invention, SAP(Val), the amino acid sequence of which is identifiable by a high number of valine residues. [Takahashi et al., Biochem. J., 236, 85-89 (1986)]. A small molecular weight protein with composition consistent with the protein previously termed "surfactant apolipoprotein B" [King et al., Am. J. Physiol., 224, 788-795 (1973)] is identifiable as a C-terminal domain of SAP-35 [Ross et al., J. Biol. Chem., 261, 14283-14291 (1986)]. Mixtures of phospholipids and a surfactant-associated protein of 35,000 daltons, SAP-35, or its fragments are reported to have relatively weak surface active properties compared to the surfactant proteolipids $M_r = 6{,}000\text{-}14{,}000$ [Whitsett et al., Pediatr. Res., 20, 460-467 (1986); and Ross et al., J. Biol. Chem., 261, 14283-14291 (1986)].

In Schilling et al., PCT Application No. WO86/03408, a human 35,000 dalton protein and nonhuman 10,000 dalton proteins are described. Schilling et al. indicates that both classes of proteins may be needed to confer full biophysical activity upon surfactant phospholipids. Schilling et al. also discloses a complete DNA sequence encoding the 35,000 dalton protein, $NH_2$-terminal amino acid sequences for the 10,000 dalton nonhuman proteins and a preliminary (81 bp) partial sequence for a human cDNA clone for the 10,000 dalton protein. The 10,000 dalton canine and 81 bp human sequences have substantial regions indentical in sequence to the sequence of human SAP(Phe) according to the present invention.

In Taeusch, PCT Patent Publication No. WO 87/02037, two separate proteins are characterized by molecular weights of about 35 kd and two separate proteins are characterized by molecular weights of about 5.5-9 kd. The two approximately 6 kd proteins differ significantly from each other with respect to amino acid composition, as well as from the protein described in Tanaka, Chem. Pharm. Bull., 311, 4100 (1983). Additionally, the N-terminal peptide sequence of the cold butanol-insoluble 6 kd protein is disclosed as well for a cDNA and deduced amino acid sequence for human SAP(Phe).

Monoclonal antibodies raised against alveolar surfactant protein are disclosed in Lewicki, U.S. Patent No. 4,562,003.

Hydrophobic small molecular weight proteins soluble in organic solvents are detectable in a variety of mammalian surfactants [Phizackerly et al., Biochem. J., 183, 731-736 (1979); Tanaka et al., Chem. Pharm. Bull., 31, 4100-4109 (1983); Suzuki, J. Lipid Res., 23, 62-69 (1982); Claypool et al., J. Clin. Invest., 74, 677-684 (1984); Yu et al., Biochem. J., 236, 85-89 (1986); Takahashi et al., Biochem. Biophys. Res. Commum., 135 527-532 (1986); Whitsett et al., Pediatr. Res., 20, 460-467 (1986); and Whitsett et al., Pediatr Res., 20, 744-749 (1986)]. SAPs, identifiable as proteins of $M_r$ 6,000-14,000 are identified in the etherethanol extracts of surfactant preparations used clinically for treatment of hyaline membrane disease [Whitsett et al., Pediatr. Res., 20, 460-467 (1986); Whitsett et al., Pediatr Res., 20, 744-749 (1986); Fujiwara et al., Lancet, 1, 55-59 (1980); Enhorning et al., Pediatrics, 76, 145-153 (1985); and Kwong et al., Pediatrics, 76, 585-592 (1985)]. cDNAs encoding one of these proteins, human surfactant proteolipid with amino terminus of phenylalanine, designated herein as "SAP(Phe)," an $M_r = 7,500$ peptide derived from an $M_r = 40,000$ precursor, are disclosed in Glasser et al., Proc. Nat'l Acad. Sci. (USA), 84, 4007-4011 (1987) and Jacobs et al., J. Biol. Chem., 262, 9808-9811 (1987). cDNA encoding a protein homologous to SAP(Phe), SP-18, is reported to be isolated from canine lung [Hawgood et al., Proc. Nat'l Acad. Sci. (USA), 84, 166-170 (1987)]. Reconstitution of small molecular weight weight surfactant proteins with synthetic phospholipids imparts virtually complete surfactant-like properties to the mixture, including rapid surface absorption and decreased surface tension during dynamic compression [Yu et al., Biochem. J., 236, 85-89 (1986); and Takahashi et al., Biochem. Biophys. Res. Commum., 135, 527-532 (1986)].

## Summary of the Invention

SAP(Val) and SAP(Phe) according to the present invention are isolated, substantially pure, hydrophobic surfactant-associated proteins of 4,000-7,000 dalton simple molecular weight which, when isolated from animal tissue, comprise at least two hydrophobic proteins of molecular weights of 4,000-7,000 daltons each and may also comprise larger multimers thereof. It may be noted that SAP(Val) is also referred to in publications as "SAP-6(Val)," as SPL(pVAL)" and as "SP-C." Similarly, SAP(Phe) is referred to in publications as "SAP-6(Phe)," "SPL-(Phe)" and "SP-B."

Hereinafter, discussions related to SAP(Val) and SAP(Phe) are intended to apply to isolates; recombinant form; multimers; chemically or enzymatically synthesized forms; deletion, substitution or addition analogs; and analogs based on a hydropathy profile or on secondary and tertiary structure.

SAP(Val) and SAP(Phe), when combined with lipids, have significant pulmonary biophysical surfactant activity that may be utilized to effectively treat and prevent HMD and other syndromes associated with lack or insufficient amounts of natural pulmonary surfactant material. Although it is presently believed that SAP-(Val) and SAP(Phe) are lung-specific, their pulmonary biophysical surfactant activity is believed not to be species-specific. Therefore, SAP(Val) and SAP(Phe) may be purified from animal tissue, specifically pulmonary tissue or amniotic fluid, extracted from a variety of animals, such as dog, cow, human, pig, rabbit, rat and the like, or made by recombinant DNA methods or direct peptide synthesis. The concentration of SAP(Val) and SAP(Phe) in pulmonary tissue and lavage is probably greater than that found in aminotic fluid. With respect to other animal tissues or fluid, however, SAP(Val) and SAP(Phe) are believed to be present in substantially smaller or undetectable concentrations or completely absent.

It is believed that when SAP(Val) and SAP(Phe) are combined with lipids, they enhance the surfactant properties of the lipids imparting to the combination significant pulmonary biophysical surfactant activity. As a result of this remarkable property, such a combination is highly useful for replacing or supplementing natural pulmonary surfactant material and for reducing or maintaining normal surface tension in the lungs, especially in the lungs of patients suffering from HMD and other syndromes associated with the lack or insufficient amounts of natural pulmonary surfactant material. Because SAP(Val) and SAP(Phe) may be highly purified from animal tissue, or made by recombinant DNA techniques or by direct peptide synthesis, the immunologic risks currently associated with the less pure preparations available heretofore for treating or preventing HMD or related syndromes are substantially reduced.

It is to be understood that the terms "hydrophobic surfactant-associated protein" and "SAP(Val) or SAP(Phe)" are used interchangeably herein, and that whenever referenced herein, they are meant to include any small hydrophobic surfactant-associated proteins having surfactant-like activity, having simple molecular weights of about 4,000-7,000 daltons determined in polyacrylamide gels containing sodium dodecyl sulfate and having substantial resistance to protease enzymes, endoglycosidase F and collagenase. These terms are also meant to include any such proteins or polypeptides having surfactant-like activity which are made by recombinant DNA methods or direct peptide synthesis comprising the amino acid sequences of such hydrophobic surfactant-associated proteins or translations of DNA sequences or portions thereof encoding such proteins, or deletion, substitution, or addition analogs of such proteins.

It is also to be understood that the term "hybridization" as used herein is meant to encompass conditions within a range of functional equivalents to and generally according to the conditions set forth in Examples 6, 7, 11, 12, 15 and 16.

The present invention further resides in a method of separating SAP(Val) from animal tissue which involves separating the animal tissue into a particulate fraction and a liquid fraction, and extracting SAP(Val) in a substantially pure state from the liquid fraction. The methods substantially reduces risks currently associated with the less pure preparations available heretofore for treating or preventing HMD or related syndromes.

The methods of this invention are further concerned with separating SAP(Val) from the larger, novel hydrophobic multimers thereof by, for instance, gel electrophoresis or other suitable techniques.

Also described are methods for isolating genes encoding SAP(Val) and SAP(Phe), the characterization of these genes, and methods for making SAP(Val) by recombinant DNA methods and direct peptide synthesis.

In accordance with the present invention, SAP(Val) and SAP(Phe) may also be made by recombinant DNA methods. The following are examples of such methods. In summary, human lung poly(A)$^+$ RNA was used as a template to synthesize complementary DNA (cDNA) copies of which were subsequently cloned into a lambda vector in E.coli. cDNA copies of the genes encoding SAP(Val) and SAP(Phe) were identified, and then characterized by DNA sequence analysis. These DNA sequences allowed for further characterization of SAP(Val) and SAP(Phe) by comparison with the amino acid sequences for both SAP(Val) and SAP-(Phe). In addition, the cDNA encoding SAP(Phe) was expressed as a fusion product with the E.coli $\beta$-galactosidase (lacZ) gene, and the resulting expressed gene product has been shown to be antigenic by reacting with polyclonal antisera specific for SAP(Phe).

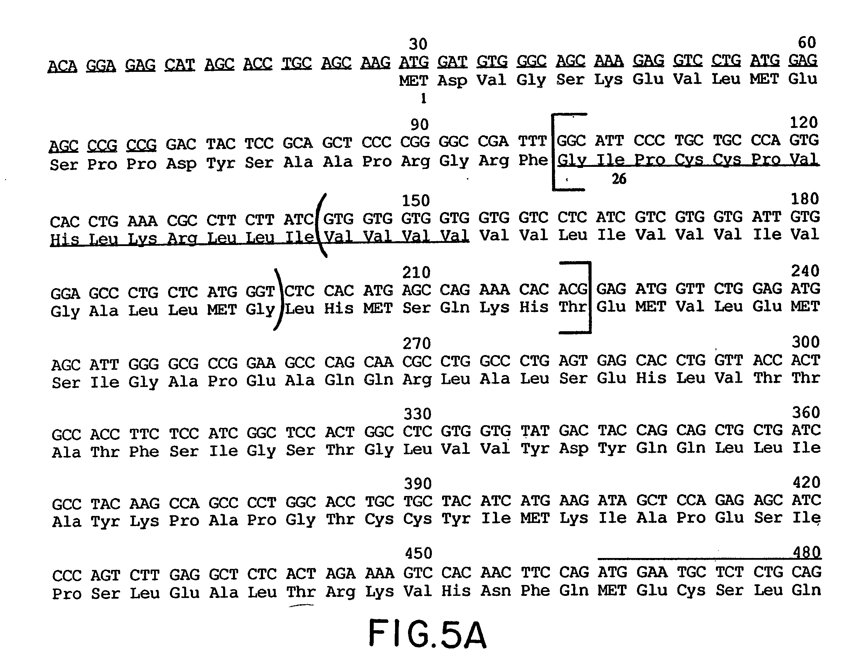

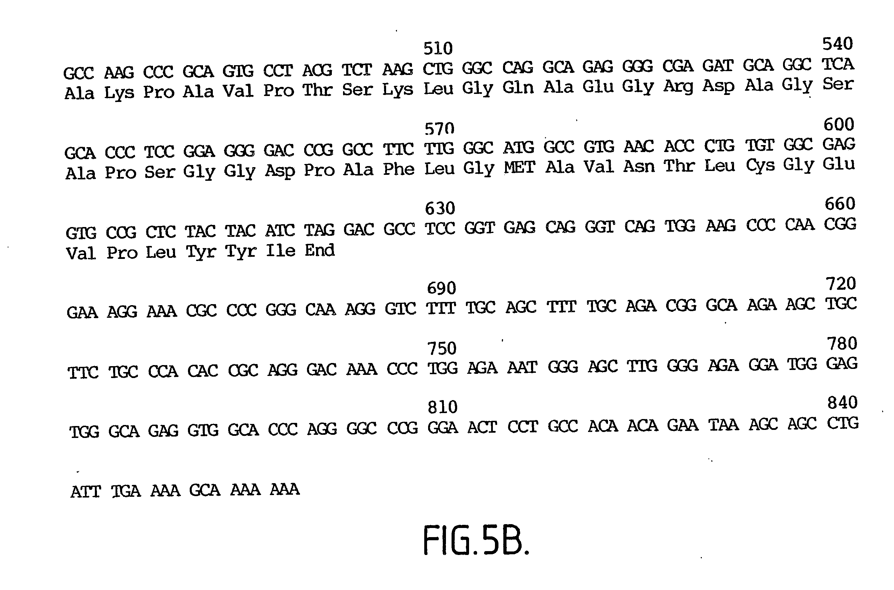

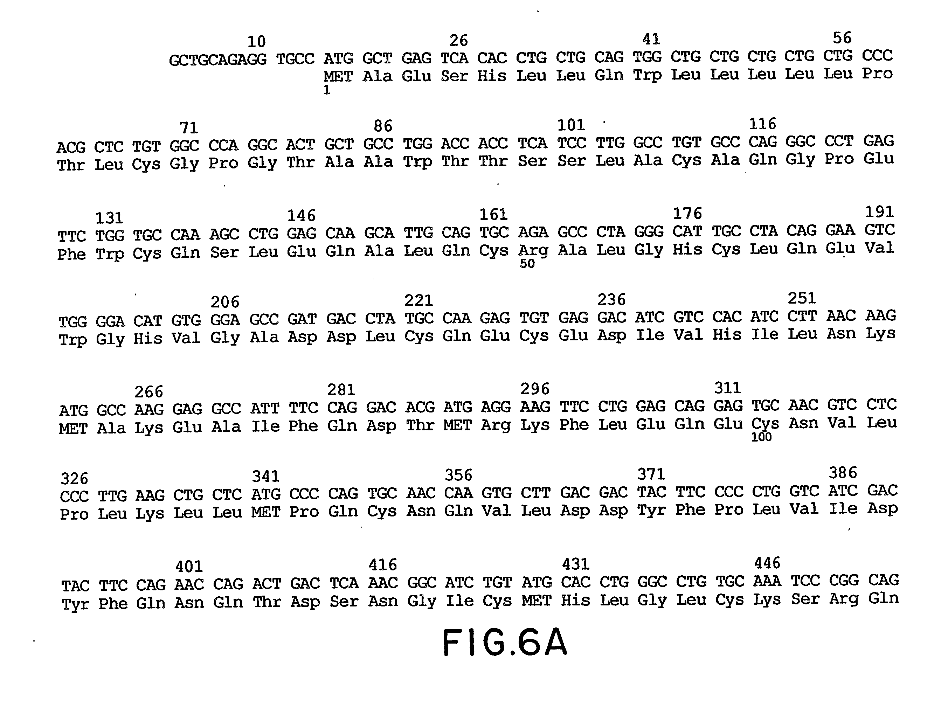

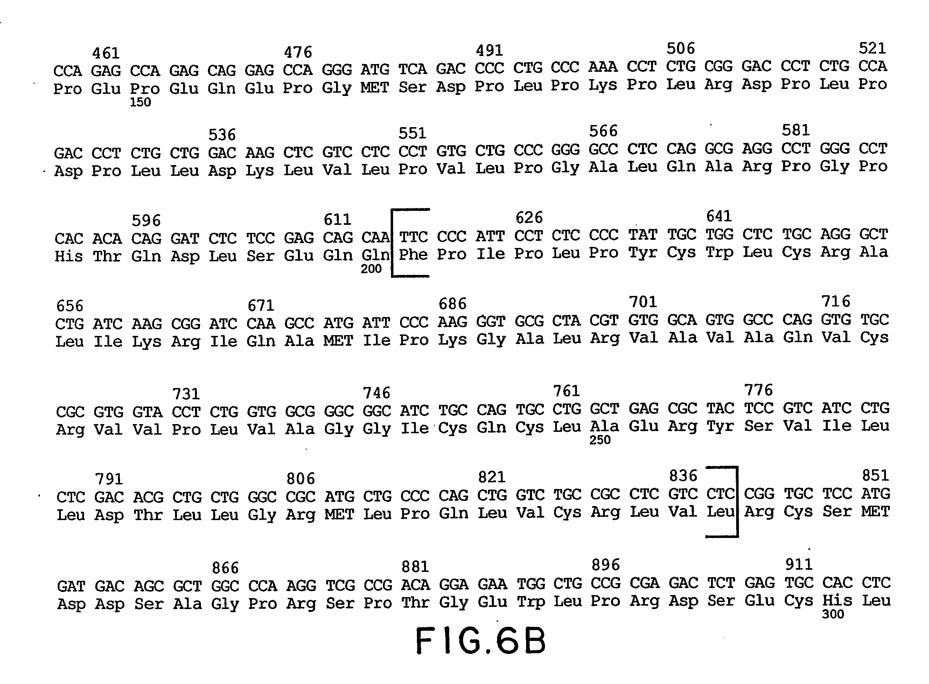

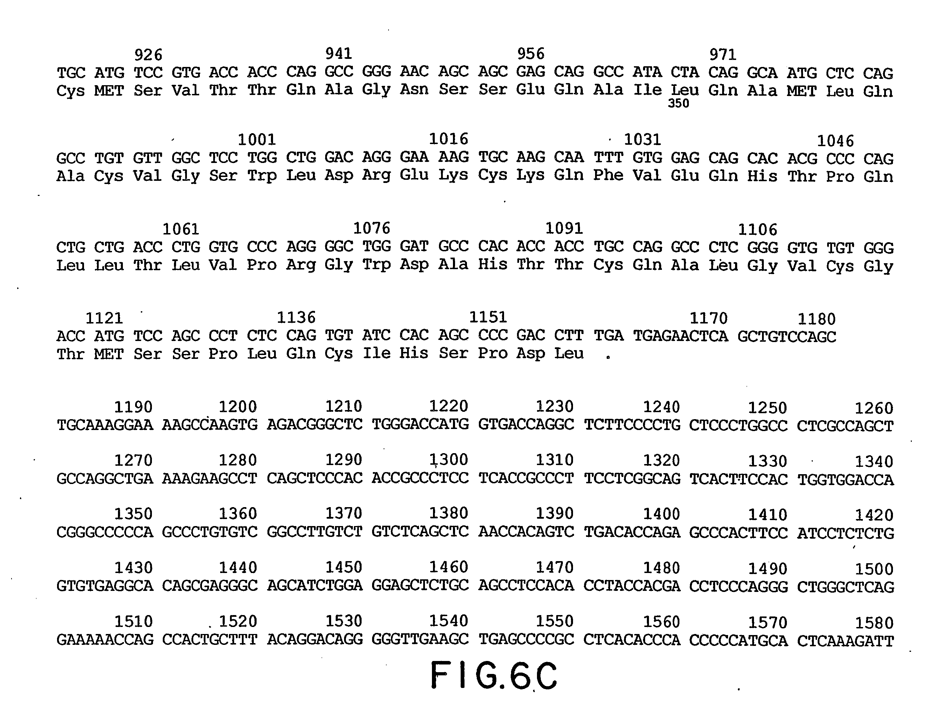

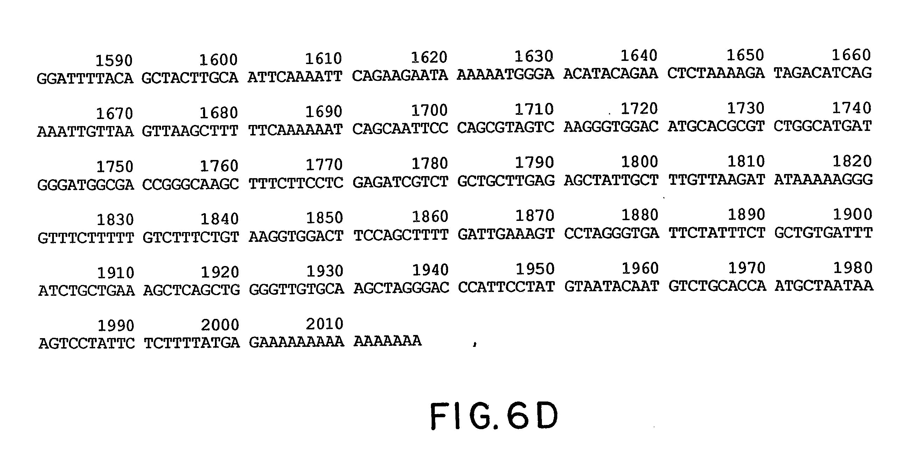

Polypeptides may be made by chemical or enzymatic synthesis based on the sequences given herein including the $NH_2$-terminal amino acid sequences given in Tables 2 and 3 and the translation of DNA sequences given in FIGS. 5 and 6. These chemically synthesized polypeptides include replicas of the amino acid sequences of SAP(Val) or SAP(Phe) or translations of DNA sequences encoding SAP(Val) or SAP(Phe) or portions of these sequences, as well as addition, deletion and substitution analogs of such replicas. The synthetic polypeptides may then be combined with lipids for surfactant preparations. Although these synthetic polypeptides may have a molecular weight less than SAP(Val) or SAP(Phe) isolated from animal tissue, it is to be understood that the terms "SAP(Val)" and "SAP(Phe)," as used herein, encompasses these synthetic peptides.

In particular fragments of synthetic peptides having less than all, more particularly less than 75% and especially less than 50% of the coding region of SAP(Phe) or SAP(Val) are provided according according to the present inventor.

The present invention also includes novel medicaments, preparations, and methods employed to treat animals, including humans, suffering from HMD and other syndromes related to the lack or insufficient amounts of natural pulmonary surfactant material. Antibodies and antisera according to the present invention may be made which are directed against SAP(Val) or SAP(Phe).

## Brief Description Of The Figures

FIG. 1 is an illustration of an SDS-PAGE preparation of SAP(Val) isolated from bovine lung;

FIG. 2 is an illustration of an SDS-PAGE preparation of SAP-6 from human, canine and bovine lung.

FIG. 3 is a nucleotide sequence and deduced amino acid translation of a partial cDNA clone encoding SAP(Phe);

FIG. 4 is a schematic depiction of the structure of SAP(Val) cDNA clones according to the present invention;

FIG. 5 is a nucleotide sequence and deduced amino acid translation of complete SAP(Val) cDNA;

FIG. 6 is a nucleotide sequence and deduced amino acid translation of complete SAP(Phe) cDNA;

FIG. 7 is a flow chart of the construction of a lacZ/SAP-6-Phe fusion product;

FIG. 8A is an illustration of the organization of the SAP(Phe) gene and FIG. 8B is a nucleotide sequence of a genomic clone encoding of SAP(Phe);

FIG. 9 depicts restriction maps and illustrates the organization of two SAP(Val) genes;

FIG. 10 is a nucleotide sequence of a genomic clone encoding SAP(Val);

FIG. 11 is an illustration of a Northern blot of SAP(Val);

FIG. 12 is a depiction of results of a hybrid arrested translation of SAP(Val);

FIG. 13 is an illustration of an SDS-PAGE of CKS and CKS/SAP(Val) fusion protein;

FIG. 14 is an illustration of a SDS-PAGE of CKS/SAP(Val) fusion protein after initial purification;

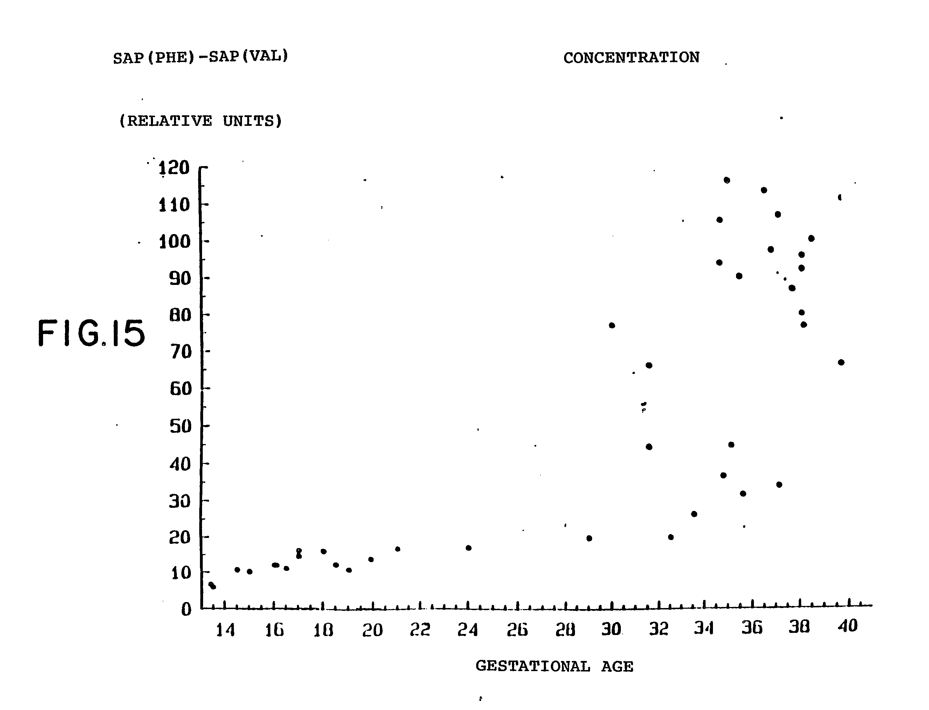

FIG. 15 is a graph of SAP(Phe) concentration versus gestational age obtained from a competitive ELISA assay using polyclonal antibodies directed against SAP(Phe) of human amniotic fluid samples for predicting pulmonary maturation; and

FIG. 16 is a hydropathy plot for the amino acid sequence of SAP(Val) precursor protein.

## Detailed Description

The following detailed description discloses SAP(Val) isolated from animal tissues, specifically pulmonary tissue and amniotic fluid, or made by recombinant DNA methods or by direct peptide synthesis, as well as novel medicaments based on SAP(Val), novel antibodies and antisera and methods of preparation and utilization thereof.

At least two small hydrophobic surfactant-associated proteins with simple molecular weights of about 4,000-7,000 daltons each (hereinafter referred to as "SAP(Val) or SAP(Phe) monomers"), are isolated from animal tissue. SAP(Val) and SAP(Phe) may also occur as larger, hydrophobic protein multimers which may be covalent or noncovalent aggregates of SAP(Val) and/or SAP(Phe) monomers having molecular weights of about 14,000, about 20,000 and about 26,000 daltons based on size estimation using sodium dodecyl sulfatepolyacrylamide gel electrophoresis (SDS-PAGE) (hereinafter referred to as "SAP(Val) and/or SAP-(Phe) multimers"). Other multimers or closely related peptides may exist but in smaller or undetectable concentrations. As used herein, "simple molecular weight" refers to the molecular mass of what is thought to be the smallest polypeptide chain after sulfhydryl reduction which serves as a repeating building block for such multimers.

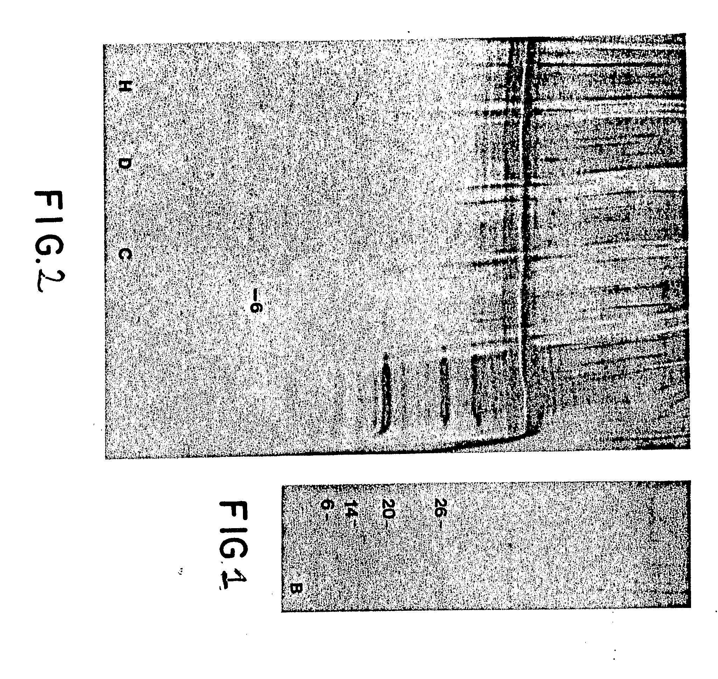

To determine the molecular weights and separate monomers from larger multimers, for instance, a gel with about 3-27% polyacrylamide, in particular about 15% polyacrylamide, and containing about 2% of SDS may be used to separate and determine the molecular weights of SAP(Val) and SAP(Phe) monomers and SAP(Val) and SAP(Phe) multimers in SDS-PAGE. FIG. 1 illustrates SAP(Val) purified and delipidated as described herein and applied to a 10-20% sodium dodecyl sulfatepolyacrylamide electrophoresis gel in the absence of $\beta$-mercaptoethanol. In FIG. 1, lane B represents approximately 2 micrograms of SAP(Val) detected by silver staining which migrated at $M_r$ = 6,000, 14,000, 20,000 and 26,000. For comparison, low molecular weight protein markers such as trypsin inhibitor (6,200), lysozyme (14,000), $\beta$-lactalbumin (18,400), $\alpha$-chymotrypsin (25,700) and ovalbumin (43,000) may be used. These may be obtained from BRL, Inc., of Bethesda, Maryland. When gel separation is performed on SAP(Val) in the absence of sulfhydryl reducing agents, such as $\beta$-mercaptoethanol, dithiothreitol (DTT), or after reduction and alkylation, an increase is observed in the larger, SAP(Val) or SAP(Phe) multimers over SAP(Val) or SAP(Phe) monomers. Presently, it is believed that sulfhydyl bonding, non-sulfhydryl aggregation and/or interpeptide bonding of a fraction of SAP(Val) or SAP(Phe) accounts for the larger, hydrophobic multimers. When run in the absence of reducing agents all sample SAP(Phe) migrates at $M_r$ = 18,000 and larger, while most of the immunoreative material thought to be SAP(Val), migrates with a predominant $M_r$ = 5,000-6,000 although some aggregates of SAP(Val) migrate at a higher $M_r$. It should be understood that the molecular weights are only estimates based upon relative migrations and that other suitable techniques may be employed to make more accurate molecular weight determinations.

As used herein, "hydrophobic", refers to solubility in non-polar solvents, such as 3:1 ether/ethanol, chloroform, chloroform/methanol in various ratios, such as 3:1, and having an abundance of hydrophobic non-charged amino acids. The term "surfactant-associated protein", as used herein, refers to proteins associated with binding to or co-purifying with the phospholipid components of mammalian surfactants during centrifugation in isotonic solution.

SAP(Val) and SAP(Phe) may be further characterized as being substantially resistant to protease enzymes (trypsin, chymotrypsin and staph. V-8), endoglycosidase F, and collagenase. It has been discovered that SAP(Val) and SAP(Phe) are not degraded nor are their size heterogeneity significantly altered by these enzymes and therefore are distinct from SAP-35 and its fragments.

In further characterizing SAP(Val) and SAP(Phe), they are localized in lung tissue and specifically in pulmonary type II cells and lung surfactant in a variety of mammalian species, as well as in human amniotic fluid near term gestation.

SAP(Val) may be purified from, for example, animal tissue and fluids, cells, cultivated cells, suitable pulmonary surfactant replacement preparations available, such as CLSE or Surfactant-TA, and the like. It should be appreciated therefore that the expression "animal tissue" as used herein is meant in a broad sense to encompass all appropriate sources for SAP(Val), including but not limited to animal tissue and fluid, cells, cultivated cells, pulmonary surfactant replacement preparations, such as CLSE and Surfactant-TA, and the like. When isolating SAP(Val) from animal tissue, it is preferred, however, to isolate SAP(Val) from animal pulmonary tissue or amniotic fluid, and more preferably human pulmonary tissue or cultured cells.

Solutions containing SAP(Val) are obtained by extraction of animal tissue in which SAP(Val) occurs. For instance, mammalian lung is excised, the trachea preferably is cannulated and washed repeatedly with several volumes of an iced, buffered solution, such as 0.9% NaCl (pH 7.0-7.4), to remove surfactant from the alveoli. The wash is centrifuged at low speed, for example, 1,000 × g for ten minutes, and is repeated, if necessary, to remove contaminating blood and white cells or macrophages. All procedures preferably should be performed at about 2-4° C. This surfactant material should then be centrifuged at high speed from the alveoli wash to sediment particulate materials at about 10,000 × g for about 30 minutes or longer. This pellet preferably should be resuspended and washed by repeated centrifugation and sonicated or freeze thawed, several times if necessary, in a suitable buffer, such as the one above, to uniformly resuspend the materials.

The resulting white surfactant is pelleted by centrifugation, the aqueous material is removed and the pellet is extracted with about 10-100 volumes of approximately 2:1 ether/ethanol, 3:1 chloroform/methanol or other suitable organic solvents at about -30° C to produce a supernatant containing SAP(Val). Extraction is carried out overnight. The material is then centrifuged at about 10,000 × g for about 20 minutes or longer. The resulting liquid fraction contains lipid and includes the SAP(Val) of this invention. The liquid fraction thereafter may be dried (concentrated) with, for instance, $N_2$ stream, resuspended in an organic solvent, such as chloroform or other suitable organic solvents, and applied to a silicic acid column or LH-20 column approximately 50 cm × 2.5 cm in size which is pre-equilibrated with chloroform or other suitable organic solvents at room temperature. The column is then eluted with, for example, 50 to 200 ml of a mixture of chloroform and alcohol, such as methanol or the like, increasing the alcohol concentration from 0% to about 100% in stepwise or gradient fashion. SAP(Val) is eluted in the solvents containing alcohol having concentrations of greater than about 40% up to about 100% and more particularly about 40% to about 80%. Most of the lipids present in the SAP(Val) are separated from the proteins. Dialysis in chloroform/methanol or acidified (0.1 N HCl) chloroform/methanol further removes phospholipid and further purifies the protein. It should be appreciated to those skilled in the art that the silicic acid column may also be eluted with any other suitable solvents capable of extracting therefrom the SAP(Val) of this invention.

## EXAMPLE 1

### Isolating Surfactant Material

SAP(Val) is isolated from lung lavage material obtained from adult dogs and cows after sacrifice. SAP-(Val) is also isolated from human surfactant which is obtained from human cadavers after autopsy or from lung lavage of consenting adults. In animals, the trachea is cannulated and the lung is lavaged three times with several volumes of iced 0.9% NaCl, 50 mM $Na_2HPO_4$, and 5 mM EDTA, pH 7.2. Cells and debris are removed by centrifugation at 80 × g for about 10 minutes (twice) and the supernatant is then centrifuged at 40,000 × g for about 30 minutes at 4° C. The pelleted material is then resuspended in the above buffer with or without 1 mM phenylmethyl-sulfonylfluoride and sonicated for 10 seconds with a Branson sonifier. Surfactant material is pelleted by repeated centrifugation at about 40,000 × g for about 30 minutes at 4° C.

## EXAMPLE 2

### Purification of Hydrophobic SAP(Val) From Surfactant Material

The surfactant pellet is further processed by extraction in about 3:1 ether/ethanol or chloroform/methanol at about -30° C for about 16 hours. Ether/ethanol or chloroform/methanol extracts containing SAP(Val) are evaporated to near dryness with $N_2$ gas and are redissolved in chloroform. The redissolved residue in chloroform is applied to a BioSil-HA column (silicic column dimensioned 2.5 cm by 40 cm, obtained from Bio-Rad, Richmond, California) and is equilibrated in chloroform. Lipid-protein fractions are recovered by stepwise elution with chloroformmethanol mixtures (200 ml each) with successive increases in concentration of methanol (10% intervals) in chloroform. SAP(Val) is eluted between 3:2 and 1:4 chloroform/methanol solvent elution or about 40% to about 80% methanol in chloroform.

Fractions containing the SAP(Val) of this invention now may assessed by, for example, fluorescamine assay and by gel electrophoresis migration followed by staining with silver stain or Coomassie brilliant blue staining. The SAP(Val) isolated at this point is considered to be substantially pure and homogeneous. By "substantially pure" it is meant herein that SAP(Val) proteins are substantially free of contaminants, such as cells, cellular debris, DNA, RNA, and major surfactant glycoproteins or fragments thereof. It should be appreciated, however, that some lipids still remain in the now substantially pure SAP(Val). Dialysis at room temperature in 2% chloroform/methanol or acidified chloroform/methanol in cellulose dialysis bags having a 3,000 molecular size cutoff is useful for further delipidating SAP(Val). In the most preferred form, the substantially pure SAP(Val) contains on the order of about 10% or less of contaminants.

If desired, to further delipidate the now isolated, substantially pure SAP(Val), consisting essentially of the SAP(Val) monomers and the larger multimers thereof, the fractions containing the eluted SAP(Val) preferably are pooled, evaporated to near dryness and subjected to repeated dialysis in, for example, a cellulose dialysis bag with about 100-500 volumes of about 2:1 of chloroform/methanol or other suitable mixtures or acidified chloroform/methanol mixtures acidified with HCl or other appropriate acids at about room temperature. This procedure should proceed until the SAP(Val) is virtually free of contaminating lipids. This substantially purified SAP(Val) may again be assessed by fluorescamine assay and gel electrophoresis migration. It should be realized that other suitable delipidating steps may also be employed to accomplish the above delipidating objective. The approximate amino acid composition also can now be determined and the proteins identified after PAGE with Coomassie brilliant blue, silver staining or other standard staining techniques.

It should be appreciated that other suitable solvents and methods may be employed to separate and duplicate the SAP(Val) from appropriate sources. A discussion of SAP(Val) and SAP(Phe) proteins appears in Whitsett et al., Pediatr. Res., 20, 460-467 (1986), and Whitsett et al., Pediatr. Res., 20, 744-749 (1986), in which these articles are incorporated by reference herein.

## EXAMPLE 3

### Characterization of SAP(Val) and SAP(Phe)

With respect to the determination of the amino acid composition of SAP(Val) and SAP(Phe), purified samples are hydrolyzed by two different methods. In the first method, the samples are hydrolyzed in constantly boiling 5.7 N HCl containing 0.3% phenol and 0.1% β-mercaptoethanol at 110° C. under a vacuum for about 24 and 48 hours. In a second method, these same samples are hydrolyzed in 12 N HCl/trifluoroacetic acid (2:1) containing 0.3% phenol at 150° C under vacuum for 2 hours, 6 hours, 24 hours and 48 hours. The cysteine composition of these samples is determined separately as cysteic acid after performic acid oxidation. Hydrolysis is then performed on the oxidized samples in 12 N HCl/trifluoroacetic acid (2:1) containing 0.3% phenol at 150° C for three hours. The amino acids are determined using a Beckman 6300 amino acid analyzer with a SICA 7000A Integrator.

Table 1 illustrates the amino acid compositions determined for bovine and canine SAP(Val) using an amino acid analyzer. It should be understood to those skilled in the art that, because the amino acid compositions have been determined using a Beckman 6300 analyzer, the amino acid residue compositions, as reported per protein molecule in Table 1, are only estimates based upon this analytical technique. In fact, because the compositions in Table 1 are generally divisible by 2 to obtain whole numbers, and because subsequent amino acid sequencing data account for about one-half of the residues reported in Table 1, it may be the case that the results reported therein represent a dimer of SAP(Val), or at least represent twice the respective composition values for a SAP(Val) monomer.

TABLE 1

| | BOVINE SAP(Val) | CANINE SAP(Val) |
|---|---|---|
| ASX | 2.1 | 0.5 |
| THR | 0.2 | 0.3 |
| SER | 0.6 | 4.0 |
| GLX | 0.3 | 0.4 |
| PRO | 4.3 | 4.6 |
| GLY | 4.1 | 5.1 |
| ALA | 2.3 | 2.3 |
| CYS | 3.6 | 1.6 |
| VAL | 23.1* | 19.2* |
| MET | 1.8 | 1.7 |
| ILE | 4.7* | 5.9* |
| LEU | 13.8* | 13.9* |
| TYR | 0.2 | -- |
| PHE | 0.3 | 1.9 |
| HIS | -- | -- |
| LYS | 2.0 | 2.0 |
| TRP | -- | -- |
| ARG | 1.9 | 2.0 |
| ESTIMATED M.W. | 6588.0 | 6620.0 |

*HIGHEST VALUE OBTAINED FROM HYDROLYSIS

The sequences for the first 25, the first 23, and the first 19 $NH_2$-terminal amino acid residues, respectively, for bovine, canine and human SAP(Val) have been determined and are shown in Table 2.

The sequences for the first 6, the first 7, and the first 14 $NH_2$-terminal amino acid residues, respectively, for the bovine, canine and human SAP(Phe) have been determined and are shown in Table 3.

## TABLE 2

### SAP(Val)
### $NH_2$-Terminal Amino Acid Sequences

| | |
|---|---|
| Bovine: | Leu-Ile-Pro-Cys-Cys-Pro-Val-Asn-Ile-Lys-Arg- |
| | Leu-Leu-Ile-Val-Val-Val-Val-Val-Val-Leu- |
| | (Val) |
| | Leu-Val-Val-Val |
| | (Val) |
| Canine: | Ile-Pro-Cys-Phe-Pro-Ser-Ser-Leu-Lys-Arg-Leu- |
| | Leu-Ile-Ile-Val-Val-Val-Val -Val-Val-Val- |
| | (Ile)(Ile) |
| | Val-Val |
| Human: | Leu -Ile-Pro-Cys-Cys-Pro-Val-Asn-Leu-Lys-Arg- |
| | (Ile)(Pro) (His) |
| | (Gly) |
| | Leu-Leu-Ile-Val-Val-Val-Val-Val |

Amino acids having alternate amino acids in parenthesis underneath indicate alternative amino acids potentially determinable by the above procedure. For.example, the human $HN_2$-terminus is now believed to be Gly, but sequences having Gly, Ile or Leu as an $NH_2$-terminus (potentially obtained as a result of variable proteolytic processing at the $NH_2$-terminus) are comprehended in the term "human" SAP(Val).

TABLE 3

SAP(Phe)

$NH_2$-Terminal Amino Acid Sequences

| | |
|---|---|
| Bovine: | Phe-Pro-Ile-Pro-Ile-Pro |
| Canine: | (Gln)-Pro-Ile-Pro-Ile-Pro-Tyr<br>(Ile)<br>(Phe)<br>(Gly) |
| Human: | Phe-Pro-Ile-Pro-Leu-Pro-Tyr-Cys-Trp-Leu-Cys-Arg-Ala-Leu |

The sequences shown in Tables 2 and 3 were generated by Edman sequence analysis by first dialyzing SAP(Val) or SAP(Phe) samples against water. The samples are taken up in methanol and applied to a polybrene-treated glass fiber filter. The analysis is performed on an Applied BioSystems model 470A Gas Phase Protein Sequencer. Analysis of the resulting phenylthiohydantoins (PTH) is accomplished by high pressure liquid chromatography (HPLC) at 50°C on an Altex reversed-phase PTH-$C_{18}$ column. A binary buffer system consisting of ammonium acetate and buffered acetonitrile, pH 4.5, is used.

The chromatography procedure is conducted utilizing buffer A which contains about 10% acetonitrile and buffer B which contains about 90% acetonitrile. The initial chromatography conditions are 70% buffer A and 30% buffer B with a flowrate of 1.0 ml per minute. One minute after injection, the concentration of buffer B is linearly increased to 50% over a 3 minute period and held at this level for 8 minutes. The concentration of Buffer B then is reduced to 30% over one minute. After re-equilibration, which takes approximately 10 minutes, another PTH sample is injected. A discussion of this separation procedure appears in greater detail in Meuth et al., Analytical Biochem., 154, 478-484 (1986) which is incorporated by reference herein.

The $NH_2$-terminal amino acid residue sequences for canine, bovine and human SAP(Val), although similar to one another as indicated above, appear to be substantially different from the $NH_2$-terminal amino acid residue sequences of SAP(Phe) for all three species. Similarly, the $NH_2$-terminal amino acid residue sequences of SAP(Phe) for all three species are similar to one another but substantially different from the SAP(Val) sequences. In addition, when SAP(Val) is subjected to gel electrophoresis migration in the absence of β-mercaptoethanol, a sulfhydryl reducing agent, major and minor amounts of protein appear. This may be better understood with reference to FIG. 1 wherein major amounts of silver stained protein exist at the 6,000 and 14,000 molecular weight migratory regions, whereas only minor amounts of silver stained protein exist at the 20,000 and 26,000 molecular weight migratory regions. Thirdly, when an isolate is subjected to gel electrophoresis migration in the presence of β-mercaptoethanol, silver stained proteins appear only at the 6,000 and 14,000 molecular weight migratory regions as shown in FIG. 2. In FIG. 2 gel electrophoresis of SAP(Val) purified and delipidated as described herein, and applied to 10-20% sodium dodecyl sulfatepolyacrylamide electrophoresis gels in the presence of β-mercaptoethanol is illustrated. Each lane represents approximately 2 micrograms of each protein detected by silver staining. SAP(Val) from each species, H = human, D = canine, and C = bovine, migrated at Mr=6,000 and 14,000. The β-mercaptoethanol artifact is also observed at Mr=65,000-70,000. Standard molecular weight markers are seen on the right. There is the possibility that SAP(Phe) is migrating between the 6,000 and 14,000 molecular weight regions, which may arise as a result of proteolysis of a precursor protein. Thus, there may be a slight heterogeneity of the forms between 6,000 and 14,000 which may reflect variable proteolytic processing of both SAP(Phe) and SAP(Val) precursors.

Therefore, at least two distinct 4,000-7,000 dalton SAP monomers exist that are believed to co-elute together, to co-purify together via gel electrophoresis migration, to have similar molecular weights of about 4,000-7,000 daltons determined in a polyacrylamide gel containing sodium dodecyl sulfate, to have similar biophysical surfactant-like activity and to have similar enzyme resistance. Further, it is believed that the larger multimers, i.e., $M_r$ = 18,000 and 26,000, resulting from one or more SAP(Val) and/or SAP(Phe) monomers are possibly bonded together via sulfhydryl bonds in view of their migration patterns in the absence and presence of β-mercapoethanol, as illustrated in FIGS. 1 and 2. Therefore, it should be understood that any 4,000-7,000 dalton hydrophobic surfactant-associated proteins, SAP(Val) and SAP-(Phe), are well within the contemplation of this invention.

EXAMPLE 4

Extraction of mRNA

RNA was extracted from lung tissue of an adult male immediately after death. Tissue was provided by the National Diabetes Tissue Interchange, Washington, D.C. The RNA was extracted by the method of Chirgwin et al., Biochem., 18, 5294-5499 (1979). Poly(A)$^+$ RNA was isolated by purification through oligo-(dT) column chromatography essentially as described in Maniatis et al., Molecular Cloning - A Laboratory Manual, Cold Spring Harbor Laboratory, Cold Spring Harbor, New York (1982) (hereinafter referred to as Maniatis et al.). A cDNA library was constructed in λ gtll as described by Young et al., Proc. Nat'l Acad. Sci. (USA), 80, 1194-1198 (1983).

EXAMPLE 5

Immunological Screening of cDNA Library

Polyclonal rabbit antisera were raised against bovine surfactant proteolipid, prepared from chloroform/methanol extraction of bovine lung surfactant [see Notter et al., Chem. Phys. Lipids, 33, 67-80 (1980), and were preabsorbed to minimize nonspecific background to E. coli proteins in a screening procedure. The preabsorption was done by the following procedure. An E. coli strain Y1090 lysate was disrupted by treatment with EDTA (pH 8.0) followed by sonication on ice with six bursts from a Branson sonifier at 50 watts for 10 seconds each. The sonicate was centrifuged at low speed to clear the supernatant. This supernatant was incubated with the above antisera for 48 hours at 4° C, then centrifuged to remove antibody/antigen complexes.

The library was plated on E. coli strain Y1090 at 20,000 pfu per 150 mm dish, grown for 5 hours at 42° C. and then blotted to nitrocellulose filters soaked in 10 mM isopropylthiogalactoside (IPTG). Filters were incubated overnight at 37° C. and then blocked for 1 hour at 4° C. in 50 mM Tris HCl, pH 7.4, 150 mM NaCl (TBS), and 50 gm/L powdered milk. Primary antibody reactions were conducted at a dilution of 1:1000 by incubation with the filters at 4° C. overnight. The filters were then incubated with horseradish peroxidase conjugated goat anti-rabbit IgG (GAR-HRP) at a dilution of 1:3000 at 4° C. for 16 hours. Color was developed with 4-chloro-1-napthol for 3-5 minutes and reactions were then terminated by serial washings in distilled $H_2O$. Second and third screens were performed at 10-fold and 100-fold less phage per plate to identify isolated plaques.

EXAMPLE 6

Synthesis Of Oligonucleotide Probes

An oligonucleotide of mixed sequence was made using the phosphoramidite method of Matteucci and Caruthers, J. Am. Chem. Soc., 103, 3185-3191 (1981) on an Applied Biosystems DNA synthesizer Model No. 380A. The sequence for the probes was $(GTN)_6G$ wherein N is either deoxycytidine or deoxyinosine [Wood et al., Proc. Nat'l Acad. Sci. (USA), 52, 1585-1588 (1985)]. This sequence was based on the stretch of valine residues seen in human, canine and bovine SAP(Val) $NH_2$-terminal amino acid sequences shown in Table 2. The uniqueness of the valine stretch in SAP(Val) allowed for remarkable selectivity in the screening of the cDNA library. Due to the codon redundancy in this region, it would not have been feasible to utilize this region as a template for a probe without the use of deoxyinosine and the selection of deoxycytidine as a preferred base in the third position.

A second oligonucleotide probe was synthesized corresponding to the reverse translation of the 9 amino acids of the $NH_2$-terminal sequence of human SAP(Phe) seen in Table 2. The sequence of this probe was 5′-CCAICAITAIGGIATIGGIATIGGIAA-3′, where I stands for deoxyinosine.

## EXAMPLE 7

### Screening With Oligonucleotide Probes

The positive clones selected with bovine surfactant polyclonal antisera were plated out by spotting 1 ul of a phage supernatant on a lawn of E. coli strain Y1090 (Δ lac U169 pro $A^+$ lon ara D139 strA supF {trpC 22:Tn10} pMC9) (Clontech Laboratories, Palo Alto, California). The plates were incubated at 42° C. for 5-6 hours and then removed and overlaid with nitrocellulose filters. The filters were removed and denatured in 1.5 M NaCl, 0.5 M NaOH for 1 minute, neutralized in 0.5 M Tris, pH 8.0, 1.5 M NaCl for 5 minutes, rinsed in 2 × SSPE (20 × SSPE = 3.6 M NaCl, 200 mM $NaH_2PO_4$, 20 mM EDTA, pH 7.4), and air dried. The filters were then baked for 1 hour at 80° C. in a vacuum oven. Filters were prehybridized for 6 hours at 45° C. in prehybridization buffer (5 × SSPE, 5 × Denhardt's, 50 mM sodium phosphate pH 7.0, 1 mM sodium pyrophosphate, 100 uM ATP, 50 ug/ml boiled salmon sperm DNA). While the filters were prehybridizing, the SAP(Val) oligonucleotide probe described in Example 6 was end-labeled with $T_4$ polynucleotide kinase and $\gamma$-$^{32}$P-ATP essentially as described by Maniatis et al., "Molecular Cloning: A Manual," Cold Spring Harbor, New York (1982). The SAP(Val) probe was added to the prehybridization buffer at a concentration of approximately 12 ng/ml, the filters were added, and incubated at 30° C. overnight. The filters were then washed in 0.2 × SSPE, 0.1% sodium dodecyl sulfate (SDS) at room temperature for 30 minutes, then at 32° C. for 45 minutes in fresh wash solution. They were then air-dried and subjected to autoradiography. This procedure was repeated with a second set of filters using the SAo(Phe) probe described in Example 6.

When the above procedures were completed for both the SAP(Val) and SAP(Phe) probes, it was found that only the SAP(Phe) probe hybridized to any of the clones. The reason for this is not clear, but it is theorized that the antisera used for screening the cDNA library was only sensitive enough to detect SAP-(Phe). This may have been because the SAP(Val) was not present in large enough quantities in the bovine lung surfactant preparation used for immunization to elicit an immunogenic response or because the SAP-(Val) was less immunogenic than the SAP(Phe). As will be readily appreciated by those skilled in the art, this could be overcome by use of purification and screening of polyclonal antisera prior to use or by use of specific monoclonal antibodies.

Another approach which was used was to simply screen the cDNA library directly for clones encoding SAP(Val) using the SAP(Val) oligonucleotide of Example 6, end-labeled as described above. The cDNA library was plated out at approximately 30,000 pfu per 150 mm dish. The plates were incubated at 37° C. overnight then removed and overlaid with nitrocellulose filters. After the first set of filters was removed, a duplicate lift was made from each plate. Both sets of filters were treated as described above, with the following changes. Prehybridization was done in 6 × SSPE. 90 mM sodium citrate pH 7.6, 2 × Denhardt's, and 500 µg/ml boiled salmon sperm DNA at 37° C. for 5 hours. Hybridization was done in 6 × SSPE, 1 × Denhardt's, 50 µg/ml boiled salmon sperm DNA with a probe concentration of 1.5 ng/ml. The filters were hybridized at 55° C. for 1 hour then overnight at 37° C. They were washed in 6 × SSPE 2 times at room temperature and then 2 times at 37° C. Clones found to hybridize to the probe on both sets of filters were isolated and purified by replating and screening at lower densities.

## EXAMPLE 8

### Characterization Of SAP(Val) and SAP(Phe) cDNA Clones

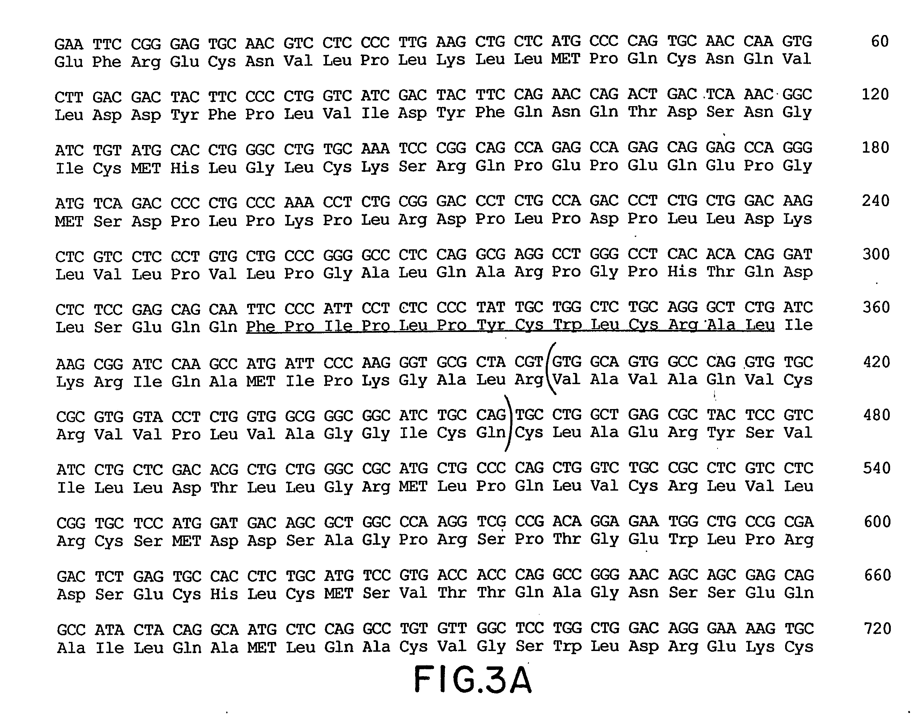

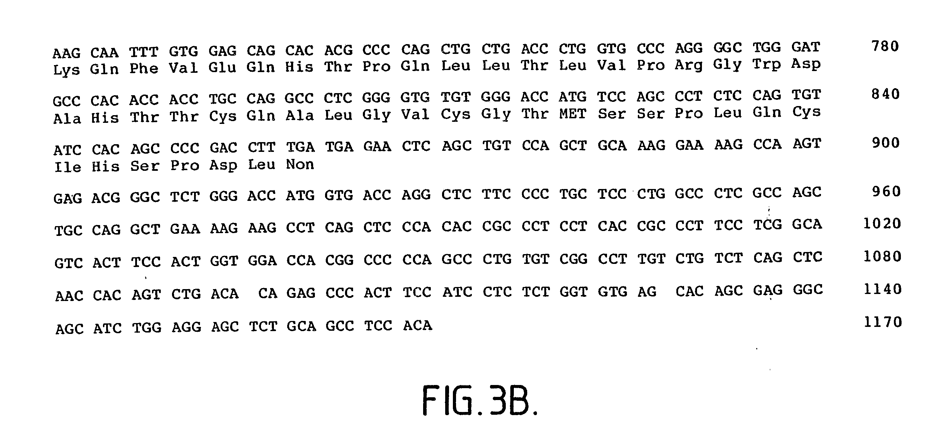

Several clones found to hybridize with either the SAP(Val) or SAP(Phe) probes were subcloned into the EcoRI site of E. coli plasmid puC19 (described in Yanisch-Perron et al., Gene, 33, 108-119 (1985)) for further characterization. The clones selected using the SAP(Phe) probe were mapped by restriction analysis, and then a clone was selected for further characterization by sequence analysis. This clone was subcloned into an M13 sequencing vector (described by Messing et al., Methods in Enzymology, 101, 70-78 (1983) and sequenced using M13 oligonucleotide primers according to standard methodologies as described by Sanger et al., Proc. Nat'l Acad. Sci. (USA), 74, 5463-5467 (1977). E. coli JM103 of JM109 (Pharmacia, Inc., Piscataway, NJ) were used for growth of pUC plasmid and M13 phage subclones. The sequence of this SAP(Phe) clone is shown in FIG. 3. Underlined in this figure is the region which corresponds to the $NH_2$-terminal amino acid sequence for human SAP(Phe) shown in Table 3.

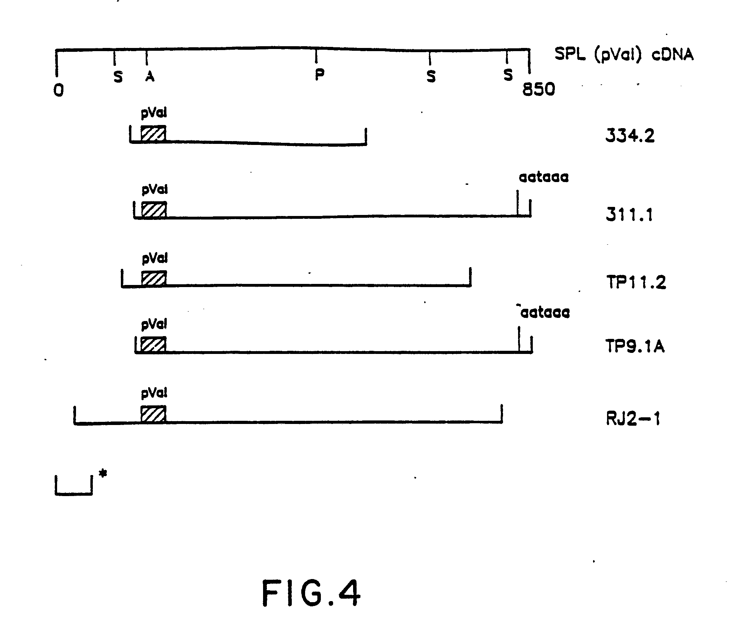

Nucleotide sequence analysis of one SAP(Val) clone, designated 334.2, of 0.3 kilobases comprised an open reading frame predicting close identity to the amino acid sequence determined directly from the human protein and was used to isolate other clones from the same cDNA library, as illustrated in FIG. 4. Sequence analysis of nine unique clones resulted in a consensus sequence predicting a larger polypeptide precursor.

In FIG. 4, clone 334.2 is the initial SAP(Val) isolate and was used as a probe for re-screening of the cDNA library. Clones 311.3 and 13-1 have an 18 base pair deletion not seen in clones TP9-1 and TP11-2. In FIG. 4: a notched box indicates the valine rich hydrophobic domain of SAP(Val) clones; "A" denotes ApaLI, a restriction endonuclease that cleaves phage λ DNA infrequently and cuts at the start of the valine rich domain of SAP(Val); P = PstI; S = SmaI; a "*" indicates a sequence obtained from human lung mRNA directed dideoxy sequencing utilizing 5′ SAP(Val) oligo primers.

In FIG. 5, the nucleotide sequence was determined using overlapping cDNA clones 311.3, 13-1, TP11-2, TP9-1 and RJ2-1. The broken under line indicates sequence from mRNA directed dideoxy sequencing using RJ2-1 as a primer that is present from sequence of the first exon of the SAP(Val) genomic clone VG519. Solid underlined amino acid sequence obtained directly on human SAP(Val) protein. The predicted sequence and obtained sequence match at 16 or 17 amino acids, the difference being $His_{32}$ instead of Asn. Overlined DNA sequence is the 18 bp sequence that is absent in cDNA clones 311.3 and 13-1.

The Ile-Pro-Cys-Cys peptide was found within the reading frame of a larger polypeptide suggesting that the hydrophobic peptide of $M_r$ = 4,000-7,000 arises from proteolytic processing of a precursor protein at both the amino- and carboxy-terminus.

Full length cDNAs were not detected within this human lung library. RNA sequencing and analysis of genomic DNA were therefore utilized to predict the complete SAP(Val) mRNA.

A direct nucleotide sequence of SAP(Val) RNA was derived from human lung RNA. Dideoxynucleotide RNA sequencing was done by a modification of one procedure described by Geliebter et al., Proc. Nat'l Acad. Sci. (USA), 83, 3371-3375 (1986). An oligonucleotide primer specific for the 5′ terminus of SAP(Val) cDNA was synthesized and end-labelled. Poly $(A)^+$ mRNA (6 µg) and [$^{32}$P]labelled primer (5 ng) were heated at 80 °C for 3 minutes in 10 µl of annealing buffer (0.25 M KCl, 10 mM Tris, pH 8.3) and then allowed to anneal for 45 minutes at 50 °C.

RNA template:primer solution (2 µl) was added to 3.3 µl of transcription buffer (24 mM Tris pH 8.3, 16 mM $MgCl_2$, 8 mM dithiothreitol, 0.4 mM dATP, 0.4 mM dCTP, 0.4 mM dTTP, 0.8 mM dGTP, 100 µg/ml actinomycin D) containing 5 units of AMV reverse transcriptase (Amersham) and 1 µl of one dideoxynucleotide triphosphate (0.5 mM ddGTP, 0.285 mM ddATP, 1.0 mM ddTTP, or 0.15 mM ddCTP). The reactions were incubated at 50 °C for 45 minutes, heated at 80 °C for 3 minutes in loading buffer (10 mM EDTA, 0.2% bromphenol blue, 0.2% xylene cyanol in 100% formamide), then loaded onto a sequencing gel.

Analysis after autoradiography revealed that the RNA sequence overlapped with the cDNA sequence and ended at a clear stop.

The SAP(Phe) cDNA clones isolated by antibody screening and oligonucleotide hybridization were incomplete on the 5′ and 3′ ends. Several of these cDNA clones were used as hybridization probes to isolate additional cDNA clones from the library. Sequence analysis of one of these clones showed it to be complete on both 5′ and 3′ ends. This sequence is shown in FIG. 6. The SAP(Phe) transcript encodes a precursor protein of approximately 42 kilodaltons.

In order to determine the exact 5′ end of the transcript, SI nuclease mapping was performed using with a fragment from the 5′ end of the SAP(Phe) genomic clone described in Example 11. Probes were prepared and SI nuclease protection was done by the method of Kay et al., Mol. Cell. Biol. 6, 3134-3143 (1986). In addition, adult human lung poly$(A)^+$ RNA was sequenced directly as described above. Both the SI mapping and the direct RNA sequencing identified the 5′ end as shown in FIG. 6.

The hydrophobic regions of SAP(Phe) and SAP(Val) as shown in FIGS. 3 and 5 in parentheses are somewhat homologous. Although these two proteins are encoded by distinct genes, it is believed that they are structurally related. Therefore, these proteins may be members of a family of related proteins which bind phospholipids and are useful in surfactant replacement therapy and diagnosis.

EXAMPLE 9

Expression of LacZ/SAP(Phe) Fusion Protein in E.coli

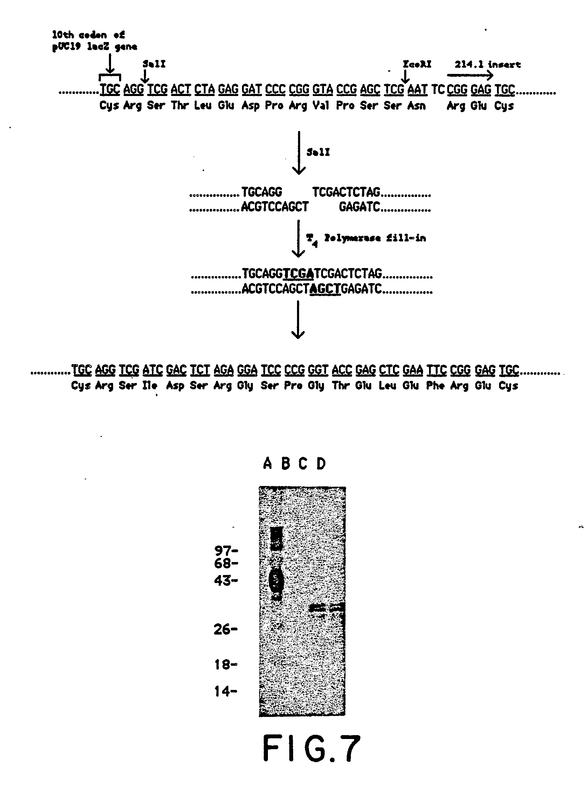

The SAP(Phe) cDNA clone shown in FIG. 3 was inserted into the EcoRI site of plasmid pUC9 in the same orientation as the lacZ gene. The reading frame was then altered by cutting the plasmid DNA with the restriction enzyme SalI, then filling in the ends with $T_4$ polymerase and deoxynucleotides. This resulted in the insertion of 5 nucleotides within the lacZ gene upstream from the site of the fusion with the SAP(Phe) cDNA. This then led to the production of a lacZ/SAP(Phe) fusion product, which contains approximately 2400 daltons of lacZ at the $NH_2$-terminal end of the SAP(Phe) gene product. FIG. 7 shows a schematic diagram of this construction.

EXAMPLE 10

Testing of Fusion Protein With SAP(Phe) Polyclonal Antisera

E.coli strain JM109 containing the plasmid described in Example 9 was grown up at 37° C. until the optical density at 600 nm reached 0.5, then IPTG was added to a concentration of 1 mM. The cells were grown for an additional 3-4 hours at 37° C. then pelleted and resuspended in 1/15th volume lysis buffer (62.5 mM Tris pH 6.8, 2% SDS, 10% glycerol, 5% $\beta$-mercaptoethanol, 0.1 mg/ml bromphenol blue). The samples were boiled for 5 minutes and loaded on a 12.5% Laemmli electrophoresis gel (Laemmli, Nature, 227, 680-685 (1970). After the gel was run, the proteins were transferred to nitrocellulose essentially by the method of Towbin et al., Proc. Nat'l Acad. Sci. (USA), 76, 4350-4354 (1979).

The nitrocellulose filter was preblocked in TBS containing 50 gm/l powdered milk at room temperature for 1 hour, then incubated with the preabsorbed antisera described in Example 5 at a dilution of 1:1000 at room temperature overnight. After this, the filter was washed 2 times in TBS, then incubated with GAR-HRP at a dilution of 1:2000 for 1 hour at room temperature.

A color reaction was developed as described in Example 5. FIG. 7 shows results of this test. Lane B contains the lacZ/SAP(Phe) fusion protein, while lane A contains protein from cells containing the plasmid which had not undergone the fill-in as described in Example 9. The SAP(Phe) antisera of Example 5 recognized proteins of 32,000-34,000 daltons in molecular weight which is in agreement with the predicted size of the translation product from the open reading frame of the SAP(Phe) clone plus the 2400 dalton portion of lacZ.

EXAMPLE 11

SAP(Phe) Genomic Clones

A human embryonic kidney cell genomic library was constructed in the $\lambda$ vector EMBL3 [Frischauf et al., J. Mol. Biol., 170, 827-842 (1983)]. DNA isolated from these cells was partially digested with MboI, size selected in an agarose gel for fragments larger than 20 kb, and ligated into the BamHI site of the $\lambda$ vector. This library was screened with hybridization probes made from SAP(Phe) cDNA clones. The SAP(Phe) cDNA probes were labeled by the random oligonucleotide primer labeling technique using a kit purchased from Pharmacia, Inc. (Milwaukee, Wisconsin). The library was plated on E. coli strain NM539 at a density of 40,000 pfu per plate then blotted to nitrocellulose filters. The filters were prehybridized at 55° C. for 5 hours in 2 × SET (20 × SET=3M NaCl, 1M Tris, 20 mM EDTA, pH 7.8), 50 mM sodium phosphate, 0.5% SDS, 5 × Denhardt's solution, 0.1 mg/ml denatured salmon sperm DNA. The filters were then hybridized overnight at 50° C. in 3 × SET, 50 mM sodium phosphate, 0.5 mg/ml heparin, 0.1 mg/ml denatured salmon sperm DNA, 30% formamide plus the labeled probe at approximately $10^6$ cpm/ml. After hybridization, the filters were washed 2 times at 60° C. for 30 minutes each in 0.2 × SET, 0.1% SDS. They were then air dried and exposed to Kodak XAR film. Second and third screens were performed at lower densities to isolate hybridizing clones.

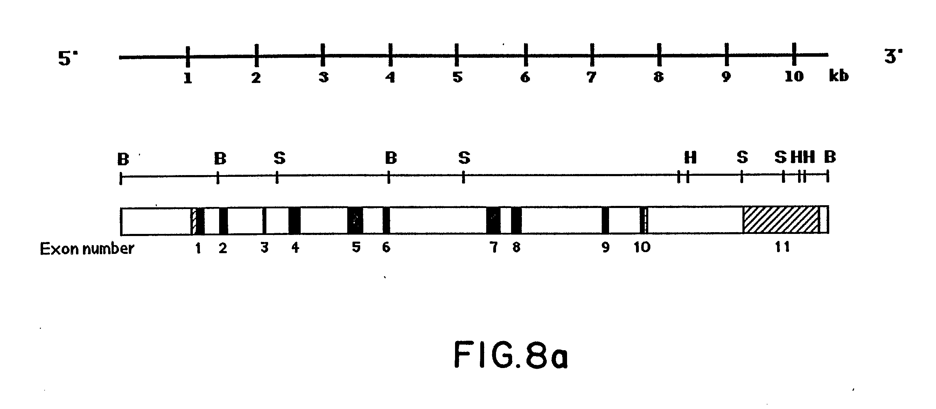

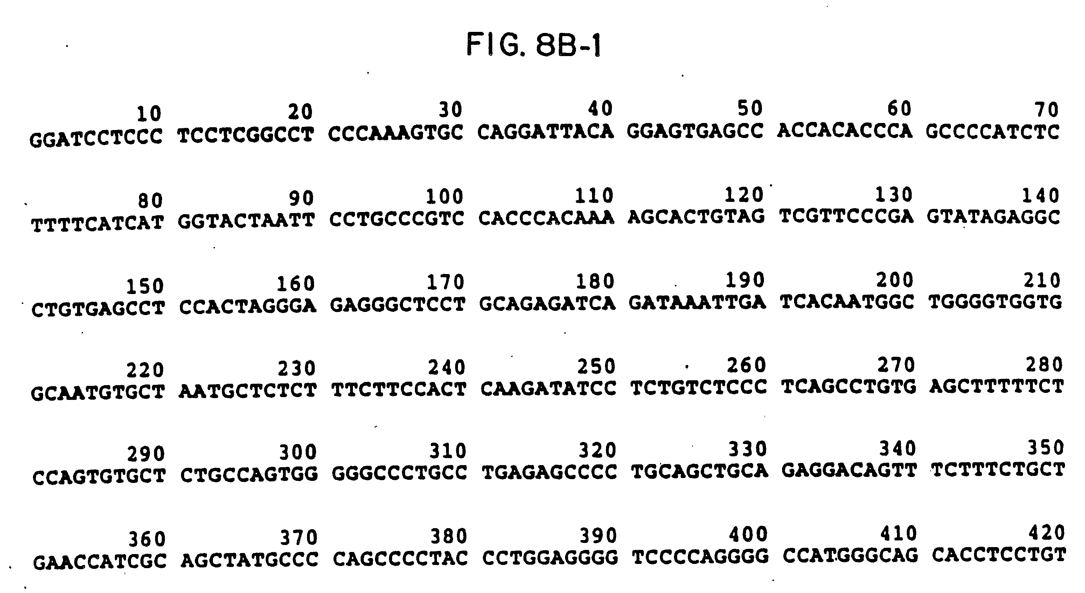

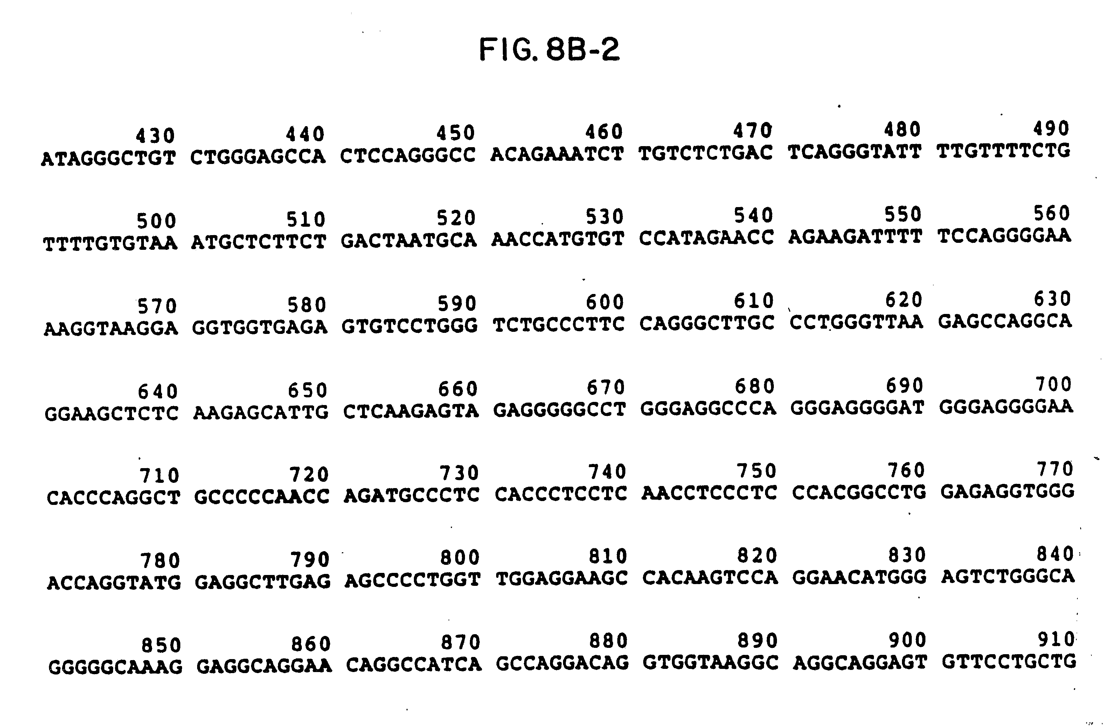

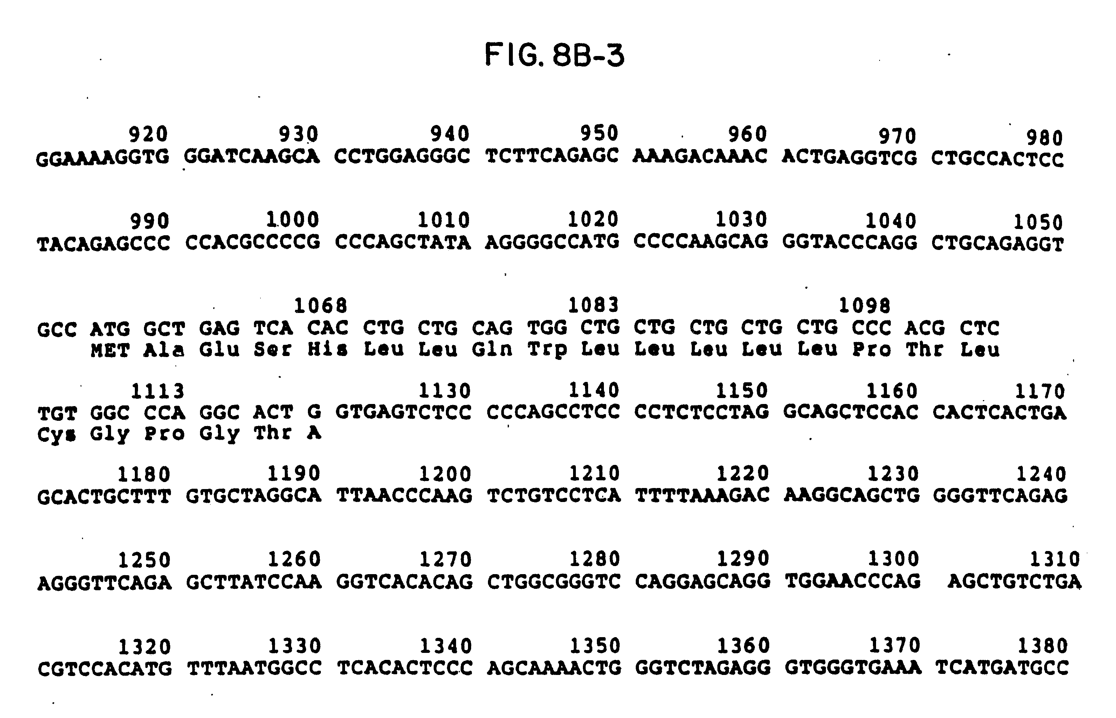

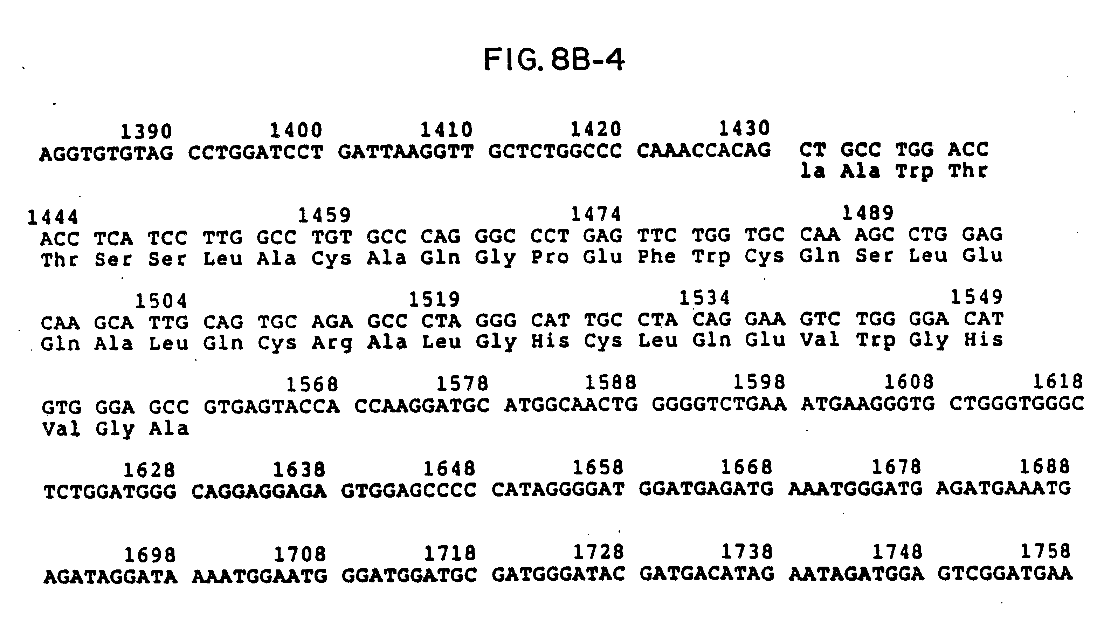

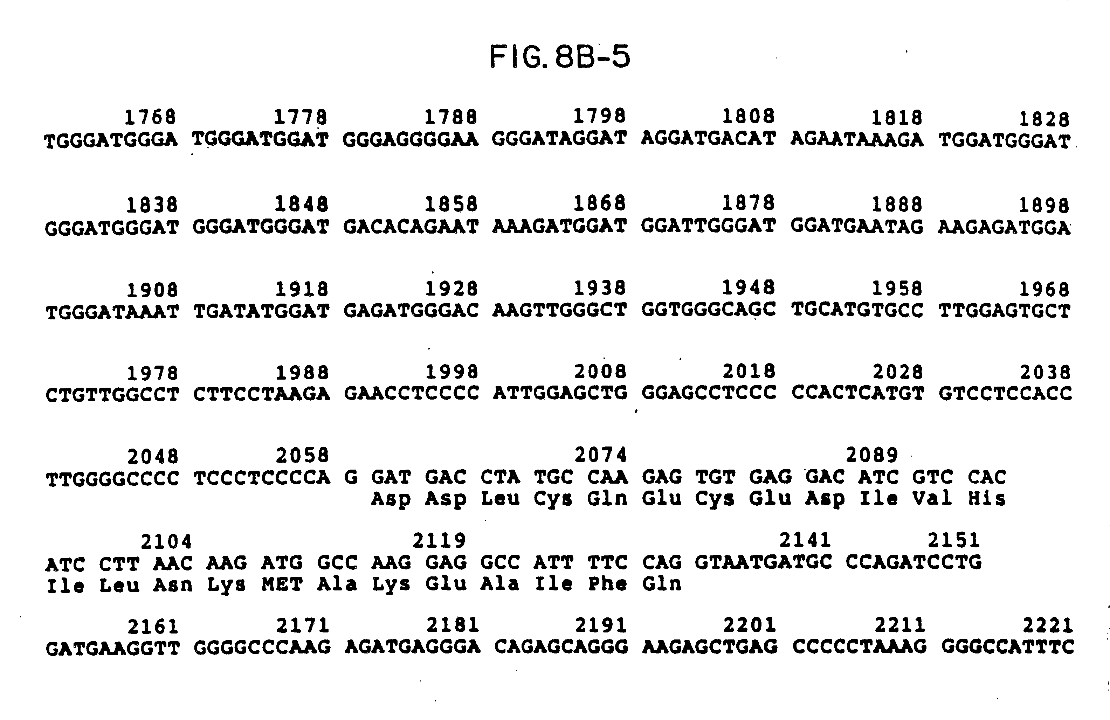

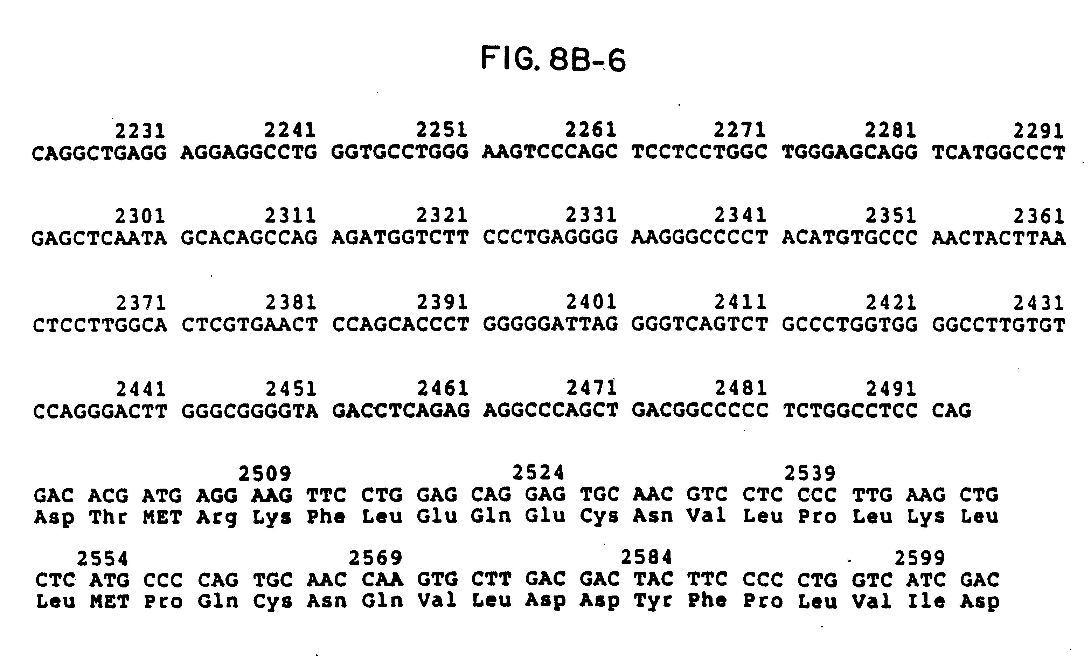

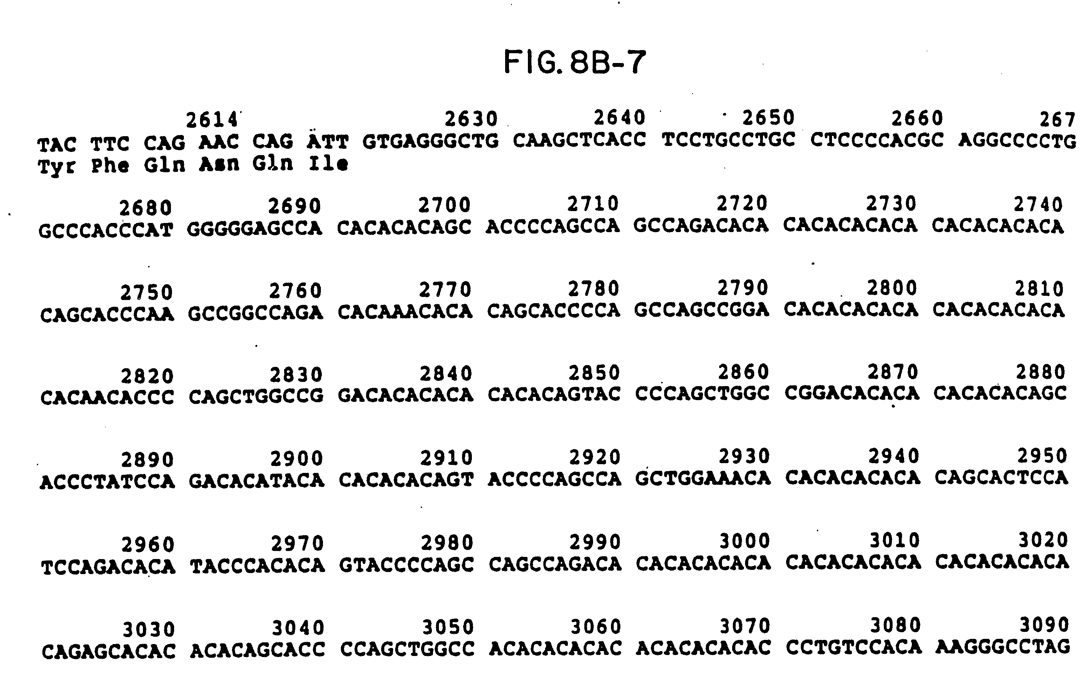

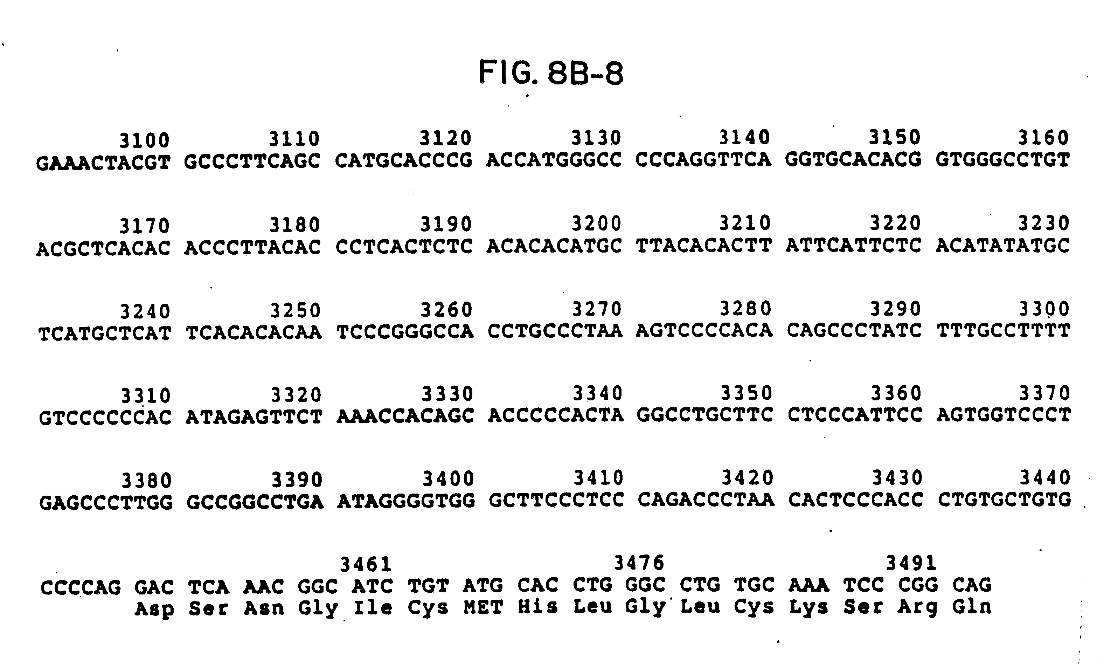

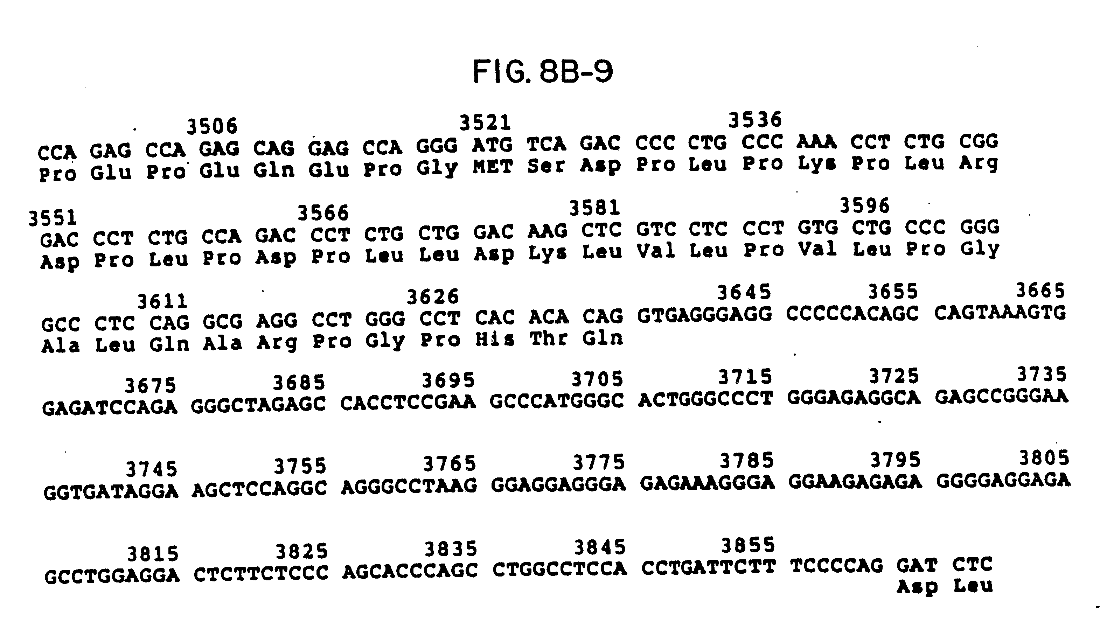

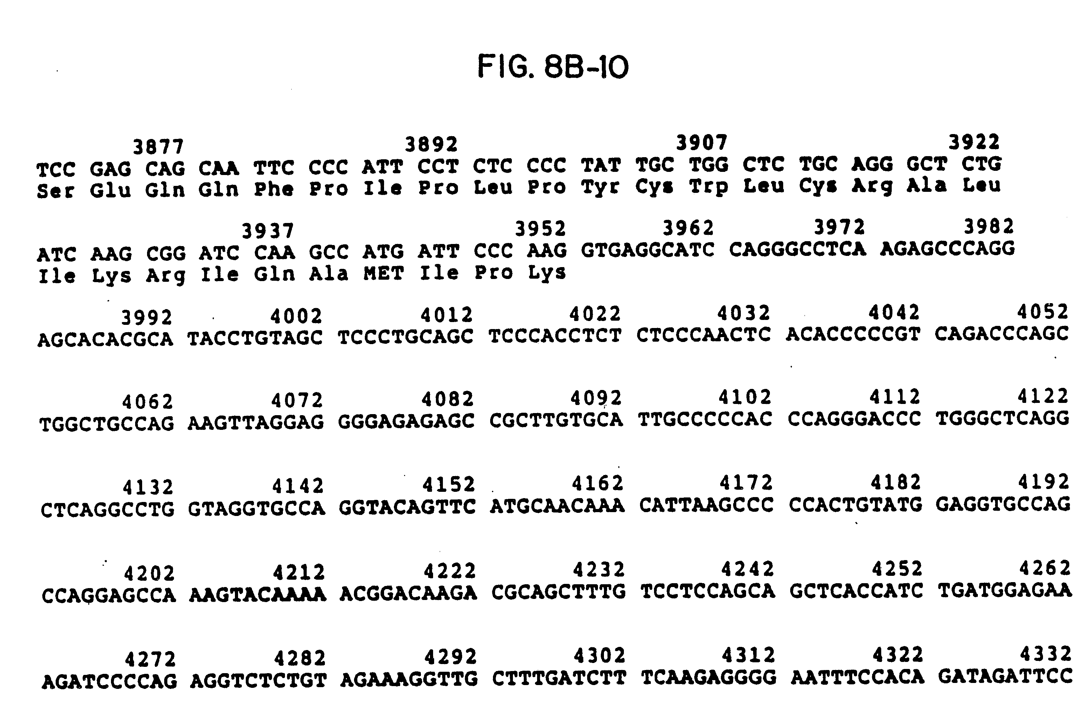

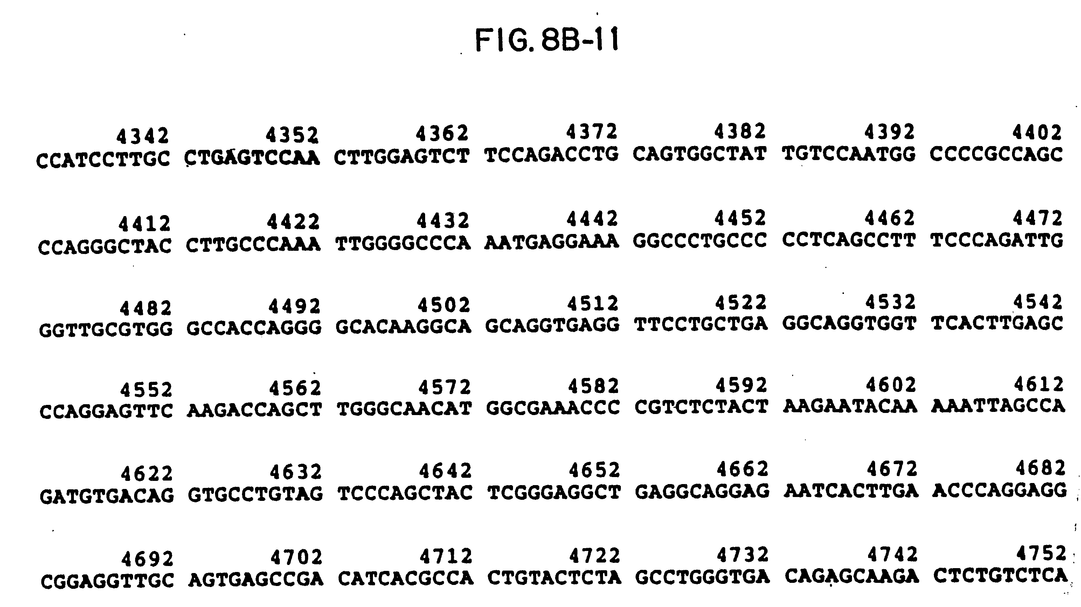

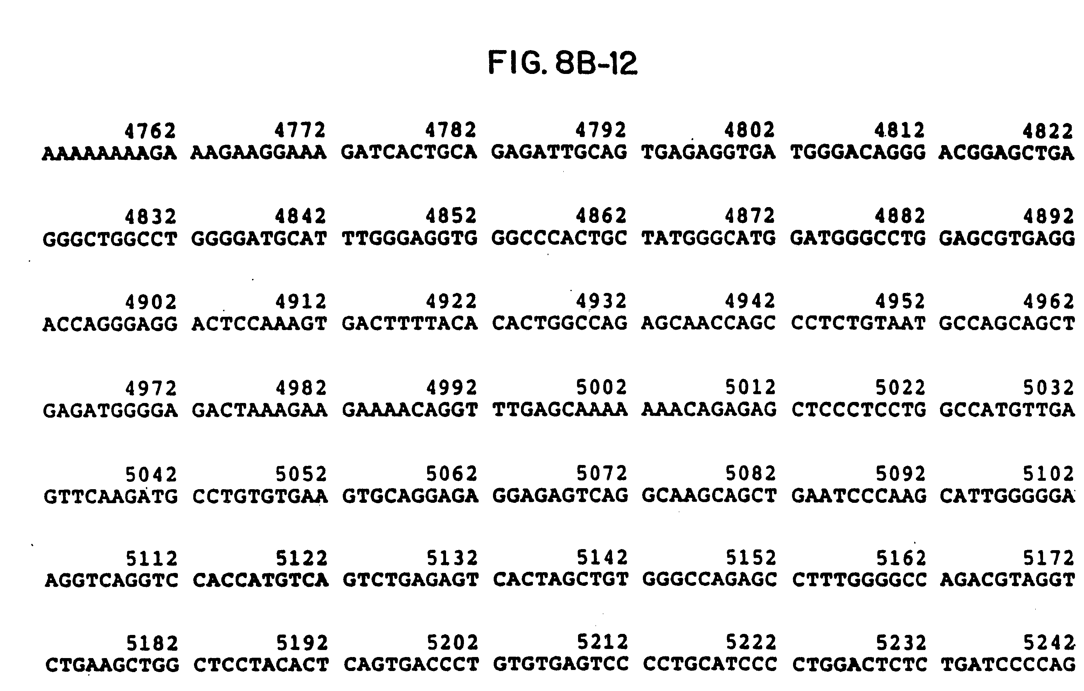

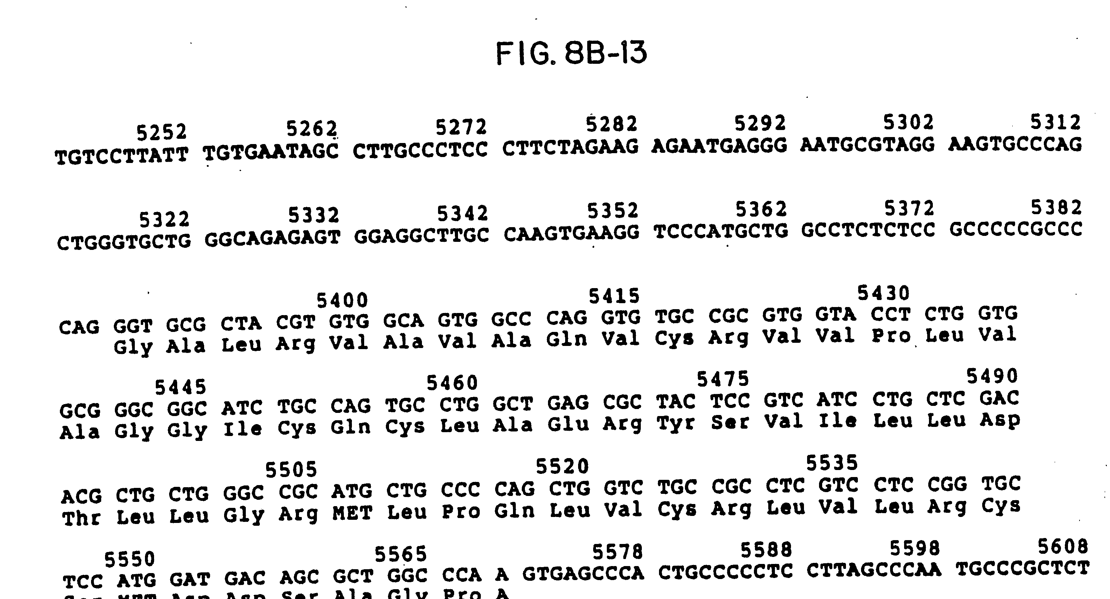

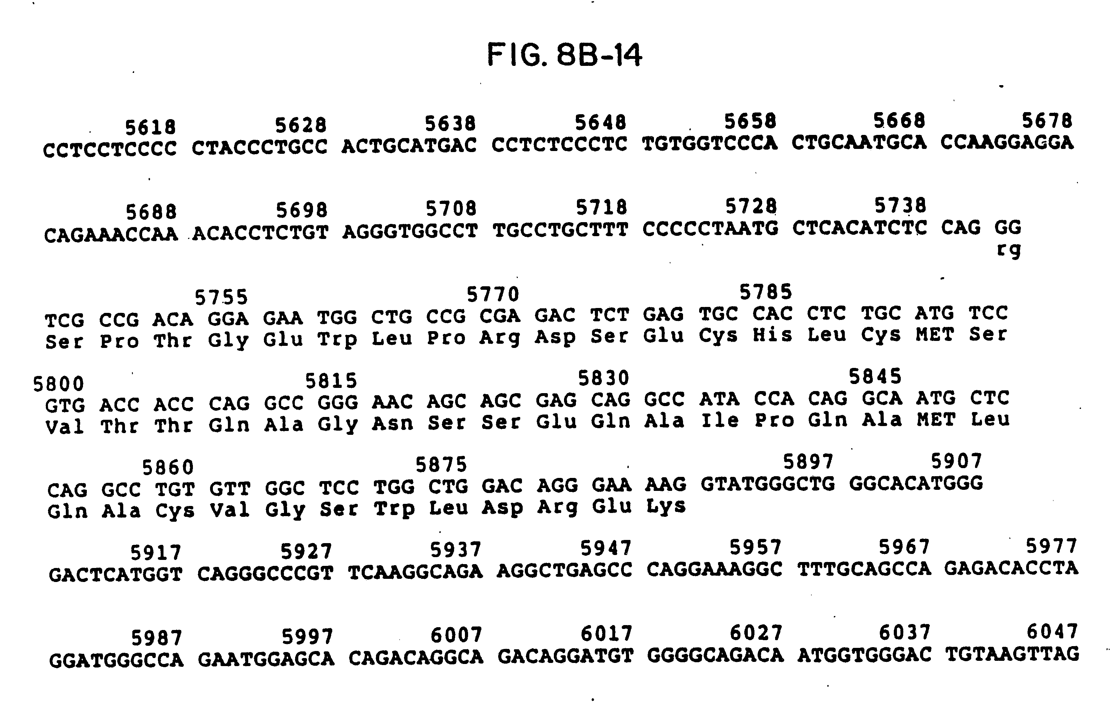

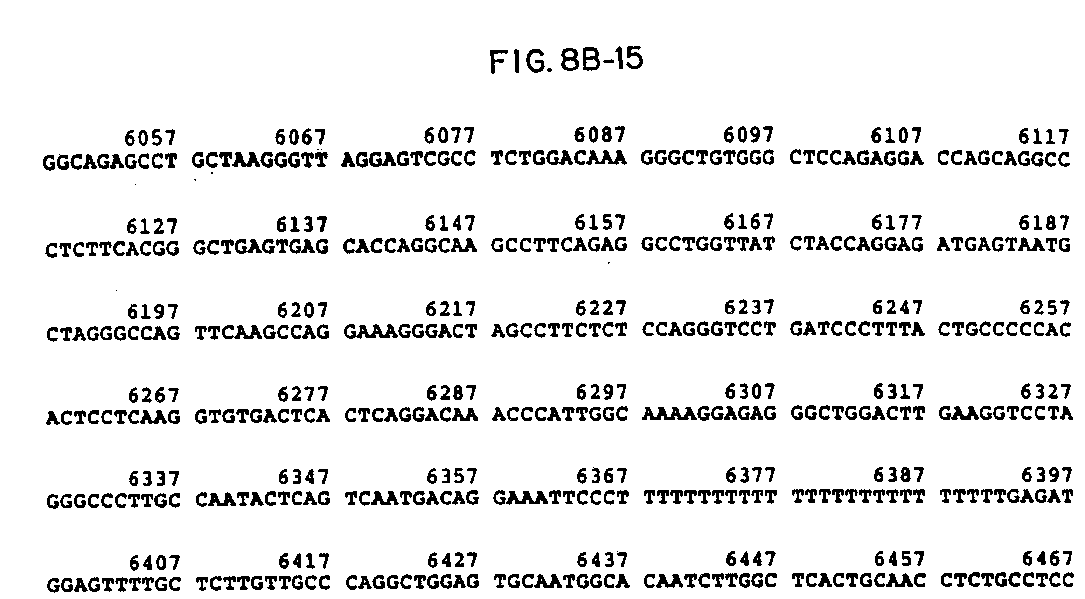

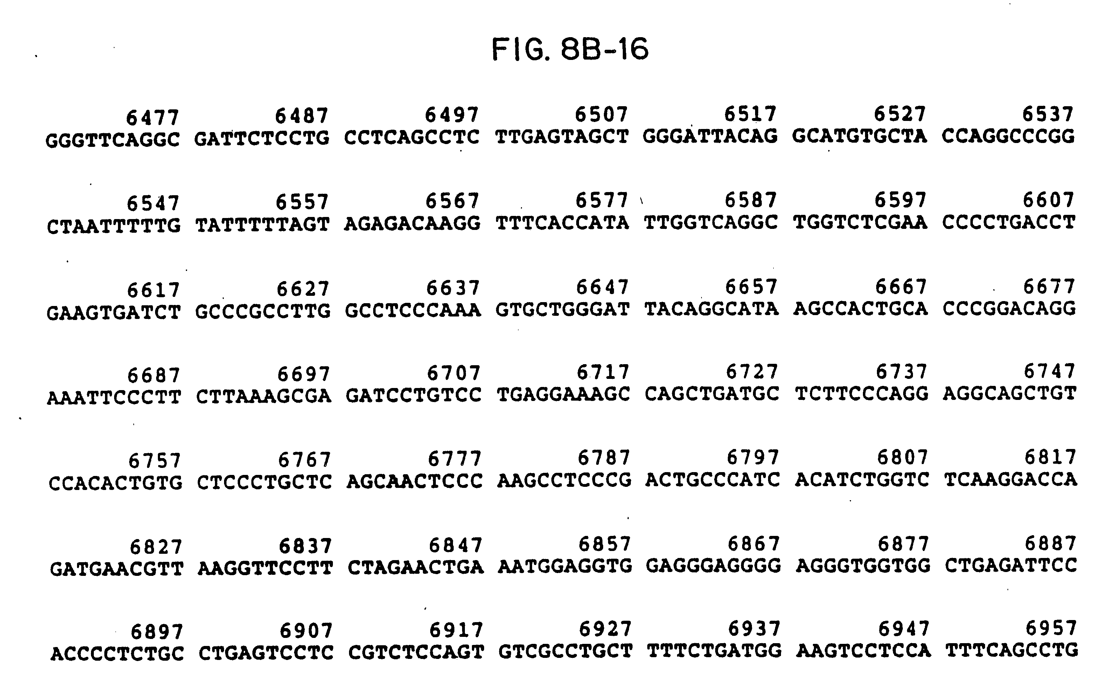

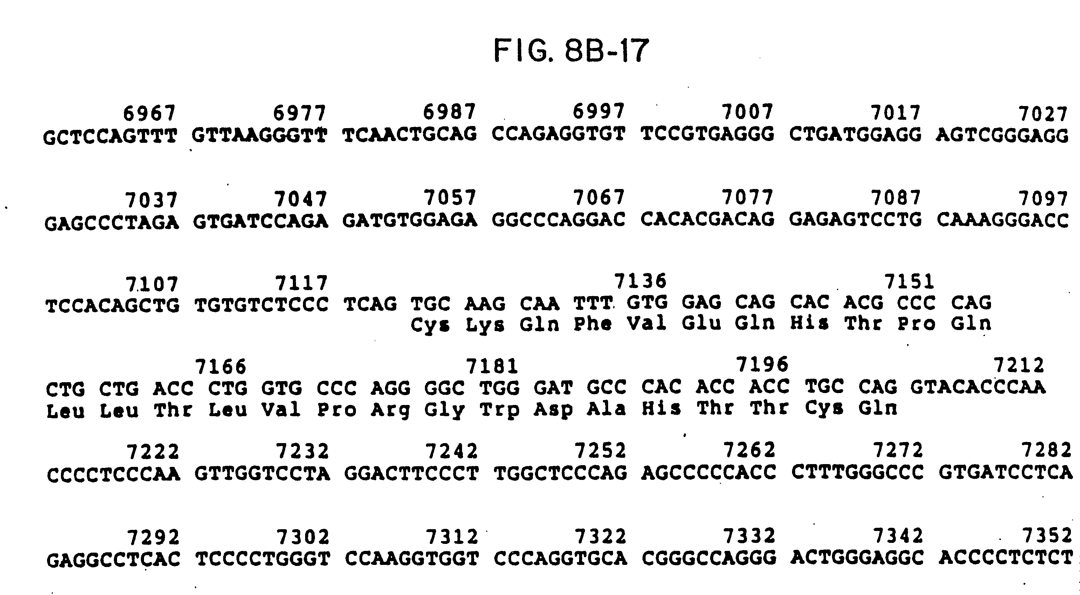

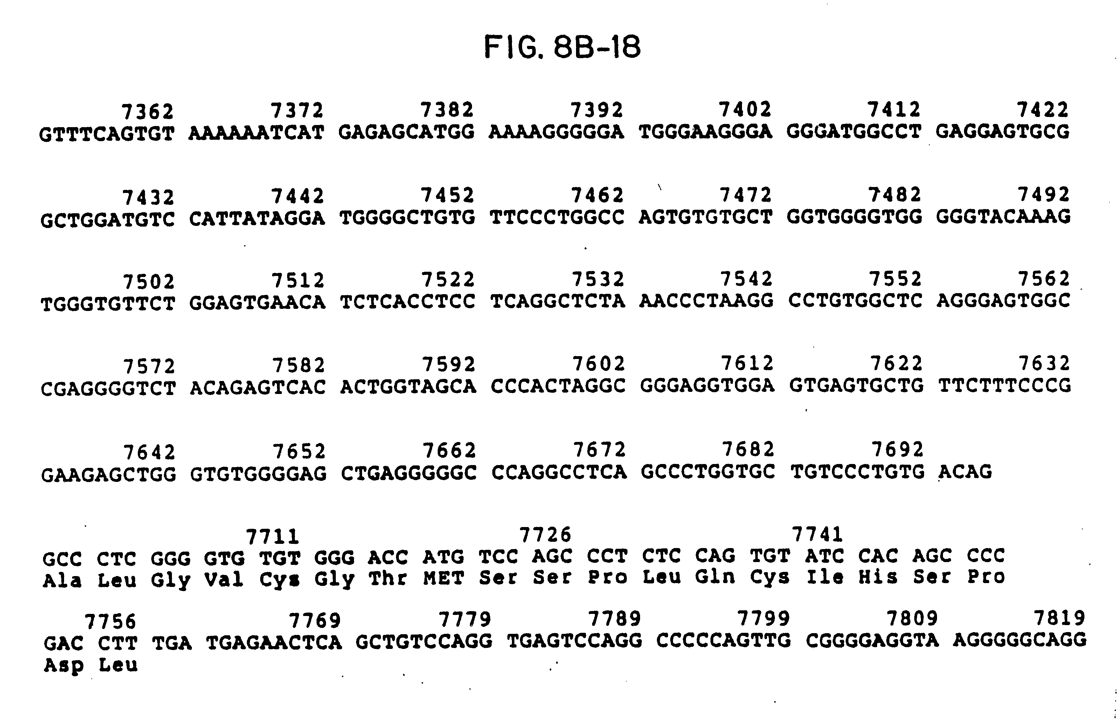

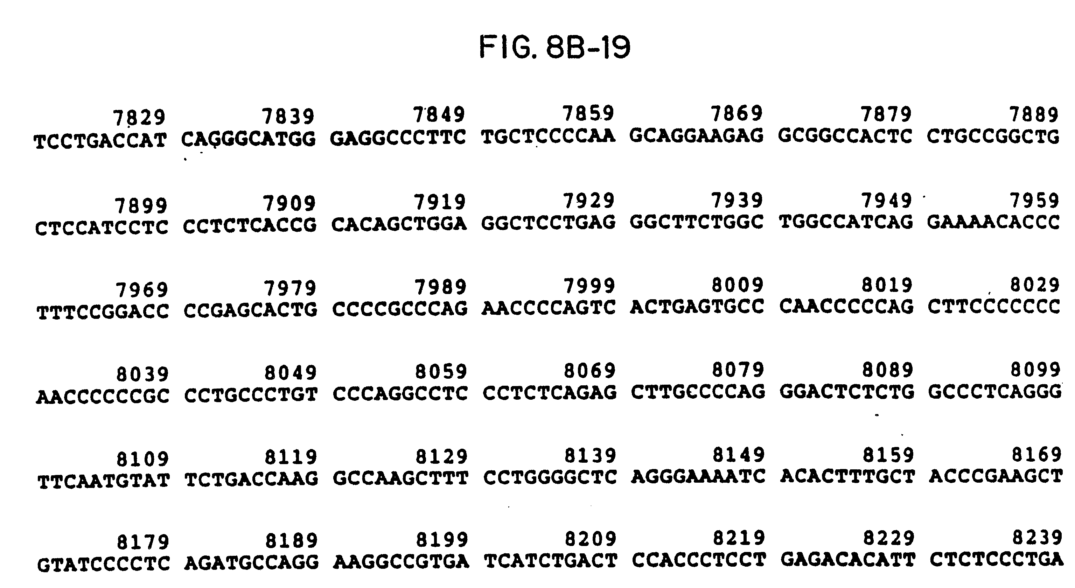

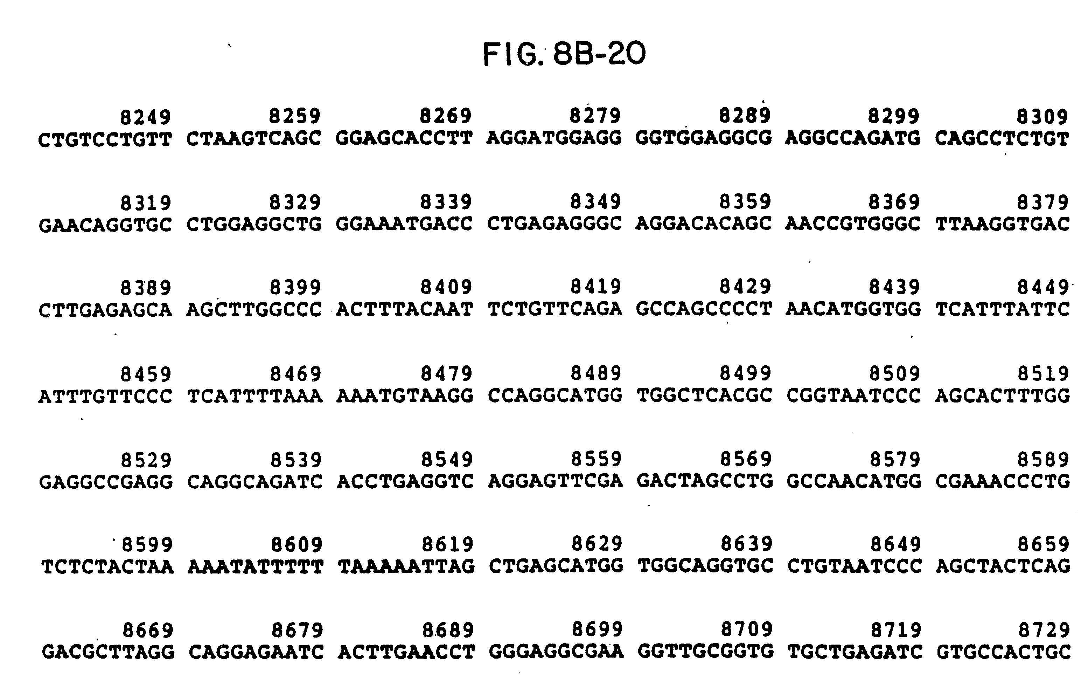

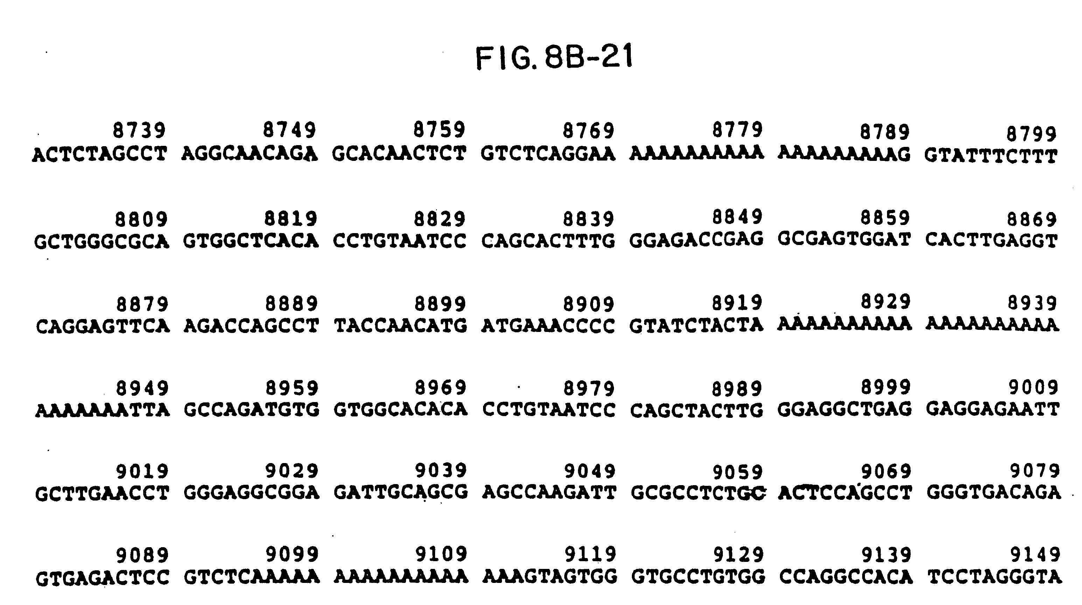

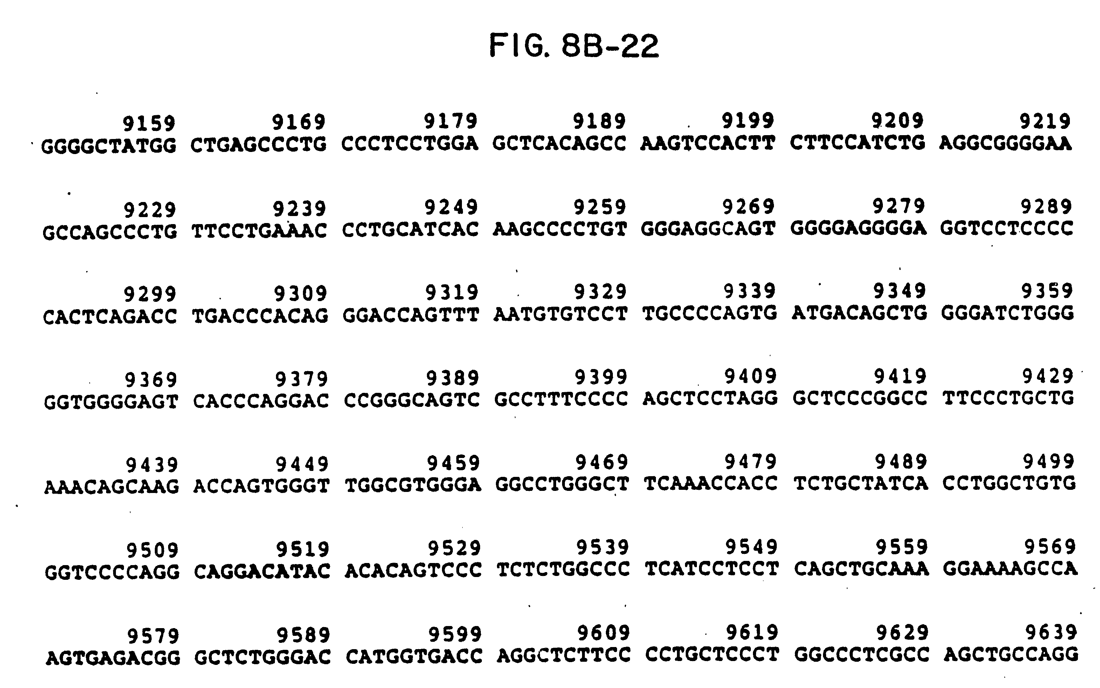

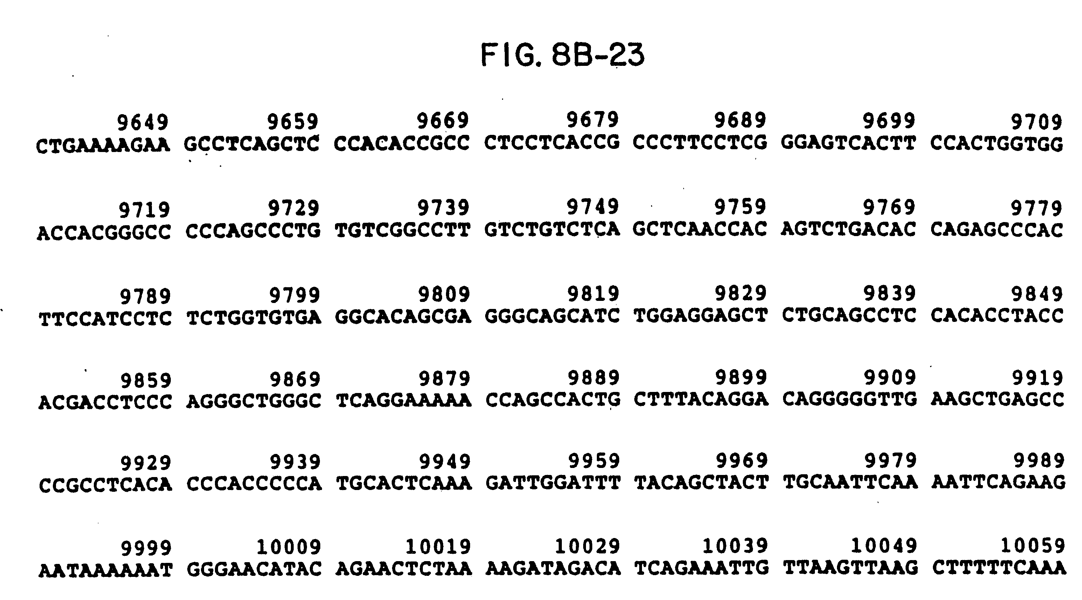

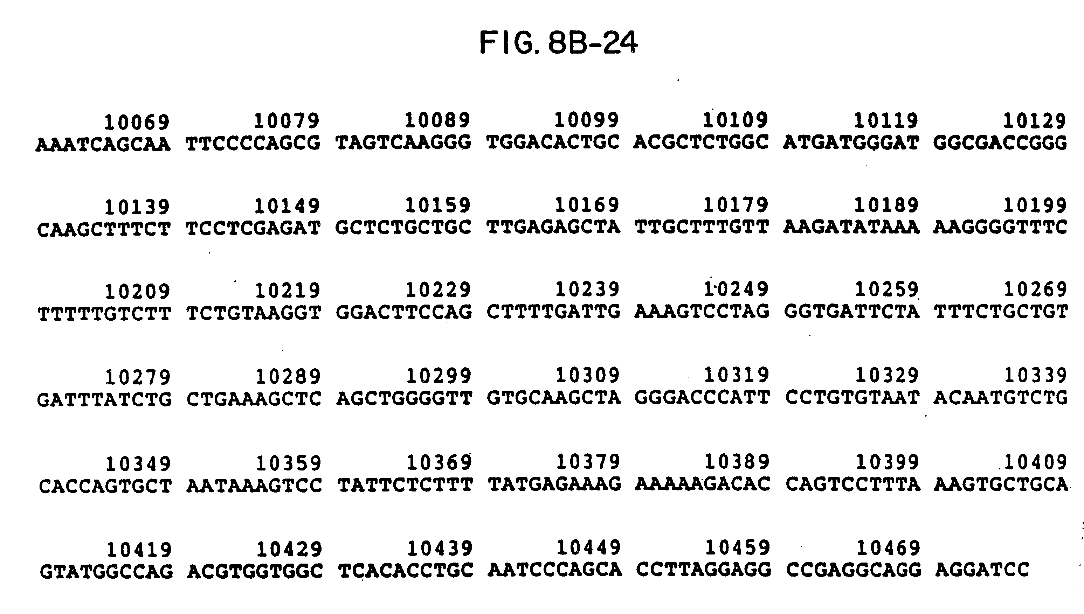

Twelve independent clones containing the SAP(Phe) gene were isolated, purified, and characterized by restriction analysis and Southern hybridization. Characterization by these methods revealed that all 12 clones encoded the same gene or portions of the same gene. One of these clones was subjected to sequence analysis as described in Example 8 and found to contain the entire SAP(Phe) coding region within approximately 10.5 kilobases. This clone also contained approximately 2 kb of 5′ flanking region and approximately 3 kb of 3′ flanking region. FIG. 8A shows the structure of the SAP(Phe) gene. FIG. 8B shows a nucleotide sequence for genomic SAP(Phe) DNA obtained by standard sequencing techniques known to those skilled in the art.

In FIG. 8A; B = BamHI; E = EcoRI; H = HindIII; S = SacI; filled areas are translated regions of exons and cross hatched areas are untraslated regions of exons.

It is believed that genomic sequences further the ability to clone and express SAP(Phe) genes, e.g. by providing regulatory sequences which may be particularly useful for regulating expression.

## EXAMPLE 12

### SAP(Val) Genomic Clones

A SAP(Val) cDNA designated 334.2 was labelled with α[$^{32}$P]dCTP using a nick translation reagent kit (Bethesda Research Laboratories Life Technologies, Inc., Gaithersburg, MD). This probe was used to screen a human lymphocyte genomic library in λEMBL3 (Clontech Laboratories, Inc., Palo Alto, California). Approximately 2 × $10^6$ clones were screened at an initial plating density of 30,000 pfu per plate. The filters were screened in duplicate. The second and third screens were performed at 10 fold and 100 fold lower dilution, respectively. Phage DNA was prepared essentially as described by Maniatis et al., supra, 371-372.

To identify genomic fragments containing SAP(Val) sequences, cDNA described above was used in Southern blot analysis of genomic DNA after digestion with various restriction endonucleases. Southern blot analysis was performed using random primer labelled probes labelled with the random primer labelling technique (Pharmacia, Inc., Milwaukee, Wisconsin) hybridized with the DNA in 5 × SSC, 5 × Denhardt's solution, 0.1% SDS at 65° C. The nitrocellulose filters were washed twice (5 minutes each) at room temperature in 2 × SSC, 0.1% SDS, then twice in 2 × SSC, 0.1% SDS at 65° C. and four times in 0.2 × SSC, 0.1% SDS at 65° C. (20-30 minutes per wash). DNA fragments were subcloned into M13 vectors for dideoxy sequencing.

An oligonucleotide based on the overlapping sequence was synthesized and utilized to locate this 5′ exon from genomic DNA encoding SAP(Val): 5′A-G-C-A-A-G-A-T-G-G-A-T-G-T-G-G-G-C-A-G-3′. Sequence from the 5′ coding region of genomic clone λVG519 overlapped exactly with the direct RNA sequence and the 5′ cDNA sequences. This genomic sequence is in the first exon of the SAP(Val) gene located 30 base pairs downstream from a consensus TATAA sequence further identifying the 5′ untranslated region of the SAP(Val) RNA.

The 5′ untranslated portion of the mRNA contains a potential initiation site which fits the criteria for a mammalian ribosomal binding site. There are two potential ATG start sites (bases 26 and 53) at the 5′ end of the mRNA, the more 3′ one most closely meeting selection criteria. The 3′ end of several cDNAs demonstrates a polyadenylation addition sequence predicting the end of SAP(Val) RNA. Two distinct classes of cDNA were isolated which encode the SAP(Val) active region, the predicted mRNA differing in the coding region 3′ to the active $M_r$ = 4,000-7,000 peptide, wherein a deletion of 18 base pairs may result in two distinct polypeptide precursors differing by six amino acids. However, this deletion does not arise from nucleotide differences in the 2 types of genomic clones isolated for SAP(Val). Both clones contain the 18 base pair sequence deleted in one class of cDNA. This deletion may arise by alternative splicing, as there are 2 sets of sequences which conform to intron-exon splice sites just before and after the 18 base pairs in the genomic clones.

The deleted sequence is located at the start of exon 5 and is preceeded by six nucleotides (TTCCAG) which are also found at the end of exon four. Sequences at the 5′ proximal end of exon five show two sets of sequence that conform to an intron-exon splice site consisting of a 10-15 base pair polypyrimidine tract followed by the dinucleotide AG, signaling the end of intronic sequence. The base pairs (CTCACTTCCTACATTCC) comprise a 17 base pair tract preceeding the AG of the major cDNA species. A second sequence (TGCTCTCTGC) preceeds the AG at the end of the deleted 18 base pair sequence and may represent an alternate splice site to account for the two observed SAP(Val) cDNA clones. If splicing occurs by a scanning mechanism, then this downstream alternate site may be less favored and result in the minor species lacking the 18 bp of cDNA. Only two of the nine SAP(Val) cDNAs detected during cDNA cloning contained this 18 base pair deletion. In summary, it appears that the 18 bp cDNA deletion arises from alternative splicing. It remains possible that the 18 base pair deletion resides in an allele of the SAP-(Val) genes not detected in the present study.

Restriction endonuclease mapping of twelve clones identified by hybridization with SAP(Val) cDNA clone 334.2 demonstrated two distinct patterns of DNA fragments. Seven clones were represented by clone λVG519 and five clones were represented by clone λVG524. Restriction endonuclease fragments containing SAP(Val) coding regions were identified by their hybridization with cDNA clone 334.2. A distinct 1.8 kb HindIII/EcoRI hybridizing fragment was identified in clone λVG519, while, a 4 kb HindIII hybridizing fragment was identified in clone λVG524. Both bands were reduced by 250 bp after digestion with ApaLI which cuts the SAP(Val) cDNAs in the region encoding the active hydrophobic peptide. Thus, these preliminary analyses delineated the restriction endonuclease fragments encoding SAP(Val) and were consistent with the restriction analysis of the cDNA clones encoding SAP(Val).

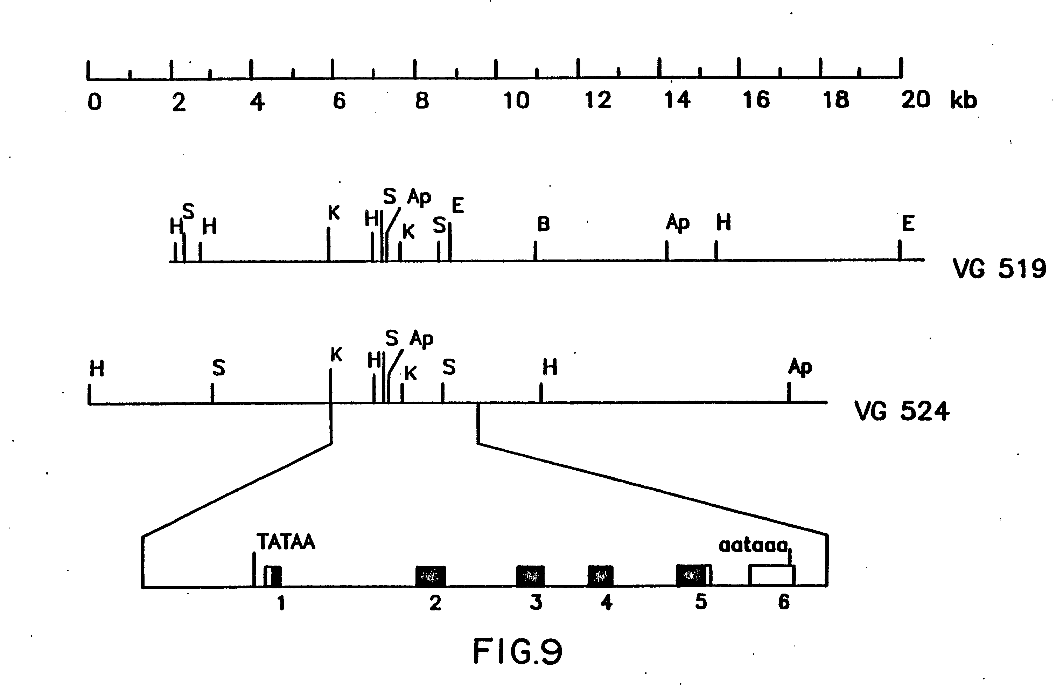

The restriction map and organization of the two SAP(Val) clones are represented in FIG. 9. In FIG. 9, The first two lines are restriction endonuclease maps of λVG519 and λVG524 representing two classes of restriction enconuclease patterns identified from the genomic clones. The third line is expanded from the second line and represents sequences in and near the gene for λVG524. Overbrackets identify sequences hybridizing to the SAP(Val) cDNA probe (exons 2-5). An ApaLI restriction site identifies the polyvaline domain. Restriction enconuclease sites are represented by the following letters: A = ApaLI; H = HindIII; B = BamHI; E = EcoRI; K = KpnI; and S = SmaI. Line C indicates the relative position of exons of SPL-(Val). Open blocks indicate untranslated exons; dark blocks indicate translated exons.

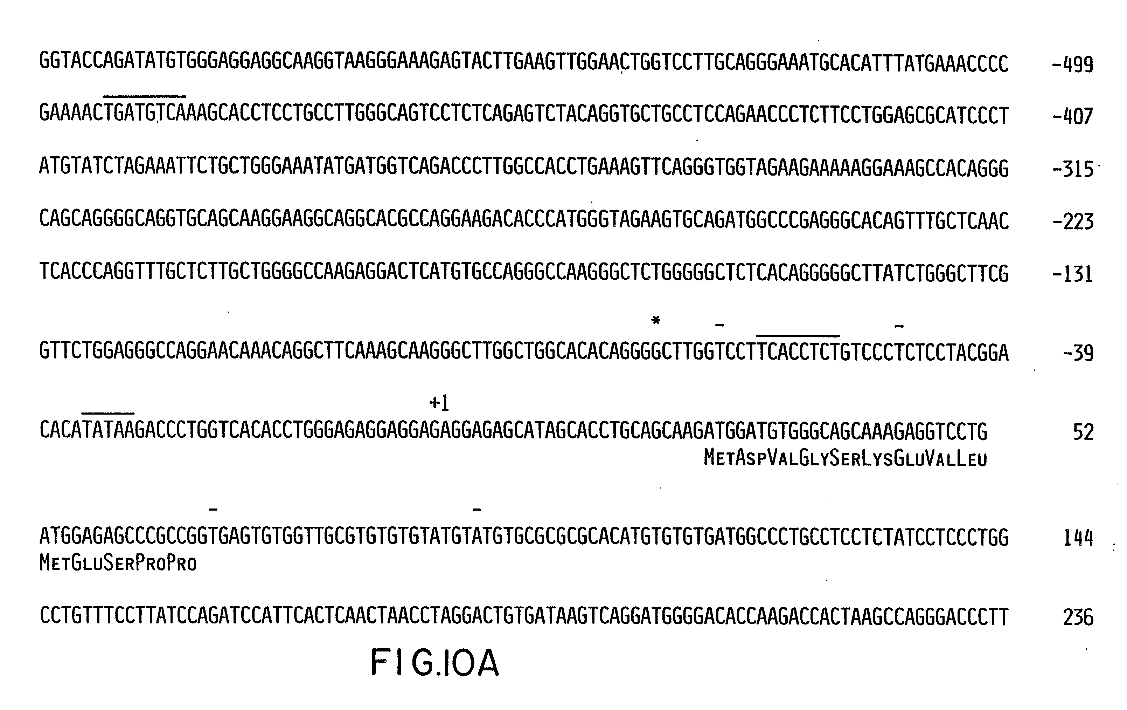

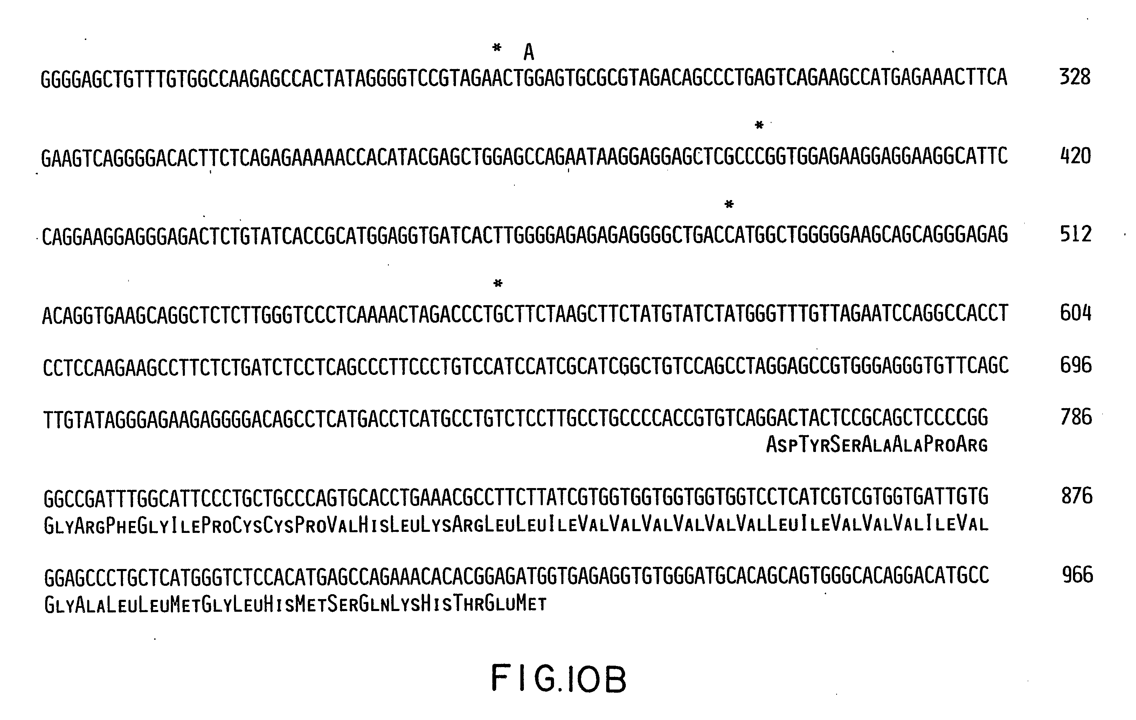

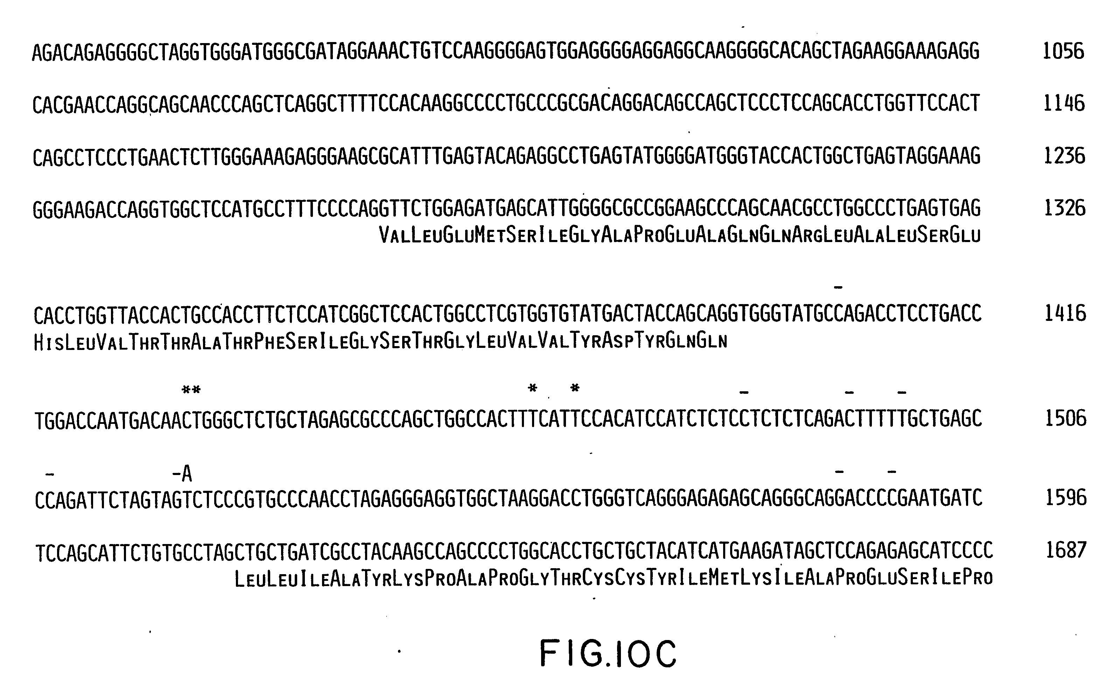

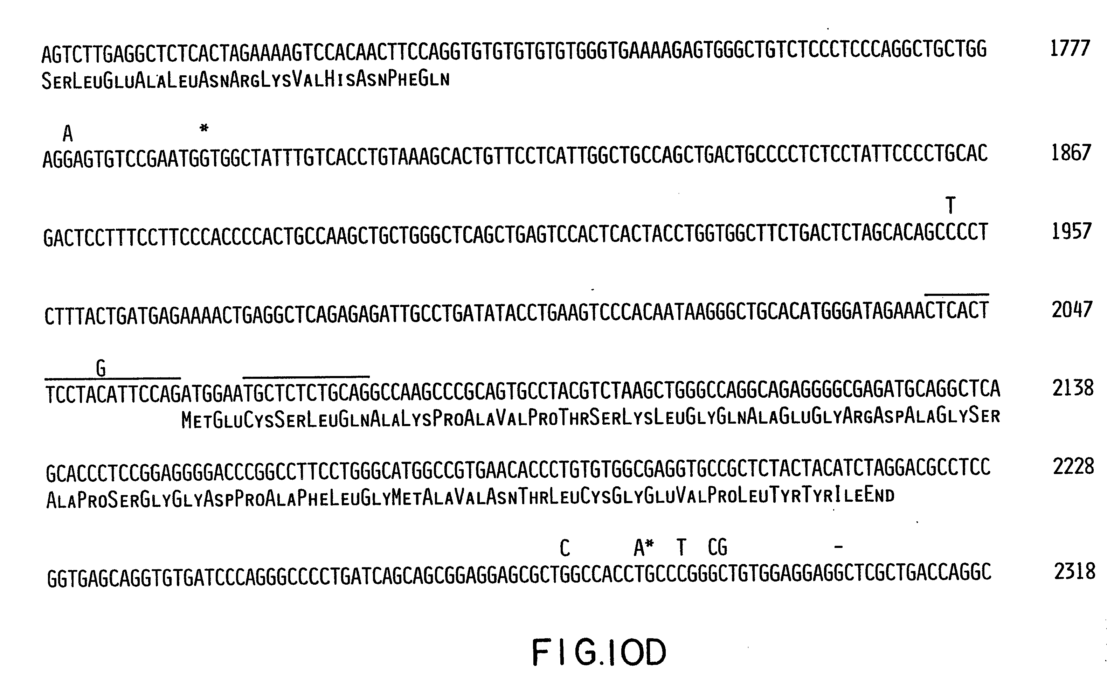

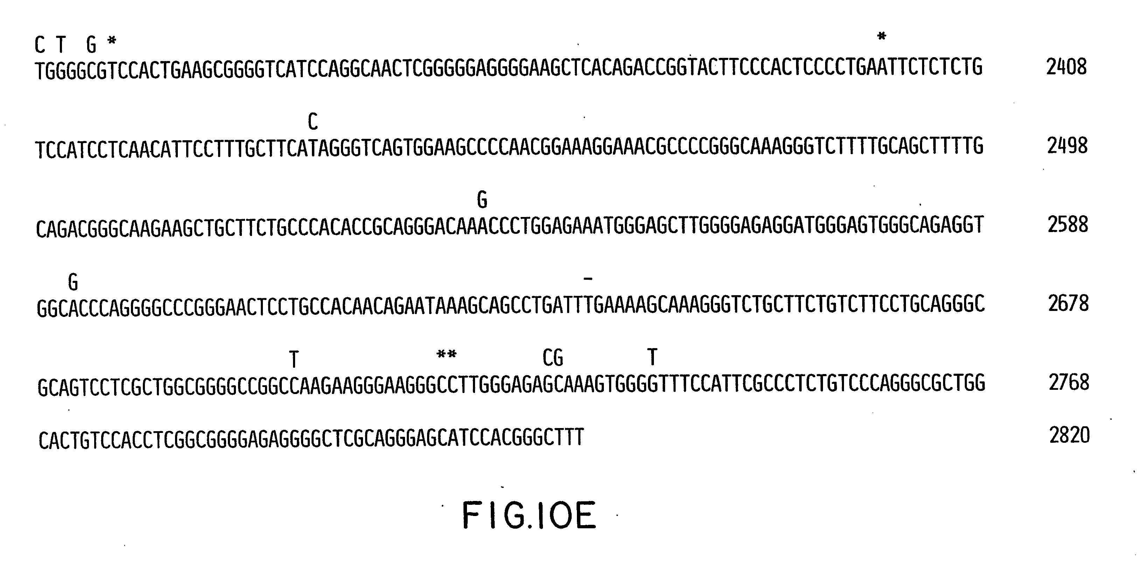

The flanking regions of the genomic clones contained restriction site differences identified with SmaI, HindIII, and BamHI restriction enzymes. Restriction maps of the two classes of SAP(Val) genes were readily distinguishable by this analysis. Both SAP(Val) genes were composed of 6 exons and 5 introns from TATAA to the end of the 6th exon. The two SAP(Val) genes demonstrated few but clear differences in nucleotide sequence, as illustrated in FIG. 10.

In preparing FIG. 10, nucleotide sequence analysis was performed as described by Brunner et al., Biochemistry, 25, 5028-5035 (1986). Base pairs are numbered with +1 indicating the transcription initiation site determined as described above. Nucleotides 3′ to this site are labelled as (+) and 5′ nucleotides are labelled as (-). The overlined sequence TGACGTCA represents a potential cAMP recognition sequence; the sequences TCACCTCT and TATAA representing consensus promoter and sequences are overlined. Alternative donor/acceptor splice sequences are overlined at the 5′ end of exon 5. The differences in sequence between λVG519 and λVG524 are shown by asterisks, dashes, and letters above the λVG519 sequence representing nucleotide insertions, deletions or substitutions, respectively.

Part of the first, fifth and the entire sixth exons of SAP(Val) are untranslated. The $M_r = 22{,}000$ polypeptide precursor is encoded by exon 2, 3, 4, and 5. The most hydrophobic region of the peptide (beginning $NH_2$-Ile-Pro-Cys-Cys-Pro-Val...) is located within exon two which encodes a peptide of 44 amino acids.

Because complete cDNAs encoding SAP(Val) were not identified, RNA directed sequencing was performed to identify the nucleotide sequence from the available cDNA sequence to the transcription initiation site. A strong reverse transcriptase stop was detected in the RNA-directed sequence analysis. It is inferred that this demonstrates the site of transcription initiation.

To identify the 5′ untranslated exon and upstream flanking sequences of the SAP(Val) genes, an oligonucleotide (3′-CAGCCAGATGGATGTGGGCAG-5′) spanning the 5′-most cDNA and eight base pairs of extended RNA directed sequence was synthesized and used to analyze Southern blots of λVG519 and λVG524. Identical restriction fragment patterns were detected in both classes of genomic clones with this oligonucleotide after digestion with KpnI and KpnI/HindIII. The 1.8 kb KpnI and 1.1 kb KpnI/HindIII fragments were sequenced and found to include nucleotide sequences from the 5′-most cDNA clone (clone RJ2-1) and the 5′ nucleotide sequence derived from mRNA-directed sequence analysis.

A consensus promoter sequence TATAA was located 30 base pairs 5′ to the predicted transcription initiation site in both clones. As used throughout the present application, distances are given with respect to the location of the 3′ end of the sequence being discussed. The sequence TCACCTCT nearly matches a consensus eucaryotic promoter sequence TCAATCT in 6 of 7 nucleotides Breatnach et al., Ann. Rev. Biochem., 50, 349 (1981). It was located 55 nucleotides upstream of the transcription initiation site. A sequence, TGATGTCA, was located 504 base pairs upstream from the transcription initiation site and matches the sequence TGACGTCA in 7 of 8 base pairs. This latter element comprises a sequence previously associated with cAMP responsivity [Montminy et al., Proc. Nat'l Acad. Sci. (USA), 83, 6682-6686 (1986)]. Consensus sequences previously recognized as corticosteroid-responsive elements were not detected in these clones, although glucocorticoid enhanced SAP(Val) expression in organ culture of fetal lung.

The predicted protein sequence of the exons encoded by the λVG519 and λVG524 were identical. No differences were observed in the first five exons of the SAP(Val) gene and three nucleotide differences were detected in the sixth exon, which is untranslated. A high degree of homology was observed even in the introns of the SAP(Val) genes which varied by only 1%.

Hydropathy analysis of the predicted SAP(Val) precursor peptide demonstrates that the hydrophobic, potentially membrane-associated domain of the $M_r = 22,000$ precursor protein is contained within the second exon of both SAP(Val) genes. The predicted peptide domain derived from exons three and four are rich in charged amino acids which are not compatible with the amino acid composition of the SAP(Val) preparations. The location of the C-terminus of SAP(Val) peptide is suggested by its migration with markers of $M_r = 4,000\text{-}7,000$ and by its hydrophobic properties, including its association with lipids and its solubility in organic solvents, all of which are consistent with its derivation primarily from the peptide encoded by the second exon. Proteolytic processing in both amino and carboxy termini may account for the generation of the smaller peptide detected in pulmonary surfactant.

No homology was noted between the 5′ region of SAP(Val) and a published SAP-35 genomic clone.

The nucleotide sequence of λVG519 and λVG524 were entirely conserved in the amino acid coding region. The genomic sequences were identical to the SAP(Val) cDNA except for a single nucleotide difference in exon 5 in which leucine is encoded by CTG rather than the TTG observed in the cDNAs. Nucleotide differences were noted in only those exons encoding the 3′ untranslated regions. Nucleotide sequence difference frequencies (1-2%) were similar in both introns and the 5′ and 3′ flanking sequences available for analysis (approximately 500 base pairs from the TATAA). The 5′-most fragment of λVG519 (600 bp HindIII fragments) was not present in λVG524 by Southern blot hybridization, demonstrating a difference in flanking sequence in that region.

The nearly complete conservation of the nucleotide sequence in the exons, the small divergence in the introns, and the differences in restriction mapping of the 3′ and 5′ regions are most consistent with the interpretation that the SAP(Val) genomic clones represent two distinct gene loci encoding SAP(Val). However, these differences may be due to allelic variation.

The predicted amino acid sequence of the entire SAP(Val) precursor was deduced from the cDNA clones, RNA sequencing and the genomic DNA. The precursor comprises 188 or 197 amino acids depending on assignment of the N-terminal methionine (or 182 or 191 with the deletion) representing a 21,000 Dalton polypeptide. The size of the predicted polypeptide is consistent in size with the hybrid selected translation product of $M_r = 22,000$. There was no discernable signal peptide at the amino terminus and the precursor polypeptide contain no asparagine-linked glycosylation sites, contrasting with the SAP-(Phe) precursors which contain one or two potential asparagine-linked glycosylation sites. The SAP(Val) peptide begins at $Gly_{25}$ or $Ile_{26}$ and the domain including amino acids $Leu_{37}$ to $Ser_{61}$ is compatible with a membrane-associated or spanning domain of 25 amino acids. This region contains the repeated valine residues. The precise C-terminus of SAP(Val) has not been identified directly and numerous attempts to isolate proteolytic or CNBr fragments of the canine or bovine proteolipid have been unsuccessful.

However, NMR studies of bovine SAP(Val) support a tentative conclusion that the C-terminus is His-Thr, i.e. that SAP(Val) may comprise Gly or Ile to His(65) for a total of 40 or 41 amino acids.

The N-terminal amino acid sequences of the bovine and canine proteolipid preparations was greater than 90% SAP(Val) in multiple determinations. The predicted amino acid sequence of an $M_r = 4,000\text{-}7,000$ peptides from the human cDNA predicts a hydrophobic peptide lacking in phenylalanine and tyrosine. In contrast, tyrosine and phenylalanine are present in the amino acid sequence of the small molecular weight hydrophobic surfactant protein, human SAP(Phe). Lack of phenylalanine and tyrosine also distinguishes SAP(Val) from SAP(Phe) and from small molecular weight surfactant proteins previously reported.

It is believed that genomic sequences further the ability to clone and express SAP(Val) genes, e.g. by providing regulatory sequences which may be particularly useful for regulating expression.

EXAMPLE 13

SAP(Val) Poly($A^+$) RNA Sequencing

SAP(Val) cDNA clones isolated by olignucleotide hybridization were incomplete on the 5′ and 3′ ends. Additional clones were isolated using SAP(Val) cDNA for the hybridization probe. Several were found to be complete on the 3′ end, but none on the 5′ end. Two distinct classes of SAP(Val) cDNA were detected by sequence analysis, differing by the absence of 18 nucleotides in the 3′ coding region of some cDNAs. The complete 5′ sequence was determined by direct sequencing of adult human lung poly(A)$^+$ RNA and by SI nuclease mapping of a 5′ fragment from a SAP(Val) genomic clone as described for SAP(Phe). The complete sequence including the 5′ end identified by these two methods is shown in FIG. 5. The SAP(Val) transcript encodes a precursor protein of approximately 21 kilodatons.

## EXAMPLE 14

### Chromosomal Location of SAP(Val) Gene

A [$^{32}$P]-labeled SAP(Val) cDNA clone was hybridized to DNA obtained from the mouse-human chromosomal panels. The [$^{32}$P]-labelled SAP(Val) clone was hybridized to mouse-human chromosomal hybrids containing all human chromosomes as previously characterized. Hybridization was only observed with hybrids containing chromosome 8.

## EXAMPLE 15

### SAP(Val) Northern Blot Analysis

Poly (A)$^+$ RNA was prepared and isolated by oligo (dT) cellulose chromatography from a fetal lung at 19 weeks gestation and human adult lung. Adult and fetal lung tissue was homogenized in buffer containing 4 M guanidine thiocyanate, 0.5% N-lauroyl sarcosine, 20 mM sodium citrate, 0.1 M $\beta$-mercaptoethanol and 0.1% antifoam A. RNA was extracted by centrifugation through a cushion of 5.7 M cesium chloride [Hsu et al., J. Histochem. Cytochem., 29, 577-580 (1981)]. The RNA pellet was dissolved in water, extracted with phenol and chloroform, and precipitated with ethanol. The amount of RNA in an aqueous solution was determined by optical density at 260 nm.

RNA (5$\mu$g) was separated on 1.2% agarose, 7% formaldehyde gels, transferred to nitrocellulose and hybridized [Weaver et al., J. Appl. Physiol., 61, 694-700 (1986)] with [$^{32}$P]SAP(Val) DNA clone 334.2 (1.4 × $10^6$ cpm/ml, approximate specific activity 4 × $10^8$ cpm/$\mu$g), washed and exposed to Kodak XAR-film at -70 °C overnight.

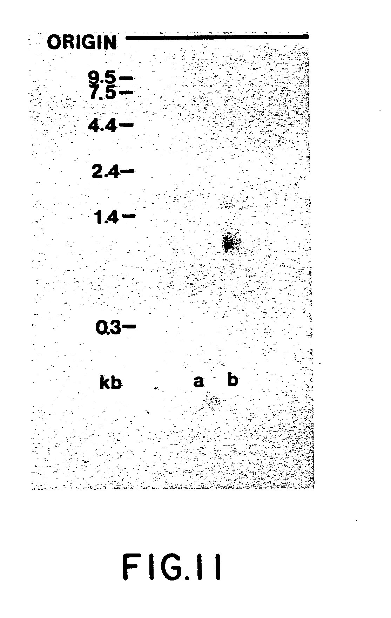

Northern blot analysis of human lung RNA using a SAP(Val) cDNA probe as shown in FIG. 11 detected an approximately 0.9 kilobase RNA, distinct from that of SAP(Phe) or SAP-35 [Glasser, Proc. Nat'l. Acad., Sci. (USA), 84, 4007-4011 (1987); and Whitsett et al., J. Biol. Chem., 262, 5256-5261 (1987)]. SAP(Val) RNA was less abundant in human fetal lung (approximately 19-20 weeks of gestation) than in adult lung. The finding that the RNA for SAP(Val) is developmentally regulated is consistent with the possible role of SAP-(Val) in surfactant function required for perinatal adaptation to air breathing at birth.

## EXAMPLE 16

### Hybrid Arrest Translation

Approximately 5 $\mu$g of EcoRI restricted SAP(Val) cDNA was heat denatured at 100 °C for 10 minutes and hybridized with 5 $\mu$g of human lung RNA for 2 hours at 50 °C in 80% formamide, 10 mM Pipes, (pH 6.4), 0.25 mM EDTA and 0.4 M NaCl. Hybridization was terminated by the addition of 200 $\mu$l $H_2O$ and 25 $\mu$g yeast tRNA. The solution was divided into two samples, one of which was melted at 100 °C for 1 minute followed by rapid chilling, and the other which was preserved in hybrid form. Both samples were precipitated in ethanol and translated in a wheat germ transcription assay (Promega Biotec, Inc.) in the presence of 50 $\mu$Ci [$^{35}$S]-methionine (New England Nuclear). The proteins were immunoprecipitated with anti-bovine surfactant proteolipid antiserum subjected to 11% SDS-PAGE, transferred to nitrocellulose and subjected to autoradiography.

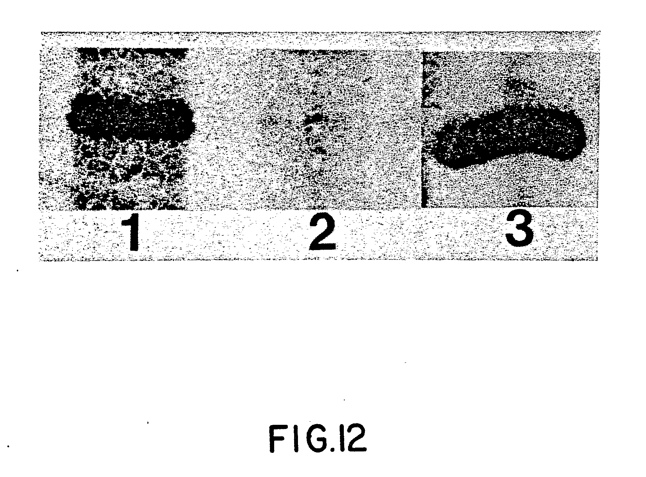

Hybrid arrested translation and immunoprecipitation with antiserum generated against bovine proteolipid resulted in complete arrest of a single $M_r = 22{,}000$ polypeptide, as shown by the autoradiograph gels illustrated in FIG. 12. The $M_r = 22{,}000$ peptide detected by hybrid arrested translation of human lung RNA was consistent with that predicted from the cDNA encoding SAP(Val) further distinguishing it from SPL(Phe) and SAP-35 polypeptide precursors of Mr = 40,000 and $M_r = 26{,}000$ respectively.

## EXAMPLE 17

### Expression of SAP (Val) in E. coli

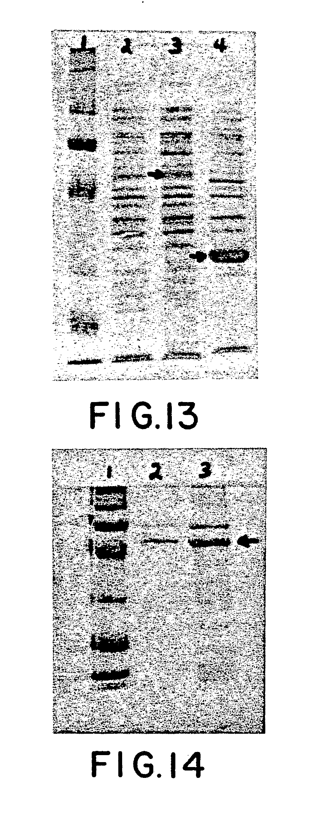

A E. coli expression vector has been developed for expression of heterologous proteins as fusions with the E. coli protein CMP-KDO synthetase (CKS) [Goldman et al., J. Biol. Chem., 261, 15831-15835 (1986)]. This vector was deposited including a SAP(Val) insert as ATCC Deposit No. 67517, on September 29, 1987, with the American Type Culture Collection, 12301 Parklawn Drive, Rockville, Maryland 20852. The plasmid is a derivative of pWMIII[Mandecki et al., Gene, 43, 131-138 (1986)] from which the EcoRI-HindIII fragment containing the promoter, operator, ribosome binding site, and C5A gene have been replaced with corresponding ones for CKS. The CKS gene is under the control of a wild type lac operator and a modified lac promoter, designated lacPT9-D. The -9 position has been changed from G to T, and there is a 1 nucleotide deletion in the spacer region between the -35 and -10 regions. The plasmid also contains the trpA Rho-independent transcription terminator [Christie et al., Proc. Nat'l. Acad. Sci. (USA), 78, 4180-4184 (1981)] at the 3′ end of the CKS gene. In addition, there is a linker region at the 3′ end of the CKS gene which contains multiple restriction sites as well as an in-frame Asp-Pro. The peptide bond between Asp and Pro is acid-labile as described by Landon, Meth. Enzymol., 47, 145-149 (1977). Under induced conditions (1 mM IPTG) when no insert is present, the CKS protein accummulates to levels >50% of the total cellular protein as shown in FIG. 13, lane 4.

In FIG. 13, a 10% SDS-PAGE stained with Coomassie brilliant blue is illustrated in which: lane 1 contained pre stained high molecular weight standards (BRL Life Technologies, Gaithersburg, Maryland); lane 2 contains E. coli total cell lysate (no plasmid); lane 3 contains E. coli total cell lysate with CKS/SAP-(Val) expressed; and lane 4 contains E. coli total cell lysate with CKS alone expressed.

A DNA fragment containing the SAP(Val) cDNA was inserted into the multiple cloning site of this vector at the 3′ end of the CKS gene. The two coding regions are in the same frame, so that expression results in a fusion protein containing the CKS protein and the entire SAP(Val) proprotein. The fusion protein is 48.7 kDal; 27.4 kDal from CKS and 21.3 kDal from SAP(Val). There is one Asp-Pro at the fusion junction between CKS and SAP(Val) and one located 20 amino acids in from the C-terminal end of SAP(Val). When a culture of cells containing this plasmid is grown under induced conditions, the CKS/SAP(Val) protein represents approximately 2-5% of the total protein as shown in FIG. 13, lane 3. The cells may be lysed by a variety of methods, one example being the use of lysozyme, sodium deoxycholate, and sonication as described in Marston et al., Bio/Technolcgy, 2, 800-804 (1984). When lysed by this method, the CKS/SAP(Val) protein is in the insoluble pellet and may be purified away from the bulk of the E. coli proteins. The CKS/SAP(Val) protein represents >60% of the total insoluble protein as shown in FIG. 14, lanes 2 and 3.

FIG. 14 illustrates a 12.5% SDS-PAGE stained with Coomassie brilliant blue in which: lane 1 contains pre stained high molecular weight standards (BRL Life Technologies, Gaithersburg, Maryland) lane 2 contains 1 µl of insoluble protein preparation; lane 3 contains 5 µl of insoluble protein preparation; and the arrow indicates CKS/SAP(Val) fusion protein.

Several conditions for acid cleavage of Asp-Pro bonds have been described by Szoka et al., DNA, 5, 11-20 (1986), and others. One example is the use of 70% formic acid for 24-48 hours at 37° C. When the isolated protein is treated in this way, the resultant nearly full-length SAP(Val) proprotein is insoluble and can be recovered by extraction with organic solvents.

Similar constructions can be made using the SAP(Val) or SAP(Phe) active regions alone. For example, the SAP(Val) active region, as encoded by nucleotides 100-222 of FIG. 5, may be assembled using synthetic oligonucleotides and inserted into the CKS expression vector. This construction can be designed with an Asp-Pro at the N-terminus of the active region. Acid cleavage at this bond would generate SAP(Val) containing an extra Pro at the N-terminus. As an alternative, assuming that the first 3 amino acids are Gly-Ile-Pro, one may replace the Gly-Ile with Asp such that acids cleavage would leave the SAP(Val) active region minus the first 2 amino acids. An alternative cleavage method using hydroxylamine might also be employed. This chemical cleaves at the peptide bond between Asn and Gly as discussed in Bornstein et al., Meth. Enzymol., 47, 132-145 (1977). Assuming that the SAP(Val) N-terminal amino acid is Gly, this cleavage method yields intact SAP(Val).

EXAMPLE 18

Expression of SAP(Val) and SAP(Phe) in Mammalian Cells

SAP(Val) or SAP(Phe) genomic or cDNA may be inserted into, for example, a mammalian expression plasmid adjacent to the herpes simplex type I (HSV) thymidine kinase (tk) promoter. This plasmid is then added to 1 ml HeBS (8 gm/l NaCl, 0.37 gm/l KCl, 0.25 gm/l $Na_2HPO_4$-12 $H_2O$, 1 gm/l dextrose, 5 gm/l Hepes buffer pH 7.1), and mixed well. 2.5M $CaCl_2$ is added to the mixture to a final concentration of 0.125 M while a gentle stream of air is bubbled through the mixture during the addition of the $CaCl_2$ and for an additional 30 seconds. The DNA is then allowed to precipitate for 30 minutes at room temperature and then 0.5 mls of the DNA suspension is added to 25 $cm^2$ dishes of baby hamster kidney cells, for example, or other mammalian cells of choice. Four hours after addition of the DNA, the culture medium is removed, the cells washed 1 time with Eagle's medium containing 5% fetal calf serum (EC-5), and 1 ml 25% dimethyl sulfoxide (DMSO) in HeBS is added to each dish for 4 minutes at room temperature. The 25% DMSO is then removed, the dish is washed 2 times with EC-5, and the cells are then incubated in EC-5. Twenty-four hours after addition of DNA, the cells are infected with HSV (10 pfu/cell) and the infection allowed to proceed for 48 hours. The expressed SAP(Val) or SAP(Phe) protein is then isolated from the culture fluids by, for example, immunoprecipitation and assayed by, for example, SDS-PAGE.

Genes encoding SAP(Phe) or SAP(Val) may also be expressed in any suitable expression system wellknown to those skilled in the art. Examples of such expression systems include E. coli, Bacillus, yeast, baculovirus or other mammalian cell expression systems.

The sequences shown in FIGS. 3, 5, 6, 8B and 10 indicate that the SAP(Phe) and SAP(Val) are derived from larger precursor proteins. It is believed, based upon a best current estimate of molecular weight of 7500 to 7800 daltons, that a 75-80 residue active portion of the larger precursor protein of SAP(Phe) is encoded by the nucleotide region shown in brackets in FIG. 6. Similarly, it is believed, based upon NMR (NOESY-COSY) analysis of bovine SAP(Val), that the active portion of the larger precursor protein of SAP-(Val) is encoded by 120 or 123 nucleotides, depending upon assignment of N-terminus as Ile or Gly, respectively. This nucleotide region is shown in brackets in FIG. 5. Therefore, it should be apparent to those skilled in the art that expression of either SAP(Val) or SAP(Phe) may be accomplished through expression of the DNA encoding the larger precursor proteins followed by a processing step to further isolate the active portion of these proteins.

Alternatively, one skilled in the art recognizes that another approach for producing SAP(Val) or SAP-(Phe) proteins is to express smaller regions of DNA encoding portions of the larger precursor proteins. For example, the region encompassing nucleotides 627-851 shown in brackets in FIG. 6 may be selected for expression of a 75 amino acid polypeptide which is homologous to SAP(Phe) at the $NH_2$-terminal end and has an approximate molecular weight of 7500 daltons.

It is also apparent to those skilled in the art that the genes encoding SAP(Phe) or SAP(Val) or portions of these genes can be made by chemical or enzymatic synthesis. Further, it should be apparent that deletion, substitution, or addition analogs of such genes or portions of such genes may be made by techniques well known to those skilled in the art. Therefore, it should be understood that SAP(Val) or SAP-(Phe) genes or portions thereof, whether isolated from genomic or cDNA libraries or made by chemical synthesis, and the proteins arising from expression of these genes, are well within the contemplation of this invention.

EXAMPLE 19

Synthesis Of A Polypeptide Based on Canine SAP(Val)

A polypeptide having the following sequence, $NH_2$-Tyr-Ile-Pro-Cys-Phe-Pro-Ser-Ser-Leu-Lys-Arg-Leu-Leu-Ile-COOH, was synthesized to provide a replica of the initial 13 $NH_2$-terminal amino acid residues of canine SAP(Val) and to include a terminal tyrosine residue allowing for potential labeling with $^{125}I$. This polypeptide was assembled on a resin support by stepwise solid phase synthesis (starting with the carboxy terminal residue) according to the general procedure described in Barany et al., The Peptides, 2, Gross et al., eds., Academic Press, New York, 1284 (1980). A BOC-L-Ile-$OCH_2$-Pam resin was transferred to a reaction vessel on an Applied Biosystems Synthesizer, Model 430A.

Protected amino acids were coupled in a stepwise manner to the resin support by preformed symmetric anhydride chemistry, except in the case of arginine addition, wherein the DCC/HOBT protocol [Konig et al., Chem. Ber., 103, 788-798 (1970)] was employed. All $NH_2$-terminal residues were protected by t-butyloxy carbonyl (t-Boc) linkage, and side chains of various amino acid residues were protected by the following groups: Tyr, 2-Br-Z; Cys, 4MeBzl; Ser, Bzl; Lys, 2-Cl-Z; and Arg, Tos.

The fully protected peptide-resin (790 mg) was allowed to swell in methylene chloride ($CH_2Cl_2$) for 5 minutes. The Nα-Boc protecting group was removed using 60% trifluoroacetic acid (TFA)/$CH_2Cl_2$ deprotection, $CH_2Cl_2$ washes, 10% N,N-diisopropylethylamine (DIEA)/$CH_2Cl_2$ neutralization and a final wash with $CH_2Cl_2$. The resin was dried in vacuo. The peptide-resin so obtained was treated with 9 ml of anhydrous hydrofluoric acid (HF) to which 0.5 ml of p-cresol and 0.5 g p-thiocresol had been added for 60 minutes at 0° C.

The HF was distilled away in vacuo at 0° C. The cleaved free peptide and resin were washed 3 times with 15 ml aliquots of diethyl ether, and the peptide was extracted by means of 3 extractions with 15 ml of 40% aqueous acetic acid. The aqueous extracts were combined and washed three times with 10 ml aliquots of diethyl ether, whereupon the aqueous layer was lyophilized to provide the crude polypeptide for purification.

The crude polypeptide was purified by reversed-phase HPLC on $C_4$ columns employing gradients of 0.1% TFA/water (A) and 100% acetonitrile (B) as the solvent systems at a 1 ml/min flow rate for the analytical (Vydac-214-TP54, Vydac Separation Group, Hesperia, California) or 3 ml/min flow rate for the semi-preparative (Vydac-214-TP510). The gradient was started with 30% B, and after one minute was linearly increased to 55% B over a period of 20 minutes before being reduced to 30% B over a period of one minute.

The presence of polypeptide was monitored at 225 nm and 280 nm. The composition of the polypeptide was confirmed by hydrolysis in 6 N hydrochloric acid (HCl)/0.3% phenol at 150° C. for 2 hours in vacuo and was subsequently analyzed on a Beckman 6300 Amino Acid Analyzer.

EXAMPLE 20

Synthesis Of A Polypeptide Based on Bovine SAP(Val)

A polypeptide having the following sequence, $NH_2$-Leu-Ile-Pro-Cys-Cys-Pro-Val-Asn-Ile-Lys-Arg-Leu-Leu-Ile-Val-Val-Val-Val-Val-Val-Val-Val-COOH, was synthesized to provide a replica of the initial 22 $NH_2$-terminal amino acid residues of bovine SAP(Val), and was assembled, purified and analyzed according to the procedure of Example 19, except that the aqueous layer was very turbid and immediately formed an emulsion during washing with ether. At this stage, the aqueous layer was centrifuged at 7500 rpm, 4° C., and a cake-like solid was obtained. The solid so obtained was dissolved in TFA, immediately filtered, cooled in an ice bath and then distilled water was slowly added. A white powdery solid precipitated out. This solid was centrifuged and then washed 3 times with water and 3 times with ether. The white powdery polypeptide so obtained was analyzed and purified on silica columns (Partisil M9 10/50, Whatman, Inc., Clifton, New Jersey; Vydac HS54). The solvent gradient system used was 0.1% TFA/$H_2O$ and 100% $CH_3CN$(B).

EXAMPLE 21

Synthesis Of A Polypeptide Based on Human SAP(Val)

A polypeptide having the following sequence, $NH_2$-Phe-Pro-Ile-Pro-Ile-Pro-Tyr-Cys-Trp-Leu-COOH was synthesized to provide a replica of the initial 10 $NH_2$-terminal amino acid residues of human SAP(Phe) except that, at position 5, Ile was selected rather than Leu. This peptide was assembled according to the general method of Example 19. Side-chain protecting groups employed for the synthesis were: Trp, CHO; Tyr, 2-Br-Z; and Cys, 4-MeBzl. The fully protected peptide-resin (610 mg) was allowed to swell in $CH_2Cl_2$ for 5 minutes and then the Nα-BOC group was removed as described in Example 19. The dried peptide-resin was treated with a mixture of anhydrous HF (2.5 ml):p-cresol (0.5 ml):thiocresol (0.5 g):dimethylsulfide (DMS) (6.5 ml) for 60 minutes at 0° C. DMS and HF were distilled off in vacuo at 0° C. Nine ml of fresh anhydrous HF was distilled into the reaction vessel at 0° C. and then stirred for another 60 minutes at 0° C. The HF was distilled away in vacuo at 0° C. The cleaved free peptide and resin were washed 3 times with 15 ml aliquots of diethyl ether. The rest of the extraction procedure was similar to the one described in Example 19. The crude peptide so obtained was analyzed and purified on reversed-phase HPLC as described before except the gradient was started at 25% B, and after one minute was linearly increased over 20 minutes to 40% B. It was then returned to 25% B in one minute. The purified peptide was analyzed for its amino acid composition as described before.

## EXAMPLE 22

Synthesis Of a Polypeptide Based On Translation of DNA Sequence of Human SAP(Phe)

A polypeptide having the following sequence, $NH_2$-Phe-Pro-Ile-Pro-Leu-Pro-Tyr-Cys-Trp-Leu-Cys-Arg-Ala-Leu-Ile-Lys-Arg-Ile-Gln-Ala-Met-Ile-Pro-Lys-Gly-Ala-Leu-Arg-Val-Ala-Val-Ala-Gln-Val-Cys-Arg-Val-Val-Pro-Leu-Val-Ala-Gly-Gly-Ile-Cys-Gln-Cys-Leu-Ala-Glu-Arg-Tyr-Ser-Val-Ile-Leu-Leu-Asp-Thr-COOH, was synthesized to provide a replica homologous to SAP(Phe) at the $NH_2$-terminal end of human SAP(Phe) based on a translation of a portion of the DNA sequence given in FIG. 6 and further based on the approximate molecular weight of SAP(Phe). This fully protected polypeptide was assembled on a resin support by stepwise solid phase synthesis (starting with the carboxy-terminal residue) as described in Example 19. A BOC-L-Thr(Bzl)-$OCH_2$-Pam-resin was transferred to a reaction vessel. The next nine amino acids were coupled in a stepwise manner according to standard coupling protocols as described before, except where double coupling protocols were used for amino acids Leu, Ile and Val. After a decapeptide-resin (residues 51-60) had been assembled, a small amount of the peptide-resin was taken out for quantitative ninhydrin analysis Sarin et al., Analytical Biochem., 117, 147-157 (1981). This resin was dried in vacuo at room temperature and then weighed (0.6 mg). Such analyses showed that stepwise coupling yields were greater than 99.8%.

The stepwise synthesis was then continued by using double coupling protocols on the remainder of the decapeptide-resin. In a first coupling, protected amino acids were coupled by preformed symmetric anhydride in DMF. A second coupling of symmetric anhydride was conducted in $CH_2Cl_2$. The amino acid glutamine was coupled using the DCC/HOBT protocol referenced hereinabove. After incorporation of methionine at position 21, indole (1%, w/v) and ethanedithiol (0.1%, v/v) were added to TFA. It was this modified solution which was used for all subsequent removals of Nα-BOC protective groups. The weight of the dried peptide-resin at the end of synthesis was 2.72 g. Side chain functional groups of various amino acid residues were protected by the following groups: Tyr, 2Br-Z; Cys, 4MeBzl; Ser, Bzl; Thr, Bzl; Asp, OBzl; Glu, OBzl; Lys, 2Cl-Z; Arg, TOS.

The integrity of the assembled peptide sequence on the resin support was verified by solid phase sequencing on the gas phase sequencer [Sarin et al., Analytical Biochem., 154, 542-551 (1986). A sequencing run was carried out to 29 cycles and confirmed the sequence of the assembled peptide chains with an average preview of 0.5-0.8% per step. The sequencing results also indicated some problem with coupling of arginine and isoleucine at positions 17 and 18, respectively. However, the majority of assembled chains contained the expected sequence.

The fully protected peptide-resin (610 mg) was allowed to swell in $CH_2Cl_2$ for 5 minutes. The Nα-BOC protecting group was removed using TFA containing 1% indole and 0.1% EDT as described before in Example 19. The peptide-resin was then treated with 8.5 ml of anhydrous HF to which 1.0 ml p-cresol, 0.3 ml DMS and 0.2 ml EDT had been added for 60 minutes at 0° C. The HF/DMS was distilled off in vacuo at 0° C. The cleaved peptide and resin were washed 3 times with diethyl ether and then the peptide was extracted using TFA followed by precipitation as a white solid as described in Example 20. The crude peptide so obtained was analyzed and purified by reversed-phase HPLC on a $C_4$ column (Vydac-214-TP54) employing 0.1% TFA/water and 100% acetonitrile as the solvent system as described before. However, for preparative purposes, the column used was Vydac-214-TP1022 at a flow rate of 12 ml/min. The gradient was started at 40% B, and, after 5 minutes was linearly increased to 85% over 20 minutes. The gradient was next reduced to 40% B in 2 minutes.

The presence of peptide was monitored at 225 nm and 280 nm. The composition of the purified peptide was determined by acid hydrolysis (12 N HCl/TFA, 2:1, 5% thioglycolic acid) at 150 °C for 4 hours in vacuo followed by analysis on a Beckman 6300 amino acid analyzer.

During this synthesis, resin samples were taken out at amino acid positions 40 (346 mg, dry) and 22 (500 mg, dry). These 2 peptide-resin samples provide intermediate peptides of 21 (residues 40-60) and 39 (residues 22-60) residues, respectively.

Other peptides based on the sequences given herein may be synthesized and tested by methods known to those skilled in the art. For example, peptides with sequences based on the hydropathicity profiles disclosed herein, as well as those based on the secondary or tertiary structures of SAP(Phe) or SAP(Val) may be synthesized and tested for activity as generally described herein.

## EXAMPLE 23

### Synthesis of Human SAP[Val(1-60)]

The following polypeptide, human SAP[Val(1-60) was synthesized.

```
1                 10                 20
G I P C C P V H L K R L L I V V V V V V L I V V V I V G
  30                 40                 50

A L L M G L H M S Q K H T E M V L E M S I G A P E A Q Q
      60
R L A L
```

A fully protected peptide-resin was assembled on resin support by stepwise solid phase synthesis as described in Examples 19-22. A Boc-L-Leu-$OCH_2$-Pam-Resin was transferred to a reaction vessel. The next 28 amino acids were coupled according to the standard single coupling protocol described before except for amino acids Ile and Val where double coupling protocols were used. After incorporation of methionine at position 47, indole (1% w/v) and ethanedithiol (0.25% v/v) were added to TFA. It was this modified solution which was used for all subsequent removal of Nα-BOC protecting group. After the 28 residue peptide had been assembled, 0.66 g of the resin was taken out.

The stepwise synthesis was then continued on the rest of the peptide-resin using a double coupling protocol throughout the synthesis as described in Example 22. A small amount of the peptide-resin was again taken out after incorporating amino acid Val at position 19. The weight of the dried peptide-resin at the end of the synthesis was 2.53 g. In this synthesis, methionine was used unprotected. However, it may be used in the protected form as a sulfoxide. Side chain functionalities of various amino acids were protected as described above. The amino acid His was protected by a Tosyl group. The integrity of the assembled peptide sequence on the resin was checked using solid phase sequencing as described before.

The Nα-BOC protecting group was removed and then the peptide-resin was treated with 10 ml of anhydrous HF to which 1.0 ml of dimethylsulfide, 1.0 ml of p-cresol and 0.2 mg of thiocresol had been added for 60 minutes at 0 °C. The cleaved peptide and resin was washed 3 times with diethyl ether and then the peptide was extracted with 6 × 10 ml of 40% aqueous acetic acid. The aqueous extract was lyophilized to yield the crude peptide. It was analyzed and then purified by reversed-phase HPLC on a $C_4$

column (D-04-1010, Lot-37100, Brownlee Labs. Santa Clara, California) employing 0.1% TFA/water and 100% acetontride as the solvents system. The crude peptide was dissolved in approximately 75% aqueous TFA before injection on the column. A flow rate of 3 ml/min and the gradient was started at 10% 8. After one minute, it was linearly increased to 90% B over 20 min. and then reduced to 10% B in one minute.

The presence of peptide was monitored at 225 nm and 254 nm. The composition of the purified peptide was determined by acid hydrolysis (12 N HCl:TFA,2:1) at 150° C for 4, 8 24 and 48 hours in vacuo in sealed tubes followed by analysis on a Beckman 6300 amino acid analyzer.

EXAMPLE 24

The following polypeptides according to present invention may be synthesized to the procedure as generally described in Examples 19-23.

Synthesis of Human SAP[Val(1-31)]

The following polypeptide according to the present invention, human SAP[Val(1-31)] was synthesized:

**1 10**
**Leu-Ile-Pro-Cys-Cys-Pro-Val-His-Leu-Lys-Arg-Leu-Leu-Ile-**
**20**
**Val-Val-Val-Val-Val-Val-Leu-Ile-Val-Val-Val-Ile-Val-Gly-**
**31**

**Ala-Leu-Leu**

This synthesis was carried in a manner as generally described in Example 23. All amino acids were assembled on the resin support using the double coupling protocol.

The above polypeptides may have useful surfactant properties as described for the polypeptides of other Examples.

EXAMPLE 25

Human SAP(Phe) Synthetic Peptides

The following polypeptides according to present invention may be synthesized to the procedure as generally described in Example 23.

Human SAP[Phe(1-78)]

```
1                                           10
Phe-Pro-Ile-Pro-Leu-Pro-Tyr-Cys-Trp-Leu-Cys-Arg-
                                  20
Ala-Leu-Ile-Lys-Arg-Ile-Gln-Ala-Met-Ile-Pro-Lys-
                        30
Gly-Ala-Leu-Arg-Val-Ala-Val-Ala-Gln-Val-Cys-Arg-
              40
Val-Val-Pro-Leu-Val-Ala-Gly-Gly-Ile-Cys-Gln-Cys-
     50                                       60
Leu-Ala-Glu-Arg-Tyr-Ser-Val-Ile-Leu-Leu-Asp-Thr-
                                            70
Leu-Leu-Gly-Arg-Met-Leu-Pro-Gln-Leu-Val-Cys-Arg-
                        78
Leu-Val-Leu-Arg-Cys-Ser
```

Human SAP[Phe(27-78)]

```
27             30
Leu-Arg-Val-Ala-Val-Ala-Gln-Val-Cys-Arg-Val-Val-
    40                                          50
Pro-Leu-Val-Ala-Gly-Gly-Ile-Cys-Gln-Cys-Leu-Ala-
                                        60
Glu-Arg-Tyr-Ser-Val-Ile-Leu-Leu-Asp-Thr-Leu-Leu-
                                70
Gly-Arg-Met-Leu-Pro-Gln-Leu-Val-Cys-Arg-Leu-Val-
              78
Leu-Arg-Cys-Ser
```

Human SAP[Phe(53-77)]

```
53                             60
Tyr-Ser-Val-Ile-Leu-Leu-Asp-Thr-Leu-Leu-Gly-Arg-
                           70
Met-Leu-Pro-Gln-Leu-Val-Cys-Arg-Leu-Val-Leu-
          77
Arg-Cys-Ser .
```

The above polypeptides may have useful surfactant properties as described for the polypeptides of other Examples.

EXAMPLE 26

Production of SAP(Val) and/or SAP(Phe) Polyclonal Antibodies

Another utility for SAP(Val) and/or SAP(Phe) is in the preparation of antibodies or antisera. These antibodies or antisera may then be used for detecting, determining or purifying SAP(Val) and/or SAP(Phe). For example, antibodies and antisera may be used for immunoblot and ELISA analysis for immunological detection and quantification or other immunological assays. The detection and determination of SAP(Val) and/or SAP(Phe) by immunological methods is also of diagnostic importance.

SAP(Val) and/or SAP(Phe) antibodies and antisera were obtained as follows. Bovine lung lavage obtained as described in Example 1 was extracted and purified as described in Example 2, then dried and resuspended in ethanol. This suspension was mixed 1:1 with Freund's complete adjuvant and injected into albino rabbits. The rabbits were given injections at 4 separate sites, and usable antisera was obtainable after 3 sets of injections. The bovine lung lavage used for injection contained monomers of SAP(Val) and/or SAP(Phe) and a smaller amount of the multimers of SAP(Val) and/or SAP(Phe) assessed by silver staining analysis after one dimensional SDS-PAGE. If desired, polyclonal antibodies may be further isolated from the antisera by affinity chromatography.

EXAMPLE 27

Production Of Monoclonal Antibodies Against SAP(Val) and/or SAP(Phe)

With respect to the production of monoclonal antibodies ("MAbs") against SAP(Val) and/or SAP(Phe) proteins, lymphocytes from mice immunized with isolated SAP(Val) and/or SAP(Phe) may be fused with mouse myeloma cells in the presence of, for example, polyethylene glycol, generally according to the technique of Kohler and Milstein, Nature, 256, 495-497 (1975), which is incorporated by reference herein. The surviving hybrids may then be screened by solid phase ELISA to obtain only cells which produce the anti-SAP(Val) or anti-SAP(Phe) or antibody cross reacting with SAP(Val) and SAP(Phe). These MAbs may be employed in immunoassay techniques for detection of SAP(Val) and/or SAP(Phe) in, for instance, a tissue sample. Such antibodies may bind to or precipitate SAP(Val) and/or SAP(Phe) from a mixture, may increase the sedimentation rate of SAP(Val) and/or SAP(Phe), may modify elution characteristics of SAP-(Val) and/or SAP(Phe) on gel filtration, and may identify or quantify SAP(Val) and/or SAP(Phe) from a sample.

EXAMPLE 28

Production of Polyclonal Antibodies to SAP(Val) and SAP(Phe) Synthetic Peptides

Polyclonal antibodies have been generated against the SAP(Val) and SAP(Phe) synthetic peptides described in Examples 19-21. These peptides were either injected directly or were first coupled with Keyhole Limpet Hemocyanin ("KLH") (Calbiochem) using the bisdiazotized benzidine method of Bassini et al., Methods in Hormone Radioimmunoassay, Academic Press, 45-56 (1979). The peptides were combined 1:1 with Freund's complete adjuvant and injected into guinea pigs or albino rabbits 3 times at approximate intervals of 2 weeks. The first injection contained KLH, but subsequent injections did not. Approximately 1 mg of protein was injected each time. Although a usable titer was obtained after the third injection, boosts were continued using non-coupled peptides. Antibodies raised against human synthetic SAP[(Phe)(1-60)] have been used to identify, to isolate, to purify SAP(Phe) proteins and may be used as well for diagnostic purposes.

EXAMPLE 29

Diagnostic Uses Of SAP(Val) and/or SAP(Phe) Antibodies

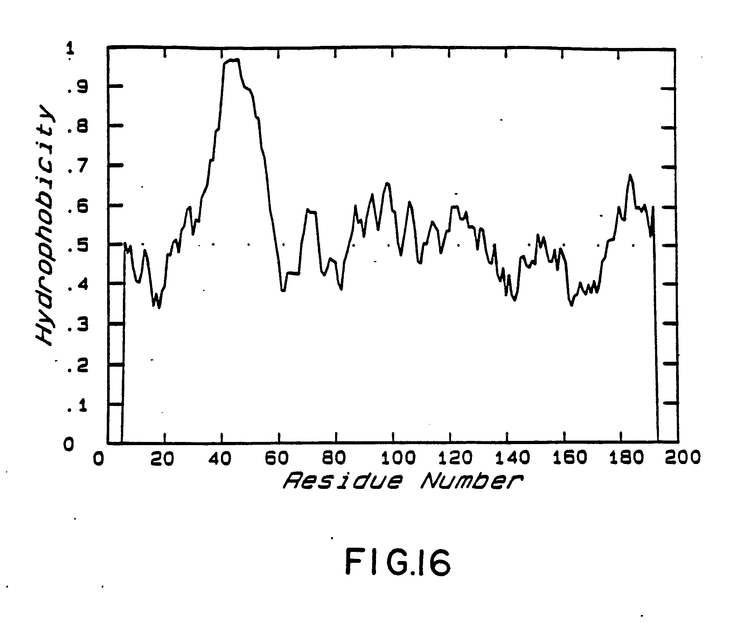

Since SAP(Val) and SAP(Phe) are normally found in certain animal tissues, for example, lung tissue, lung lavage and amniotic fluid, a quantitative or qualitative measurement of SAP(Val) and/or SAP(Phe) may be used as a diagnostic, monitoring, or prognostic tool. Under certain conditions, such as HMD, ARDS, and other syndromes associated with lack of pulmonary surfactant material, SAP(Val) and/or SAP(Phe) will not appear at or will appear in altered concentrations in the tissue of interest. For example, as shown in FIG. 17, SAP(Phe)-SAP(Val) levels have been estimated in human amniotic fluids, obtained at various gestational periods, using the antiproteolipid polyclonal antisera of Example 5 in a competitive ELISA, and the relative reactivity appears to correlate with the timing of fetal lung maturity.

EXAMPLE 30

Human SAP[Val(1-41)]

The following polypeptides according to present invention may be synthesized to the procedure as generally described in Example 23.

Gly-Ile-Pro-Cys-Cys-Pro-Val-His-Leu-Lys-Arg-Leu-Leu-Ile-Val-Val-Val-Val-Val-Val-Leu-Ile-Val-Val-Val-Ile-Val-Gly-Ala-Leu-Leu-Met-Gly-Leu-His-Met-Ser- Gln-Lys-His-Thr

Human SAP[Val(12-31)]

Leu-Leu-Ile-Val-Val-Val-Val-Val-Val-Leu-Ile-Val-Val-Val-Ile-Val-Gly-Ala-Leu-Leu

Human SAP[Val(21-41)]

21 30
Leu-Ile-Val-Val-Val-Ile-Val-Gly-Ala-Leu-Leu-Met-
41
Gly-Leu-His-Met-Ser-Gln-Lys-His-Thr

The above polypeptides may have useful surfactant properties as described for the polypeptides of other Examples.

EXAMPLE 31

Active proteins human SAP(Val) and SAP(Phe) are derived from larger precursor proteins. Some peptides corresponding to sequences from precursor regions of the active proteins may be synthesized generally according to the procedure described in Example 23. The following precursor peptides have been synthesized.

Human SAP[Val(-23 to -1)]

Asp-Val-Gly-Ser-Lys-Glu-Val-Leu-Met-Glu-Ser-Pro-Pro-Asp-Tyr-Ser-Ala-Ala-Pro-Arg-Gly-Arg-Phe

Human SAP[Phe -57 to -29)]

Lys-Ser-Arg-Gln-Pro-Glu-Pro-Glu-Gln-Glu-Pro-Gly-Met-Ser-Asp-Pro-Leu-Pro-Lys-Pro-Leu-Arg-Asp-Pro-Leu-Pro-Asp-Pro-Leu

Human SAo[Phe(-28 to -1)]

Leu-Asp-Lys-Leu-Val-Leu-Pro-Val-Leu-Pro-Gly-Ala-Leu-Gln-Ala-Arg-Pro-Gly-Pro-His-Thr-Gln-Asp-Leu-Ser-Glu-Gln-Gln

The above polypeptides may be used for the generation of antibodies for the recognition of precursor or active protein or some region thereof, and may have surfactant activity.

EXAMPLE 32

Preparation and Testing of Protein/Lipid Admixtures

When SAP(Val) or SAP(Phe) whether in monomeric or multimeric form or whether isolated from animal tissue or made by recombinant DNA methods or direct peptide synthesis based upon human or other animal nucleotide or amino acid sequences is mixed with lipids, its surfactant-like activity is believed to impart to the mixture significant pulmonary biophysical surfactant activity. Such a mixture with this activity is highly useful for replacing or supplementing natural pulmonary surfactant material for reducing or maintaining normal surface tension in lungs, especially in the lungs of animals suffering from HMD and other syndromes associates with the lack or insufficient amounts of natural pulmonary surfactant material.

To make a preparation for medicinal application comprising SAP(Val) and/or SAP(Phe) and lipids, isolated SAP(Val) and/or SAP(Phe) may be stored in chloroform under nitrogen or air at about -30° C prior to reconstitution with lipids. Preparations may also be made with alcohols. Lipids, including neutral or other lipids as well as phospholipids, employed in the preparations may be derived from a number of suitable phospholipids, such as phosphatidylcholine, disaturated phosphatidylcholine, phosphatidylglycerol, dipalmitoylphosphatidylcholine, phosphatidylinositol and mixtures thereof, cholesterol or cholesterol esters and mixtures thereof, and the like.

For example, purified SAP(Val) and/or SAP(Phe) may be mixed in an amount of about 0.1 to about 2.0% with synthetic phospholipids, such as about 65% dipalmitoylphosphatidylcholine, about 20% phosphatidylglycerol, and about 10% palmitic acid. If desired, the mixture may further contain a physiological buffer comprising 0.9% sodium chloride with or without about 0.5 to about 1.5 mM calcium chloride or any other suitable pharmaceutically accepted carrier. After reconstitution or mixing, the material may be sonicated or vortexed and tested for surface tension lowering capacity and adsorption of a Wilhelmy balance or other surface tension measuring devices. Adsorption studies may be conducted by the methodology disclosed by Notter et al., Chem. Phys. Lipids, 33, 67-80 (1983) and Ross et al., J. Biol. Chem., 261, 14283-14291 (1986) which are incorporated by reference herein.

Preparations for medicinal applications ("medicaments") comprising SAP(Val) and/or SAP(Phe) and lipid may be prepared by methods apparent to those skilled in the art. Various methods similar to those described by Yu, et al., J. Biochim. Biophys. Acta., 776, 37-47 (1984); Tanaka, et al., J. Lipid Res., 27, 475-485 (1986); Notter, et al., Ped. Res., 16, 515-519 (1982); Revak, et al., Am. J. Respir. Dis., 134, 1258-1265 (1986) and Suzuki, et al., Eur. J. Respir. Dis., 69, 336-345 (1986) may be used to prepare mixtures of lipids, fatty acids, glycerol derivatives, cholesterol or cholesterol esters with monovalent or divalent metal ion salts and mixtures thereof.

It is apparent to those skilled in the art that various formulations may be prepared using components from the phospholipid, lipid, fatty acid, triglycerides, diglycerides, metal salt and neutral lipid classes in combination with the protein or peptide to prepare suitably active surfactants. For example, a suitable mixture may be comprised of 40-75% dipalmitoylphosphatidyl choline, 0-40% phosphatidyl glycerol, 0-5% cholesterol, 0-10% palmitic acid and 0-10% tripalmitin with 0.1-10% SAP(Val) or SAP(Phe) protein or peptide. Preparation of such admixtures may be accomplished by dissolving the protein or peptide and selected lipid classes in the appropriate solvents, comixing these various solutions in the appropriate ratios, evaporating the solvents and dispersing the resulting admixture in an aqueous media appropriately buffered to physiologic conditions preferably with the chloride salts of mono- or divalent metal ions, e.g., sodium or calcium.

As example of the preparation of a suitable admixture, a protein or peptide is dissolved in a suitable solvent, e.g., chloroform, chloroform:methanol (2:1-5:1), formic acid (50-88%), trifluoroacetic (25-100% in water), dimethyl formamide or dimethylsulfoxide; mixed with the necessary phospholipids, lipids, fatty acids, triglycerides, diglycerides, cholesterol and its esters which are dissolved in suitable solvents as described above under suitable conditions of time and temperature followed by solvent evaporation with warming to 35-75° C., either in vacuo or under an "inert," e.g. nitrogen, gas. The remaining protein lipid admixture is then dispersed with agitation in a variable speed vortex mixer or in an ultrasonic bath (Branson Ultrasonic Cleaner, Model B-32, Shelton, Connecticut) in an aqueous medium containing approximately physiologic concentrations of mono- or divalent metal salts, e.g., 0.9% sodium chloride and/or 1.5 mM calcium chloride to provide a uniform dispersion.

In one approach to biophysical testing of a surface active peptide, a phospholipid mixture containing about 68% dipalmitoylphosphatidylcholine (DPPC), about 22% phosphatidylglycerol and about 10% palmitic acid is combined with delipidated SAP(Val) and/or SAP(Phe) and tested in a Wilhelmy balance. The SAP-(Val) and/or SAP(Phe) phospholipid mixture reduces the surface tension to less than 5 dynes./$cm^2$, is adsorbed rapidly and is found to be more active than the phospholipid mixtures.

The SAP(Val) and/or SAP(Phe) according to the present invention, whether based on naturally occurring proteins or modifications thereof or the described peptides or modifications thereof are believed to have useful "surfactant-like" or "surfactant-associated" activity. As used herein, "surfactant-like" or "surfactant-associated" activity refers to the ability to interact with phospholipids, lipids and other materials described to reduce surface tension at a surface or reduce surface tension at an air-liquid interface. And when SAP-(Val) and/or SAP(Phe) whether in monomeric or multimeric form, or whether isolated from animal tissue or made by recombinant DNA methods or direct peptide synthesis, is mixed with lipids, its surfactant-like or surfactant-associated activity is believed to impart to the admixture significant biophysical surfactant activity. Such a mixture with such activity is highly useful for replacing or supplementing natural pulmonary surfactant material to reduce or maintain normal surface tension in lungs, especially in the lungs of animals suffering from HMD and other syndromes associated with the lack or insufficient amounts of natural pulmonary surfactant material.

Basic characteristics of a suitable surfactant preparation are the ability of the surfactant to adsorb from the subphase to the fluid-air interface and to reduce the surface tension at the interface under dynamic compression and expansion of the surface area. Two common methods of measuring surface tension under dynamic conditions are the Pulsating Bubble Surfactormeter (PBS) [Enhorning, J. App. Physiol. 43, 198-203 (1977)] and by the use of a modified Wilhelmy Surface Balance ("WSB") [Notter, in Pulmonary Surfactant, Ch. 2, Robertson et al., eds, Elseiver, The Netherlands (1984)]. Either method provides similar information but the modified WSB is considered to provide more detail of the surfactant behavior. Substantial reduction of surface tension under the experimental conditions specified is a prerequisite to proper in vivo activity. The time course of the adsorption from the subphase and the equilibrium surface tension which results also assists in providing a measure of activity of surfactant preparations.

Specific conditions utilized for admixing materials demonstrating biophysical activity are as follows: 2.5 mg of the human SAP(Phe) protein of Example 22 prepared by solid-phase synthesis was dissolved in sufficient chloroform to provide a stock solution of 0.25 mg/ml concentration, 250 mg of dipalmitoylphosphatidyl choline (Sigma Chemical Co.) was dissolved in sufficient chloroform:methanol (2:1) to provide a stock solution of 50 mg/ml concentration, phosphatidyl glycerol (Sigma Chemical Co.) at a concentration of 10 mg/ml in chloroform was used directly, 12.5 mg of cholesterol (Sigma Chemical Co.) was dissolved in sufficient chloroform to provide a stock solution of 0.25 mg/ml concentration, 33 mg of palmitic acid (Sigma Chemical Co.) was dissolved in a sufficient quantity of chloroform:methanol (2:1) to provide a stock soluton of 3.3 mg/ml concentration, and 33 mg of tripalmitin (Sigma Chemical Co.) was dissolved in sufficient chloroform:methanol (2:1) to provide a stock solution of 3.3 mg/ml concentration.

Then, 1 ml of the protein stock solution (0.25 mg of protein) and 0.63 ml of the phosphatidtyl glycerol stock solution (6.3 mg of phosphatidtyl glycerol) were mixed together in a flask, warmed to 45° C. for 1 hour, cooled to room temperature and then 0.31 ml of dipalmitoylphosphatidyl choline stock solution (15.5 mg of dipalmitoylphosphatidyl choline) 0.4 ml of the cholesterol stock solution (0.1 mg of cholesterol), 0.42 ml of the palmitic acid stock solution (1.4 mg of palmitic acid) and 0.42 ml of the tripalmitin stock solution (1.4 mg of tripalmitin) were added to the protein-phosphatidyl glycerol solution mixture, mixed briefly on a variable speed vortex mixer for 30-60 seconds and the solvents evaporated in vacuo with warming to 45° C. until all traces of solvents had disappeared. Approximately 0.5-1 ml of 95% ethanol was added to the dried mixture, the resulting mixture mixed for approximately 1 minute on a variable speed vortex mixer and the ethanol evaporated in vacuo at 45° C. followed by the addition of 0.5 to 1 ml of water and continued evaporation at 45° C. in vacuo. The resulting admixture was dispersed in sufficient 0.9% sodium chloride solution to provide 1 ml of dispersion. The material was uniformly dispersed by vortexing for 1.3 minutes and using an ultrasonic bath at 40-45° C. for 1-5 minutes. It is apparent to those skilled in the art that various conditions of dispersion can be utilized to provide uniform dispersion.

Similar techniques for admixing bovine isolate proteins with lipids were used with the protein isolates being dissolved in either chloroform or chloroform:methanol (2:1) solvents.

Data in Table 4 also reflect the results of studies on other proteins and polypeptides in similar admixtures. Although admixtures prepared with some proteins or polypeptides do not exhibit as much dynamic surface tension reduction as those prepared with others, it is apparent to those skilled in the art that this may well be due to the relative lack of refinement of the exact lipid mixture proportions and the variables of the admixing conditions rather than the characteristics of the proteins or polypeptides themsleves. The substantial improvements in the results of dynamic surface tension reduction using the same proteins or polypeptides when changes to lipid composition are made is obvious to those skilled in the art. It is believed that upon refinements in the lipid compositions, admixing variables and protein concentrations, all proteins and polypeptides disclosed in this invention demonstrate suitable activity.

Data shown in Table 4 of surfactant admixtures were obtained by use of the modified WSB. The WSB is operated with a 0.9% sodium chloride solution as subphase at a temperature of 37° C., expanded and compressed over a 2.5-fold surface area at approximately 3 minutes/cycle with approximately 1.5 micrograms of phospholipid admixture per square centimeter of surface area of the subphase being added. Typically, minimum surface tensions of less than 10 dynes/$cm^2$ after several cycles will be observed for a reasonably performing surfactant dispersion. In addition, surface tension maximums will be less than 45 dynes/square centimeter and the percent area at which the surfactant exhibits surface tension of 10 dynes/square centimeter will be a 60% or greater expansion.

The adsorption data in Table 4 were obtained by a method similar to that of Notter et al., Chem. Phys. Lipids, 33, 67-80 (1983). The electrobalance, sensor plate and incubator from the modified WSB are used. The 0.9% sodium chloride solution subphase is stirred and approximately 5 mg of the admixture preparation diluted in a subphase solution volume is added. Surface tension is monitored continuously with time as the admixture adsorbs to the subphase surface. Typically, adsorption measurements which yield surface tensions of 35 dynes/cm$^2$ or less within 10-20 minutes indicate usable surfactants.

In Table 4:DPPC:PG: + = DPPC:PG:PA = 68:22:10; DPPC:PG: + + A = DPPC:PG:CHOLESTEROL:PA:TP = 62.7:25.5:0.4:5.7: 5.7; and DPPC:PG: + + B = DPPC:PG:CHOLESTEROL:PA:TP = 56.6:41.6:0.6:0.6: 0.6. Also in Table 4, SAP(Phe) = 60 mer (as described in Example 22) solid phase synthesized; SAP(Val) = 60 mer (as described in Example 23) solid phase synthesized; SAP[Phe (21)] = 21 mer (as described in Example 22) solid phase synthesized; SAP[Phe 39)] = 39 mer (as described in Example 22) solid phase synthesized; SAP[Val(20)] = 20 mer (as described in Example 30) solid phase synthesized; SAP[Val (22)] = 22 mer (based on the bovine sequence and as described in Example 20) solid phase synthesized; and SAP Isolate = approximately 90% Val and 10% Phe Proteins(as described in Examples 1-2). Further in Table 4, DPPC = Dipalmitoylphosphatidyl Choline; PG = Phosphatidyl Glycerol; PA = Palmitic acid; and TP = Tripalmitin.

TABLE 4

| Protein | Protein Conc | Lipid Mixture | Minima Dynes/$Cm^2$ | Maxima Dynes/$Cm^2$ | %Area @ 10 Dynes/$Cm^2$ | Adsorption @ 10-20 Min. Dynes/$Cm^2$ |
|---|---|---|---|---|---|---|
| None | N/A | DPPC | 70 | 70 | N/A | 68 |
| None | N/A | DPPC/PG (3:1) | 52 | 70 | N/A | 47 |
| None | N/A | DPPC:PG:+ | 46 | 70 | N/A | 50 |
| SAP(Phe) | 1 | DPPC:PG:+ | 0 | 49 | 63 | Not Done |
| SAP(Phe) | 1 | DPPC:PG:++A | 2.5 | 45 | 74 | 35 |
| SAP(Phe) | 4 | DPPC:PG:+ | 0 | 49 | 61 | 37 |
| SAP(Phe) | 1 | DPPC:PG:++B | 4 | 58 | 49 | 45 |
| SAP[Phe(21)] | 0.3 | DPPC:PG:+ | 11 | 55 | N/A | Not Done |
| SAP[Phe(39)] | 0.6 | DPPC:PG:+ | 9.5 | 57 | 45-50 | Not Done |
| SAP(Val) | 7.5 | DPPC:PG:+ | 0 | 54 | 64 | 31 |
| SAP(Val) | 1 | DPPC:PG:+ | 15 | 54 | N/A | Not Done |
| SAP[Val(22)] | 0.4 | DPPC:PG:+ | 11.5 | 55 | N/A | Not Done |
| SAP[Val(20)] | 0.3 | DPPC:PG:+ | 13 | 55 | N/A | Not Done |
| SAP(Phe/Val) | 3/5 | DPPC:PG:+ | 1.5 | 52 | 61 | 36 |
| SAP(Phe/Val) | 0.5/0.5 | DPPC:PG:+ | 11 | 55 | N/A | Not Done |
| SAP Isolate | 0.25 | DPPC:PG:+ | 0 | 34 | 78 | 36 |
| SAP Isolate | 1 | DPPC:PG:+ | 1 | 34 | 75 | 30 |
| SAP Isolate | 2 | DPPC:PG:+ | 0 | 30 | 79 | 23 |
| SAP Isolate | 3.5 | DPPC:PG:+ | 1 | 30 | 76 | 26 |
| SAP Isolate | 7 | DPPC:PG:+ | 0 | 34 | 77 | 22 |

It is shown in Table 4 that proper addition of protein or peptide to lipid mixtures modifies substantially the characteristics of the adsorption and dynamic surface tension diagrams in comparison to the lipid mixture or its individual components. This is demonstrated for protein isolates as well as for the synthesized peptides as shown in Table 4.

## EXAMPLE 33

### Hydropathy Profile Analysis

Hydropathy analysis was performed on the entire predicted amino acid sequences of SAP(Val) according to the procedure of Kyte and Doolittle [Feinberg et al., Anal. Biochem., 132, 6-13 (1983)] and results are presented in FIG. 18. Hydrophobicity is plotted as a function of residue number from $Met_1$ to the C-terminus, $Ile_{197}$. Values indicating hydrophobic and hydrophilic regions are above and below 0.5 respectively as indicated by the horizontal dotted line.

The hydropathy plots of SAP(Val) and SAP(Phe) demonstrate some features in common and there is structural similarity between the N-terminal primary amino acid sequence of SAP(Val) and SAP(Phe). Some of the difference between the two peptides in the valine-rich area may be accounted for by single nucleotide sequence changes suggesting a close relationship between these surfactant peptides. It is of interest that only the hydrophobic regions of SAP(Val) and SAP(Phe) are structurally similar; there is little or no homology between the amino acid or nucleotide sequences flanking the "active" hydrophobic domain. The structure of both SAP(Val) and SAP(Phe) support their hydrophobic interactions with surfactant phospholipids and clarification of their structures may therefore provide insight into the nature of their ability to impart surface activity to surfactant phospholipids. Solubility in organic solvent systems and the marked hydrophobicity of both SAP(Val) and SAP(Phe) are properties similar to those classically referred to as proteolipids and are consistent with previous descriptions of hydrophobic proteins in porcine, rat and bovine surfactants. Extensive dialysis of the hydrophobic surfactant protein preparations results in protein with little remaining phospholipid; however, whether covalent phospholipids are associated with the protein has not yet been fully established.

It is believed that the hydropathy plot generated using SAP(Val) amino acid sequence data according to the present invention permits the selectin of substitution, deletion and addition analogs of SAP(Val) on the basis that the hydropathy plot of the analog is substantially similar to that in FIG. 16.

## EXAMPLE 34

The effects on lipid uptake by Type II cells and 3T3 cells were examined for SAP[Phe(1-60)] (as described in Example 22), for SAP[Phe(40-60)] (as described in Example 22) and for SAP(Val) isolate (as described in Examples 1-2).

Type II cells or sub-confluent 3T3 cells were incubated for three hours with multilamellar vesicles prepared by sonicating 200 µg of phospholipid with 1 ml of phosphate buffered saline (PC:DPPC:PG:PI, defined as in Example 32 above and in ratios of 10:35:7.5:7.5, w:w:w:w) and diluted with 8 ml of DMEM. As a tracer, 0.25 µCi of [$^{14}$C]DPPC was included. At the end of three hours, cells were washed twice with unlabelled medium, and radioactivity was determined by liquid scintillation counting. Uptake was determined as µg phospholipid/mg DNA, and results expressed relative to the control as 100%. Peptides were present at a molar ratio of 1:11 (protein:phospholipid). Control is the uptake of labelled phospholipid by Type II cells in the absence of added peptide.

The results presented in Table 5 as lipid uptake are expressed as a percentage of lipid uptake by a control uptake. These results also demonstrate the ability to deliver phospholipid, and, therefore, possibly other agents (such as drugs, DNA or proteins) into the respiratory epithelium.

TABLE 5

LIPID UPTAKE
(% control)

| Peptide | Cell Type II | Cell Type 3T3 |
|---|---|---|
| Control | 100 | 25 |
| SAP[Phe(1-60)] | 787 | 1700 |
| SAP[Phe(40-60)] | 415 | 81 |
| Sap(Val) | 706 | 425 |
| Albumin | 116 | 31 |

In treating HMD and other related disorders, mixtures containing SAP(Val) and/or SAP(Phe) and naturally occurring or synthetic lipids may be instilled intratracheally, for instance, in liquid form or as an aerosol spray in doses of about 0.01% to about 5.0% SAP(Val) and/or SAP(Phe) by weight, with about 20 to about 100 mg phospholipid per kilogram for treatment or prevention of HMD and other related disorders.

The present invention may, of course, be carried out in other specific ways than those herein set forth without departing from the spirit and essential characteristics of the invention. The present embodiments are, therefore, to be considered in all respects as illustrative and not restrictive and all changes coming within the meaning and of equivalents.

The features disclosed in the foregoing description, in the claims and/or in the accompanying drawings may, both separately and in any combination thereof, be material for realising the invention in diverse forms thereof.

## Claims

1. A purified and isolated DNA sequence encoding SAP(Val).

2. A purified and isolated SAP(Val) nucleic acid described by a nucleotide sequence selected from the group consisting of:

the nucleotide sequence as illustrated in FIG. 5 or FIG. 10;

a nucleotide sequence comprising 20 sequential nucleotides in the nucleotide sequence illustrated in FIG. 5 or FIG. 10;

a nucleotide sequence describing a nucleic acid which hybridizes with 20 sequential nucleotides in the nucleotide sequence illustrated in FIG. 5 or FIG. 10;

a nucleotide sequence describing a nucleic acid which would hybridize with 20 sequential nucleotides in the nucleotide sequence illustrated in FIG. 5 or FIG. 10 but for the redundancy of the genetic code; and

a nucleotide sequence encoding an epitope encoded by 18 sequential nucleotides in the nucleotide sequence illustrated in FIG. 5 or FIG. 10.

3. A transformation vector comprising the nucleic acid of claim 2.

4. A cell transformed with the vector as recited in claim 3.

5. A method for obtaining purified and isolated SAP(Val) comprising the steps of:

culturing a cell as recited in claim 4 in a medium and under conditions favorable for expression of SAP-(Val); and

isolating SAP(Val) from the contents of the medium.

6. A purified and isolated SAP(Val) substantially free of other human proteins and encoded by a purified and isolated nucleic acid described by a nucleotide sequence selected from the group consisting of:

the nucleotide sequence as illustrated in FIG. 5 or FIG. 10;

a nucleotide sequence comprising 20 sequential nucleotides in the nucleotide sequence illustrated in FIG. 5 or FIG. 10; and

a nucleotide sequence encoding an epitope encoded by 18 sequential nucleotides in the nucleotide sequence illustrated in FIG. 5 or FIG. 10.

7. A SAP(Val) synthetic polypeptide made by chemical or enzymatic peptide synthesis.

8. The synthetic peptide as recited in claim 7 having the following amino acid sequence:
Tyr-Ile-Pro-Cys-Phe-Pro-Ser-Ser-Leu-Lys-Arg-Leu-Leu-Ile.

9. The synthetic peptide as recited in claim 7 having the following amino acid sequence:
Leu-Ile-Pro-Cys-Cys-Pro-Val-Asn-Ile-Lys-Arg-Leu-Leu-Ile-Val-Val-Val-Val-Val-Val-Val-Val.

10. The synthetic peptide as recited in claim 7 having the following amino acid sequence:
Leu-Ile-Pro-Cys-Cys-Pro-Val-His-Leu-Lys-Arg-Leu-Leu-Ile-Val-Val-Val-Val-Val-Val-Leu-Ile-Val-Val-Val-Ile-Val-Gly-Ala-Leu-Leu.

11. The synthetic peptide as recited in claim 7 having the following amino acid sequence:

Leu-Ile-Pro-Cys-Cys-Pro-Val-Asn-Ile-Lys-Arg-Leu-Leu-Ile-Val-Val-Val-Val-Val-Val-Leu-Leu-Val-Val-Val.

12. The synthetic peptide as recited in claim 7 having the following amino acid sequence

Ile-Pro-Cys-Phe-Pro-Ser-Ser-Leu-Lys-Arg-Leu-Leu-Ile-Val-Val-Val-Val-Val-Val-Val-Val-Val.

13. The synthetic peptide as recited in claim 7 having the following amino acid sequence;

Leu-Ile-Pro-Cys-Cys-Pro-Val-Asn-Leu-Lys-Arg-Leu-Leu-Ile-Val-Val-Val-Val-Val

14. The synthetic peptide as recited in claim 7 having the following amino acid sequence:

G I P C C P V H L K R L L I V V V V V V L I V V V I V G A L L M G L H M S Q K H T E M V L E M S I G A P E A Q Q R L A L

15. The synthetic peptide as recited in claim 7 having the following amino acid sequence:
Leu-Ile-Pro-Cys-Cys-Pro-Val-His-Leu-Lys-Arg-Leu-Leu-Ile-Val-Val-Val-Val-Val-Val-Leu-Ile-Val-Val-Val-Ile-Val-Gly-

16. The synthetic peptide as recited in claim 7 having the following amino acid sequence:
Gly-Ile-Pro-Cys-Cys-Pro-Val-His-Leu-Lys-Arg-Leu-Leu-Ile-Val-Val-Val-Val-Val-Val-Leu-Ile-Val-Val-Val-Ile-Val-Gly-Ala-Leu-Leu-Met-Gly-Leu-His-Met-Ser-Gln-Lys-His-Thr.

17. The synthetic peptide as recited in claim 7 having the following amino acid sequence:
Leu-Ile-Val-Val-Val-Ile-Val-Gly-Ala-Leu-Leu-Met-Gly-Leu-His-Met-Ser-Gln-Lys-His-Thr.

18. The synthetic peptide as recited in claim 7 having the following amino acid sequence:
Leu-Leu-Ile-Val-Val-Val-Val-Val-Val-Leu-Ile-Val-Val-Val-Ile-Val-Gly-Ala-Leu-Leu

19. A synthetic SAP(Phe) polypeptide made by chemical or enzymatic peptide synthesis.

20. The synthetic peptide as recited in claim 19 having the following amino acid sequence:
Phe-Pro-Ile-Pro-Ile-Pro-Tyr-Cys-Trp-Leu.

21. The synthetic peptide as recited in claim 19 having the following amino acid sequence:
Phe-Pro-Ile-Pro-Leu-Pro-Tyr-Cys-Trp-Leu-Cys-Arg-Ala-Leu-Ile-Lys-Arg-Ile-Gln-Ala-Met-Ile-Pro-Lys-Gly-Ala-Leu-Arg-Val-Ala-Val-Ala-Gln-Val-Cys-Arg-Val-Val-Pro-Leu-Val-Ala-Gly-Gly-Ile-Cys-Gln-Cys-Leu-Ala-Glu-Arg-Tyr-Ser-Val-Ile-Leu-Leu-Asp-Thr.

22. The synthetic peptide as recited in claim 19 having the following amino acid sequence:
Phe-Pro-Ile-Pro-Ile-Pro

23. The synthetic peptide as recited in claim 19 having the following amino acid sequence:

(Gln)-Pro-Ile-Pro-Ile-Pro-Tyr
(Ile)
(Phe)
(Gly)

24. The synthetic peptide as recited in claim 19 having the following amino acid sequence:

Phe-Pro-Ile-Pro-Leu-Pro-Tyr-Cys-Trp-Leu-Cys-Arg-Ala-Leu.

25. The synthetic peptide as recited in claim 19 having the following amino acid sequence:
Tyr-Ile-Pro-Cys-Phe-Pro-Ser-Ser-Leu-Lys-Arg-Leu-Ile.

26. The synthetic peptide as recited in claim 19 having the following amino acid sequence:
Tyr-Ser-Val-Ile-Leu-Leu-Asp-Thr-Leu-Leu-Gly-Arg-Met-Leu-Pro-Gln-Leu-Val-Cys-Arg-Leu-Val-Leu-Arg-Cys-Ser.

27. The synthetic peptide as recited in claim 19 having the following amino acid sequence:
Phe-Pro-Ile-Pro-Leu-Pro-Tyr-Cys-Trp-Leu-Cys-Arg-Ala-Leu-Ile-Lys-Arg-Ile-Gln-Ala-Met-Ile-Pro-Lys-Gly-Ala-Leu-Arg-Val-Ala-Val-Ala-Gln-Val-Cys-Arg-Val-Val-Pro-Leu-Val-Ala-Gly-Gly-Ile-Cys-Gln-Cys-Leu-Ala-Glu-Arg-Tyr-Ser-Val-Ile-Leu-Leu-Asp-Thr-Leu-Leu-Gly-Arg-Met-Leu-Pro-Gln-Leu-Val-Cys-Arg-Leu-Val-Leu-Arg-Cys-Ser.

28. The synthetic peptide as recited in claim 19 having the following amino acid sequence:
Leu-Arg-Val-Ala-Val-Ala-Gln-Val-Cys-Arg-Val-Val-Pro-Leu-Val-Ala-Gly-Gly-Ile-Cys-Gln-Cys-Leu-Ala-Glu-Arg-Tyr-Ser-Val-Ile-Leu-Leu-Asp-Thr-Leu-Leu-Gly-Arg-Met-Leu-Pro-Gln-Leu-Val-Cys-Arg-Leu-Val-Leu-Arg-Cys-Ser.

29. The synthetic peptide as recited in claim 19 having the amino acid sequence described by residues (41-60) in SAP(Phe) as illustrated in FIG. 6.

30. The syntheti.c peptide as recited in claim 19 having the amino acid sequence described by residues (22-60) in SAP(Phe) as illustrated in FIG. 6.

31. A medicament for reducing or maintaining normal pulmonary surface tension in the alveoli of an animal comprising substantially pure SAP(Val) as recited in claim 6, a lipid and pharmaceutically acceptable carrier, said SAP(Val) and said lipid being present in a effective amount to reduce or maintain normal pulmonary surface tension in the alveoli.

32. The medicament as recited in claim 31 further comprising multimers comprising SAP(Val).

33. The medicament as recited in claim 31 wherein said lipid is selected from the group consisting of: a synthetic phospholipid, a naturally occurring phospholipid or mixture thereof.

34. The medicament as recited in claim 31 wherein said lipid is selected from the group consisting of phospholipids, neutral lipids, cholesterol, cholesterol esters and mixtures thereof, and phosphatidylcholine, disaturated phosphatidylcholine, phosphatidylglycerol, dipalmitoylphosphatidylcholine, phosphatidylinositol and mixtures thereof.

35. A method of treating or preventing hyaline membrane disease or other syndromes associated with insufficient pulmonary surfactant material in animals comprised of administering to the animal in an effective dose, isolated SAP(Val) as recited in claim 1 and a lipid to reduce or maintain normal pulmonary surface tension.

36. The method as recited in claim 35 wherein the SAP(Val) and lipid are administered to the animal as a liquid or as an aerosol spray.

37. A specific, immunologically active anti-SAP(Val) polyclonal or monoclonal antibody obtained as a serum immunoglobulin component from an animal immunized with SAP(Val) as recited in claim 6 and characterized by its donation to the immunoglobulin of the following properties:

a. the ability of said immunoglobulin to bind to or precipitate SAP(Val) from a mixture containing SAP(Val);

b. the ability of said immunoglobulin to increase the sedimentation rate of SAP(Val);

c. the ability of said immunoglobulin to modify elution characteristics of SAP(Val); and

d. the ability of said immunoglobulin to identify or quantify SAP(Val) from a sample.

38. A specific, immunologically active anti-SAP(Phe) polyclonal or monoclonal antibody obtained as a serum immunoglobulin component from an animal immunized with SAP(Phe) and characterized by its donation to the immunoglobulin of the following properties:

a. the ability of said immunoglobulin to bind SAP(Phe) to or precipitate SAP(Phe) from a mixture containing SAP(Phe);

b. the ability of said immunoglobulin to increase the sedimentation rate of SAP(Phe);

c. the ability of said immunoglobulin to modify elution characteristics of SAP(Phe); and

d. the ability of said immunoglobulin to identify or quantify SAP(Phe) from a sample.

39. A SAP(Val) synthetic precursor peptide having the following sequence:
Asp-Val-Gly-Ser-Lys-Glu-Val-Leu-Met-Glu-SerPro-Pro-Asp-Tyr-Ser-Ala-Ala-Pro-Arg-Gly-Arg-Phe.

40. A SAP(Phe) synthetic precursor peptide having the following amino acid sequence:
Lys-Ser-Arg-Gln-Pro-Glu-Pro-Glu-Gln-Glu-Pro-Gly-Met-Ser-Asp-Pro-Leu-Pro-Lys-Pro-Leu-Arg-Asp-Pro-Leu-Pro-Asp-Pro-Leu.

41. A SAP(Phe) synthetic precursor peptide having the following amino acid sequence:

Leu-Asp-Lys-Leu-Val-Leu-Pro-Val-Leu-Pro-Gly-Ala-Leu-Gln-Ala-Arg-Pro-Gly-Pro-His-Thr-Gln-Asp-Leu-Ser-Glu-Gln-Gln.

42. A purified and isolated SAP(Phe) nucleic acid described by a nucleotide sequence not found in cDNA encoding SAP(Phe) wherein the nucleic acid is described by a nucleotide sequence selected from the group consisting of:

the nucleotide sequence as illustrated in FIG 8B;

a nucleotide sequence comprising 20 sequential nucleotides in an intron region of the nucleotide sequence illustrated in FIG. 8B;

a nucleotide sequence comprising 20 sequential nucleotides in a 5′ noncoding region of the nucleotide sequence illustrated in FIG. 8B;

a nucleotide sequence comprising 20 sequential nucleotides in a 3′ noncoding region of the nucleotide sequence illustrated in FIG. 8B;

a nucleotide sequence describing a nucleic acid which hybridizes with 20 sequential nucleotides a noncoding region of the nucleotide sequence illustrated in FIG. 8B.

43. Isolated and substantially pure human SAP(Val), said human SAP(Val) enhancing surfactant-like activity of lipids, having a simple molecular weight of about 4,000-7,000 daltons determined in a polyacrylamide gel containing sodium dodecyl sulfate and wherein the sequence of amino acid residues of said human SAP(Val) comprises an epitope encoded by 18 sequential nucleotides in the nucleotide sequence illustrated in FIG. 5 or FIG. 10.

44. Isolated and substantially pure bovine SAP(Phe) said bovine SAP(Phe) being isolatable using an antibody as recited in claim 34, enhancing surfactant-like activity of lipids, having a simple molecular weight of about 4,000-7,000 daltons determined in a polyacrylamide gel containing sodium dodecyl sulfate and wherein the sequence of the $NH_2$-terminal amino acid residues of said bovein SAP-(Phe) is:

Phe-Pro-Ile-Pro-Ile-Pro.

45. Isolated and substantially pure canine SAP(Val), said canine SAP(Val) enhancing surfactant-like activity of lipids, having a simple molecular weight of about 4,000-7,000 daltons determined in a polyacrylamide gel containing sodium dodecyl sulfate and wherein the sequence of the $NH_2$-terminal amino acid residues of said canine SAP(Val) is:

ILe-Pro-Cys-Phe-Pro-Ser-Ser-Leu-Lys-Arg-Leu-Leu-Ile-Val-
Val-Val-Val-Val-Val-Val-Val-Val-
(Ile)(Ile)

46. An analog of SAP(Val) having secondary structure exhibiting a hydropathy profile as illustrated by the coding region in FIG. 5 or FIG. 10.

47. An assay for the presence of SAP(Phe) in a bodily fluid comprising the step of exposing a sample of the bodily fluid to an antibody recited in claim 38.

48. The assay as recited in claim 47 further comprising the step of exposing the sample to SAP(Phe).

49. An assay for the presence of SAP(Phe) in a bodily fluid comprising the step of exposing a sample of the bodily fluid to SAP(Phe).

50. The assay as recited in claim 49 wherein said SAP(Phe) is a synthetic peptide as recited in claim 19.

51. An assay for the presence of SAP(Val) in a bodily fluid comprising the step of exposing a sample of the bodily fluid to an antibody as recited in claim 37.

52. The assay as recited in claim 51 further comprising the step of exposing the sample to a SAP(Val).

53. An assay for the presence of SAP(Val) in a bodily fluid comprising the step of exposing a sample of the bodily fluid to SAP(Val).

54. The assay as recited in claim 53 wherein said SAP(Val) is a synthetic peptide as recited in claim 7.

55. A method for delivering a substance to respiratory epithelial cells comprising the steps of:

combining the substance with SAP(Val); and

applying the combination of SAP(Val) and the substance to respiratory epithelial cells.

56. The method as recited in claim 55 further comprising the step of combining the substance with a phospholipid.

57. A method for delivering a substance to respiratory epithelial cells comprising the steps of:
combining the substance with SAP(Phe); and
applying the combination of SAP(Phe) and the substance to respiratory epithelial cells.

58. The method as recited in claim 57 further comprising the step of combining the substance with a phospholipid.

Nouvellement déposé

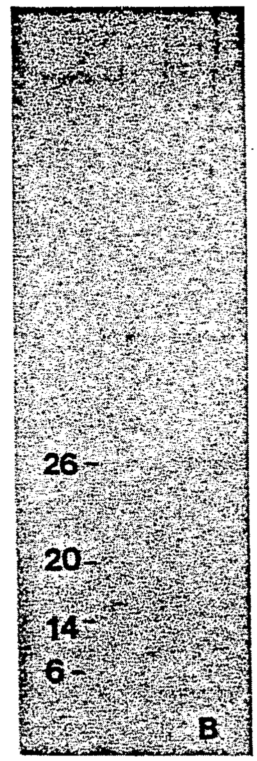


FIG.1

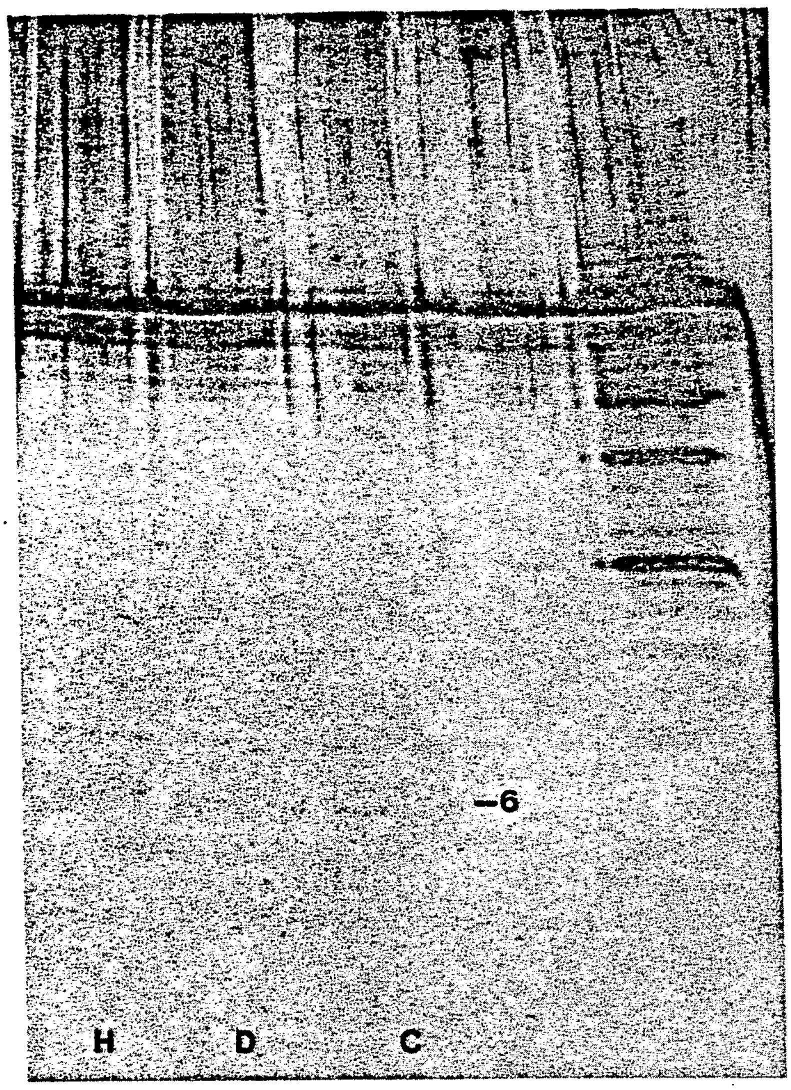


FIG.2

POOR QUALITY

```
GAA TTC CGG GAG TGC AAC GTC CTC CCC TTG AAG CTG CTC ATG CCC CAG TGC AAC CAA GTG    60
Glu Phe Arg Glu Cys Asn Val Leu Pro Leu Lys Leu Leu MET Pro Gln Cys Asn Gln Val

CTT GAC GAC TAC TTC CCC CTG GTC ATC GAC TAC TTC CAG AAC CAG ACT GAC TCA AAC GGC   120
Leu Asp Asp Tyr Phe Pro Leu Val Ile Asp Tyr Phe Gln Asn Gln Thr Asp Ser Asn Gly

ATC TGT ATG CAC CTG GGC CTG TGC AAA TCC CGG CAG CCA GAG CCA GAG CAG GAG CCA GGG   180
Ile Cys MET His Leu Gly Leu Cys Lys Ser Arg Gln Pro Glu Pro Glu Gln Glu Pro Gly

ATG TCA GAC CCC CTG CCC AAA CCT CTG CGG GAC CCT CTG CCA GAC CCT CTG CTG GAC AAG   240
MET Ser Asp Pro Leu Pro Lys Pro Leu Arg Asp Pro Leu Pro Asp Pro Leu Leu Asp Lys

CTC GTC CTC CCT GTG CTG CCC GGG GCC CTC CAG GCG AGG CCT GGG CCT CAC ACA CAG GAT   300
Leu Val Leu Pro Val Leu Pro Gly Ala Leu Gln Ala Arg Pro Gly Pro His Thr Gln Asp

CTC TCC GAG CAG CAA TTC CCC ATT CCT CTC CCC TAT TGC TGG CTC TGC AGG GCT CTG ATC   360
Leu Ser Glu Gln Gln Phe Pro Ile Pro Leu Pro Tyr Cys Trp Leu Cys Arg Ala Leu Ile

AAG CGG ATC CAA GCC ATG ATT CCC AAG GGT GCG CTA CGT(GTG GCA GTG GCC CAG GTG TGC   420
Lys Arg Ile Gln Ala MET Ile Pro Lys Gly Ala Leu Arg(Val Ala Val Ala Gln Val Cys

CGC GTG GTA CCT CTG GTG GCG GGC GGC ATC TGC CAG)TGC CTG GCT GAG CGC TAC TCC GTC   480
Arg Val Val Pro Leu Val Ala Gly Gly Ile Cys Gln)Cys Leu Ala Glu Arg Tyr Ser Val

ATC CTG CTC GAC ACG CTG CTG GGC CGC ATG CTG CCC CAG CTG GTC TGC CGC CTC GTC CTC   540
Ile Leu Leu Asp Thr Leu Leu Gly Arg MET Leu Pro Gln Leu Val Cys Arg Leu Val Leu

CGG TGC TCC ATG GAT GAC AGC GCT GGC CCA AGG TCG CCG ACA GGA GAA TGG CTG CCG CGA   600
Arg Cys Ser MET Asp Asp Ser Ala Gly Pro Arg Ser Pro Thr Gly Glu Trp Leu Pro Arg

GAC TCT GAG TGC CAC CTC TGC ATG TCC GTG ACC ACC CAG GCC GGG AAC AGC AGC GAG CAG   660
Asp Ser Glu Cys His Leu Cys MET Ser Val Thr Thr Gln Ala Gly Asn Ser Ser Glu Gln

GCC ATA CTA CAG GCA ATG CTC CAG GCC TGT GTT GGC TCC TGG CTG GAC AGG GAA AAG TGC   720
Ala Ile Leu Gln Ala MET Leu Gln Ala Cys Val Gly Ser Trp Leu Asp Arg Glu Lys Cys
```

FIG.3A

```
AAG CAA TTT GTG GAG CAG CAC ACG CCC CAG CTG CTG ACC CTG GTG CCC AGG GGC TGG GAT    780
Lys Gln Phe Val Glu Gln His Thr Pro Gln Leu Leu Thr Leu Val Pro Arg Gly Trp Asp

GCC CAC ACC ACC TGC CAG GCC CTC GGG GTG TGT GGG ACC ATG TCC AGC CCT CTC CAG TGT    840
Ala His Thr Thr Cys Gln Ala Leu Gly Val Cys Gly Thr MET Ser Ser Pro Leu Gln Cys

ATC CAC AGC CCC GAC CTT TGA TGA GAA CTC AGC TGT CCA GCT GCA AAG GAA AAG CCA AGT    900
Ile His Ser Pro Asp Leu Non

GAG ACG GGC TCT GGG ACC ATG GTG ACC AGG CTC TTC CCC TGC TCC CTG GCC CTC GCC AGC    960

TGC CAG GCT GAA AAG AAG CCT CAG CTC CCA CAC CGC CCT CCT CAC CGC CCT TCC TCG GCA   1020

GTC ACT TCC ACT GGT GGA CCA CGG CCC CCA GCC CTG TGT CGG CCT TGT CTG TCT CAG CTC   1080

AAC CAC AGT CTG ACA  CA GAG CCC ACT TCC ATC CTC TCT GGT GTG AG  CAC AGC GAG GGC   1140

AGC ATC TGG AGG AGC TCT GCA GCC TCC ACA                                           1170
```

FIG.3B.

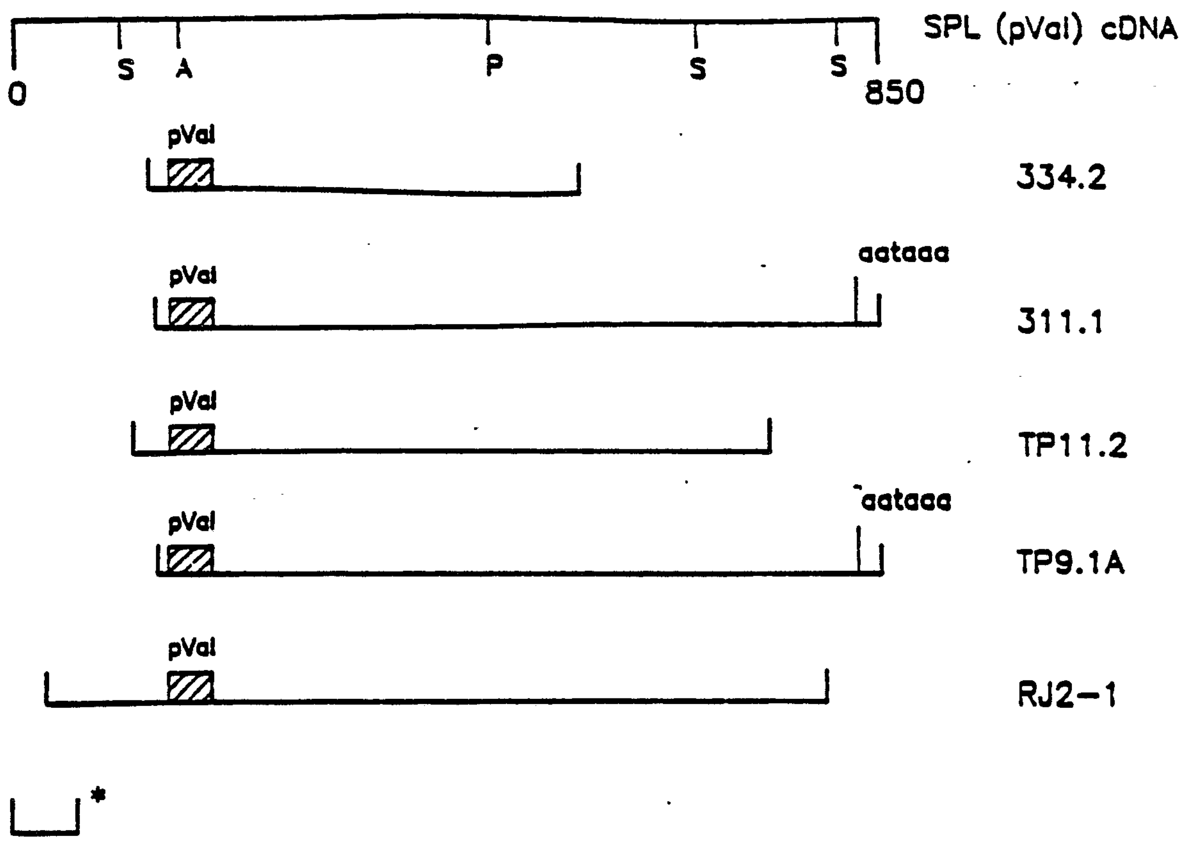
SPL (pVal) cDNA
0
S
A
P
S
S
850
pVal
334.2
aataaa
pVal
311.1
pVal
TP11.2
aataaa
pVal
TP9.1A
pVal
RJ2-1
*

FIG.4

```
                                            30                                       60
ACA GGA GAG CAT AGC ACC TGC AGC AAG ATG GAT GTG GGC AGC AAA GAG GTC CTG ATG GAG
                                    MET Asp Val Gly Ser Lys Glu Val Leu MET Glu
                                     1

                                            90                                      120
AGC CCG CCG GAC TAC TCC GCA GCT CCC CGG GGC CGA TTT GGC ATT CCC TGC TGC CCA GTG
Ser Pro Pro Asp Tyr Ser Ala Ala Pro Arg Gly Arg Phe Gly Ile Pro Cys Cys Pro Val
                                                        26

                                           150                                      180
CAC CTG AAA CGC CTT CTT ATC GTG GTG GTG GTG GTG GTC CTC ATC GTC GTG GTG ATT GTG
His Leu Lys Arg Leu Leu Ile Val Val Val Val Val Val Leu Ile Val Val Val Ile Val

                                           210                                      240
GGA GCC CTG CTC ATG GGT CTC CAC ATG AGC CAG AAA CAC ACG GAG ATG GTT CTG GAG ATG
Gly Ala Leu Leu MET Gly Leu His MET Ser Gln Lys His Thr Glu MET Val Leu Glu MET

                                           270                                      300
AGC ATT GGG GCG CCG GAA GCC CAG CAA CGC CTG GCC CTG AGT GAG CAC CTG GTT ACC ACT
Ser Ile Gly Ala Pro Glu Ala Gln Gln Arg Leu Ala Leu Ser Glu His Leu Val Thr Thr

                                           330                                      360
GCC ACC TTC TCC ATC GGC TCC ACT GGC CTC GTG GTG TAT GAC TAC CAG CAG CTG CTG ATC
Ala Thr Phe Ser Ile Gly Ser Thr Gly Leu Val Val Tyr Asp Tyr Gln Gln Leu Leu Ile

                                           390                                      420
GCC TAC AAG CCA GCC CCT GGC ACC TGC TGC TAC ATC ATG AAG ATA GCT CCA GAG AGC ATC
Ala Tyr Lys Pro Ala Pro Gly Thr Cys Cys Tyr Ile MET Lys Ile Ala Pro Glu Ser Ile

                                           450                                      480
CCC AGT CTT GAG GCT CTC ACT AGA AAA GTC CAC AAC TTC CAG ATG GAA TGC TCT CTG CAG
Pro Ser Leu Glu Ala Leu Thr Arg Lys Val His Asn Phe Gln MET Glu Cys Ser Leu Gln
```

FIG.5A

```
                                   510                                            540
GCC AAG CCC GCA GTG CCT ACG TCT AAG CTG GGC CAG GCA GAG GGG CGA GAT GCA GGC TCA
Ala Lys Pro Ala Val Pro Thr Ser Lys Leu Gly Gln Ala Glu Gly Arg Asp Ala Gly Ser

                                   570                                            600
GCA CCC TCC GGA GGG GAC CCG GCC TTC TTG GGC ATG GCC GTG AAC ACC CTG TGT GGC GAG
Ala Pro Ser Gly Gly Asp Pro Ala Phe Leu Gly MET Ala Val Asn Thr Leu Cys Gly Glu

                                   630                                            660
GTG CCG CTC TAC TAC ATC TAG GAC GCC TCC GGT GAG CAG GGT CAG TGG AAG CCC CAA CGG
Val Pro Leu Tyr Tyr Ile End

                                   690                                            720
GAA AGG AAA CGC CCC GGG CAA AGG GTC TTT TGC AGC TTT TGC AGA CGG GCA AGA AGC TGC

                                   750                                            780
TTC TGC CCA CAC CGC AGG GAC AAA CCC TGG AGA AAT GGG AGC TTG GGG AGA GGA TGG GAG

                                   810                                            840
TGG GCA GAG GTG GCA CCC AGG GGC CCG GGA ACT CCT GCC ACA ACA GAA TAA AGC AGC CTG

ATT TGA AAA GCA AAA AAA
```

FIG.5B.

```
                        10                      26                      41                      56
              GCTGCAGAGG TGCC ATG GCT GAG TCA CAC CTG CTG CAG TGG CTG CTG CTG CTG CTG CCC
                              MET Ala Glu Ser His Leu Leu Gln Trp Leu Leu Leu Leu Leu Pro
                              1

        71                      86                      101                     116
ACG CTC TGT GGC CCA GGC ACT GCT GCC TGG ACC ACC TCA TCC TTG GCC TGT GCC CAG GGC CCT GAG
Thr Leu Cys Gly Pro Gly Thr Ala Ala Trp Thr Thr Ser Ser Leu Ala Cys Ala Gln Gly Pro Glu

    131                     146                     161                     176                 191
TTC TGG TGC CAA AGC CTG GAG CAA GCA TTG CAG TGC AGA GCC CTA GGG CAT TGC CTA CAG GAA GTC
Phe Trp Cys Gln Ser Leu Glu Gln Ala Leu Gln Cys Arg Ala Leu Gly His Cys Leu Gln Glu Val
                                                50

                206                     221                     236                     251
TGG GGA CAT GTG GGA GCC GAT GAC CTA TGC CAA GAG TGT GAG GAC ATC GTC CAC ATC CTT AAC AAG
Trp Gly His Val Gly Ala Asp Asp Leu Cys Gln Glu Cys Glu Asp Ile Val His Ile Leu Asn Lys

        266                     281                     296                     311
ATG GCC AAG GAG GCC ATT TTC CAG GAC ACG ATG AGG AAG TTC CTG GAG CAG GAG TGC AAC GTC CTC
MET Ala Lys Glu Ala Ile Phe Gln Asp Thr MET Arg Lys Phe Leu Glu Gln Glu Cys Asn Val Leu
                                                                        100

326                     341                     356                     371                 386
CCC TTG AAG CTG CTC ATG CCC CAG TGC AAC CAA GTG CTT GAC GAC TAC TTC CCC CTG GTC ATC GAC
Pro Leu Lys Leu Leu MET Pro Gln Cys Asn Gln Val Leu Asp Asp Tyr Phe Pro Leu Val Ile Asp

            401                     416                     431                     446
TAC TTC CAG AAC CAG ACT GAC TCA AAC GGC ATC TGT ATG CAC CTG GGC CTG TGC AAA TCC CGG CAG
Tyr Phe Gln Asn Gln Thr Asp Ser Asn Gly Ile Cys MET His Leu Gly Leu Cys Lys Ser Arg Gln
```

FIG.6A

```
    461                     476                     491                     506                     521
CCA GAG CCA GAG CAG GAG CCA GGG ATG TCA GAC CCC CTG CCC AAA CCT CTG CGG GAC CCT CTG CCA
Pro Glu Pro Glu Gln Glu Pro Gly MET Ser Asp Pro Leu Pro Lys Pro Leu Arg Asp Pro Leu Pro
        150

                536                     551                     566                     581
GAC CCT CTG CTG GAC AAG CTC GTC CTC CCT GTG CTG CCC GGG GCC CTC CAG GCG AGG CCT GGG CCT
Asp Pro Leu Leu Asp Lys Leu Val Leu Pro Val Leu Pro Gly Ala Leu Gln Ala Arg Pro Gly Pro

        596                     611                     626                     641
CAC ACA CAG GAT CTC TCC GAG CAG CAA|TTC|CCC ATT CCT CTC CCC TAT TGC TGG CTC TGC AGG GCT
His Thr Gln Asp Leu Ser Glu Gln Gln|Phe|Pro Ile Pro Leu Pro Tyr Cys Trp Leu Cys Arg Ala
                                200

656                     671                     686                     701                     716
CTG ATC AAG CGG ATC CAA GCC ATG ATT CCC AAG GGT GCG CTA CGT GTG GCA GTG GCC CAG GTG TGC
Leu Ile Lys Arg Ile Gln Ala MET Ile Pro Lys Gly Ala Leu Arg Val Ala Val Ala Gln Val Cys

            731                     746                     761                     776
CGC GTG GTA CCT CTG GTG GCG GGC GGC ATC TGC CAG TGC CTG GCT GAG CGC TAC TCC GTC ATC CTG
Arg Val Val Pro Leu Val Ala Gly Gly Ile Cys Gln Cys Leu Ala Glu Arg Tyr Ser Val Ile Leu
                                                        250

    791                     806                     821                     836                     851
CTC GAC ACG CTG CTG GGC CGC ATG CTG CCC CAG CTG GTC TGC CGC CTC GTC|CTC|CGG TGC TCC ATG
Leu Asp Thr Leu Leu Gly Arg MET Leu Pro Gln Leu Val Cys Arg Leu Val|Leu|Arg Cys Ser MET

                866                     881                     896                     911
GAT GAC AGC GCT GGC CCA AGG TCG CCG ACA GGA GAA TGG CTG CCG CGA GAC TCT GAG TGC CAC CTC
Asp Asp Ser Ala Gly Pro Arg Ser Pro Thr Gly Glu Trp Leu Pro Arg Asp Ser Glu Cys His Leu
                                                                                300
```

FIG.6B

```
              926                      941                      956                      971
TGC ATG TCC GTG ACC ACC CAG GCC GGG AAC AGC AGC GAG CAG GCC ATA CTA CAG GCA ATG CTC CAG
Cys MET Ser Val Thr Thr Gln Ala Gly Asn Ser Ser Glu Gln Ala Ile Leu Gln Ala MET Leu Gln
                                                                350

                         1001                     1016                     1031                     1046
GCC TGT GTT GGC TCC TGG CTG GAC AGG GAA AAG TGC AAG CAA TTT GTG GAG CAG CAC ACG CCC CAG
Ala Cys Val Gly Ser Trp Leu Asp Arg Glu Lys Cys Lys Gln Phe Val Glu Gln His Thr Pro Gln

                 1061                     1076                     1091                     1106
CTG CTG ACC CTG GTG CCC AGG GGC TGG GAT GCC CAC ACC ACC TGC CAG GCC CTC GGG GTG TGT GGG
Leu Leu Thr Leu Val Pro Arg Gly Trp Asp Ala His Thr Thr Cys Gln Ala Leu Gly Val Cys Gly

    1121                     1136                     1151                         1170       1180
ACC ATG TCC AGC CCT CTC CAG TGT ATC CAC AGC CCC GAC CTT TGA TGAGAACTCA GCTGTCCAGC
Thr MET Ser Ser Pro Leu Gln Cys Ile His Ser Pro Asp Leu  .

      1190       1200       1210       1220       1230       1240       1250       1260
TGCAAAGGAA AAGCCAAGTG AGACGGGCTC TGGGACCATG GTGACCAGGC TCTTCCCCTG CTCCCTGGCC CTCGCCAGCT

      1270       1280       1290       1300       1310       1320       1330       1340
GCCAGGCTGA AAAGAAGCCT CAGCTCCCAC ACCGCCCTCC TCACCGCCCT TCCTCGGCAG TCACTTCCAC TGGTGGACCA

      1350       1360       1370       1380       1390       1400       1410       1420
CGGGCCCCCA GCCCTGTGTC GGCCTTGTCT GTCTCAGCTC AACCACAGTC TGACACCAGA GCCCACTTCC ATCCTCTCTG

      1430       1440       1450       1460       1470       1480       1490       1500
GTGTGAGGCA CAGCGAGGGC AGCATCTGGA GGAGCTCTGC AGCCTCCACA CCTACCACGA CCTCCCAGGG CTGGGCTCAG

      1510       1520       1530       1540       1550       1560       1570       1580
GAAAAACCAG CCACTGCTTT ACAGGACAGG GGGTTGAAGC TGAGCCCCGC CTCACACCCA CCCCCATGCA CTCAAAGATT
```

FIG.6C

```
      1590       1600       1610       1620       1630       1640       1650       1660
GGATTTTACA GCTACTTGCA ATTCAAAATT CAGAAGAATA AAAAATGGGA ACATACAGAA CTCTAAAAGA TAGACATCAG

      1670       1680       1690       1700       1710       1720       1730       1740
AAATTGTTAA GTTAAGCTTT TTCAAAAAAT CAGCAATTCC CAGCGTAGTC AAGGGTGGAC ATGCACGCGT CTGGCATGAT

      1750       1760       1770       1780       1790       1800       1810       1820
GGGATGGCGA CCGGGCAAGC TTTCTTCCTC GAGATCGTCT GCTGCTTGAG AGCTATTGCT TTGTTAAGAT ATAAAAAGGG

      1830       1840       1850       1860       1870       1880       1890       1900
GTTTCTTTTT GTCTTTCTGT AAGGTGGACT TCCAGCTTTT GATTGAAAGT CCTAGGGTGA TTCTATTTCT GCTGTGATTT

      1910       1920       1930       1940       1950       1960       1970       1980
ATCTGCTGAA AGCTCAGCTG GGGTTGTGCA AGCTAGGGAC CCATTCCTAT GTAATACAAT GTCTGCACCA ATGCTAATAA

      1990       2000       2010
AGTCCTATTC TCTTTTATGA GAAAAAAAAA AAAAAAA
```

FIG.6D

10th codon of pUC19 lacZ gene

SalI — EcoRI — 214.1 insert →

............TGC AGG TCG ACT CTA GAG GAT CCC CGG GTA CCG AGC TCG AAT TC CGG GAG TGC............

Cys Arg Ser Thr Leu Glu Asp Pro Arg Val Pro Ser Ser Asn Arg Glu Cys

↓ SalI

...............TGCAGG TCGACTCTAG...............

...............ACGTCCAGCT GAGATC...............

↓ $T_4$ Polymerase fill-in

...............TGCAGGTCGATCGACTCTAG...............

...............ACGTCCAGCTAGCTGAGATC...............

↓

............TGC AGG TCG ATC GAC TCT AGA GGA TCC CCG GGT ACC GAG CTC GAA TTC CGG GAG TGC............

Cys Arg Ser Ile Asp Ser Arg Gly Ser Pro Gly Thr Glu Leu Glu Phe Arg Glu Cys

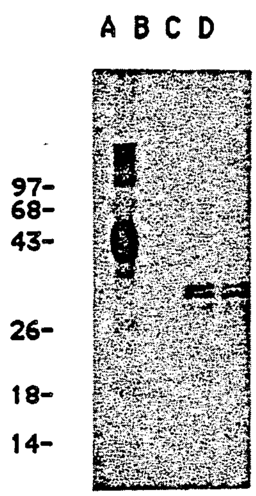


FIG.7

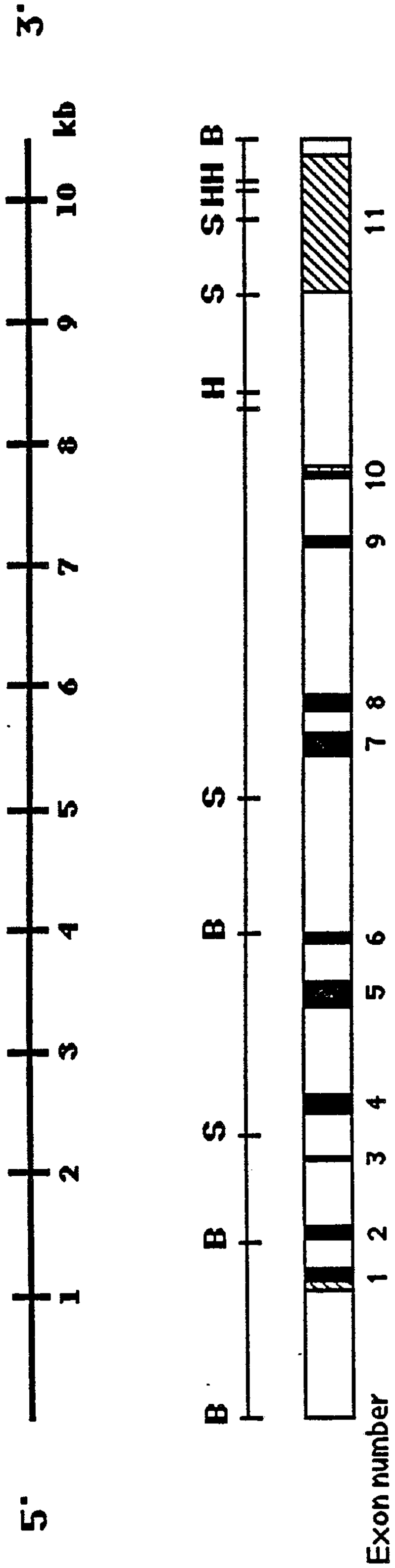


FIG.8a

FIG. 8B-1

```
        10         20         30         40         50         60         70
GGATCCTCCC TCCTCGGCCT CCCAAAGTGC CAGGATTACA GGAGTGAGCC ACCACACCCA GCCCCATCTC

        80         90        100        110        120        130        140
TTTTCATCAT GGTACTAATT CCTGCCCGTC CACCCACAAA AGCACTGTAG TCGTTCCCGA GTATAGAGGC

       150        160        170        180        190        200        210
CTGTGAGCCT CCACTAGGGA GAGGGCTCCT GCAGAGATCA GATAAATTGA TCACAATGGC TGGGGTGGTG

       220        230        240        250        260        270        280
GCAATGTGCT AATGCTCTCT TTCTTCCACT CAAGATATCC TCTGTCTCCC TCAGCCTGTG AGCTTTTTCT

       290        300        310        320        330        340        350
CCAGTGTGCT CTGCCAGTGG GGGCCCTGCC TGAGAGCCCC TGCAGCTGCA GAGGACAGTT TCTTTCTGCT

       360        370        380        390        400        410        420
GAACCATCGC AGCTATGCCC CAGCCCCTAC CCTGGAGGGG TCCCCAGGGG CCATGGGCAG CACCTCCTGT
```

# FIG. 8B-2

| 430 | 440 | 450 | 460 | 470 | 480 | 490 |
|---|---|---|---|---|---|---|
| ATAGGGCTGT | CTGGGAGCCA | CTCCAGGGCC | ACAGAAATCT | TGTCTCTGAC | TCAGGGTATT | TTGTTTTCTG |
| 500 | 510 | 520 | 530 | 540 | 550 | 560 |
| TTTTGTGTAA | ATGCTCTTCT | GACTAATGCA | AACCATGTGT | CCATAGAACC | AGAAGATTTT | TCCAGGGGAA |
| 570 | 580 | 590 | 600 | 610 | 620 | 630 |
| AAGGTAAGGA | GGTGGTGAGA | GTGTCCTGGG | TCTGCCCTTC | CAGGGCTTGC | CCTGGGTTAA | GAGCCAGGCA |
| 640 | 650 | 660 | 670 | 680 | 690 | 700 |
| GGAAGCTCTC | AAGAGCATTG | CTCAAGAGTA | GAGGGGGCCT | GGGAGGCCCA | GGGAGGGGAT | GGGAGGGGAA |
| 710 | 720 | 730 | 740 | 750 | 760 | 770 |
| CACCCAGGCT | GCCCCCAACC | AGATGCCCTC | CACCCTCCTC | AACCTCCCTC | CCACGGCCTG | GAGAGGTGGG |
| 780 | 790 | 800 | 810 | 820 | 830 | 840 |
| ACCAGGTATG | GAGGCTTGAG | AGCCCCTGGT | TGGAGGAAGC | CACAAGTCCA | GGAACATGGG | AGTCTGGGCA |
| 850 | 860 | 870 | 880 | 890 | 900 | 910 |
| GGGGGCAAAG | GAGGCAGGAA | CAGGCCATCA | GCCAGGACAG | GTGGTAAGGC | AGGCAGGAGT | GTTCCTGCTG |

FIG. 8B-3

```
       920        930        940        950        960        970        980
GGAAAAGGTG GGATCAAGCA CCTGGAGGGC TCTTCAGAGC AAAGACAAAC ACTGAGGTCG CTGCCACTCC

       990       1000       1010       1020       1030       1040       1050
TACAGAGCCC CCACGCCCCG CCCAGCTATA AGGGGCCATG CCCCAAGCAG GGTACCCAGG CTGCAGAGGT

                     1068                           1083                           1098
GCC ATG GCT GAG TCA CAC CTG CTG CAG TGG CTG CTG CTG CTG CTG CCC ACG CTC
    MET Ala Glu Ser His Leu Leu Gln Trp Leu Leu Leu Leu Leu Pro Thr Leu

      1113             1130       1140       1150       1160       1170
TGT GGC CCA GGC ACT G GTGAGTCTCC CCCAGCCTCC CCTCTCCTAG GCAGCTCCAC CACTCACTGA
Cys Gly Pro Gly Thr A

      1180       1190       1200       1210       1220       1230       1240
GCACTGCTTT GTGCTAGGCA TTAACCCAAG TCTGTCCTCA TTTTAAAGAC AAGGCAGCTG GGGTTCAGAG

      1250       1260       1270       1280       1290       1300       1310
AGGGTTCAGA GCTTATCCAA GGTCACACAG CTGGCGGGTC CAGGAGCAGG TGGAACCCAG AGCTGTCTGA

      1320       1330       1340       1350       1360       1370       1380
CGTCCACATG TTTAATGGCC TCACACTCCC AGCAAAACTG GGTCTAGAGG GTGGGTGAAA TCATGATGCC
```

FIG. 8B-4

```
      1390       1400       1410       1420       1430
AGGTGTGTAG CCTGGATCCT GATTAAGGTT GCTCTGGCCC CAAACCACAG  CT GCC TGG ACC
                                                        la Ala Trp Thr

1444                1459                1474                1489
ACC TCA TCC TTG GCC TGT GCC CAG GGC CCT GAG TTC TGG TGC CAA AGC CTG GAG
Thr Ser Ser Leu Ala Cys Ala Gln Gly Pro Glu Phe Trp Cys Gln Ser Leu Glu

      1504                1519                1534                1549
CAA GCA TTG CAG TGC AGA GCC CTA GGG CAT TGC CTA CAG GAA GTC TGG GGA CAT
Gln Ala Leu Gln Cys Arg Ala Leu Gly His Cys Leu Gln Glu Val Trp Gly His

                 1568       1578       1588       1598       1608       1618
GTG GGA GCC GTGAGTACCA CCAAGGATGC ATGGCAACTG GGGGTCTGAA ATGAAGGGTG CTGGGTGGGC
Val Gly Ala

      1628       1638       1648       1658       1668       1678       1688
TCTGGATGGG CAGGAGGAGA GTGGAGCCCC CATAGGGGAT GGATGAGATG AAATGGGATG AGATGAAATG

      1698       1708       1718       1728       1738       1748       1758
AGATAGGATA AAATGGAATG GGATGGATGC GATGGGATAC GATGACATAG AATAGATGGA GTCGGATGAA
```

FIG. 8B-5

```
      1768       1778       1788       1798       1808       1818       1828
TGGGATGGGA TGGGATGGAT GGGAGGGGAA GGGATAGGAT AGGATGACAT AGAATAAAGA TGGATGGGAT

      1838       1848       1858       1868       1878       1888       1898
GGGATGGGAT GGGATGGGAT GACACAGAAT AAAGATGGAT GGATTGGGAT GGATGAATAG AAGAGATGGA

      1908       1918       1928       1938       1948       1958       1968
TGGGATAAAT TGATATGGAT GAGATGGGAC AAGTTGGGCT GGTGGGCAGC TGCATGTGCC TTGGAGTGCT

      1978       1988       1998       2008       2018       2028       2038
CTGTTGGCCT CTTCCTAAGA GAACCTCCCC ATTGGAGCTG GGAGCCTCCC CCACTCATGT GTCCTCCACC

      2048       2058                   2074                 2089
TTGGGGCCCC TCCCTCCCCA G GAT GAC CTA TGC CAA GAG TGT GAG GAC ATC GTC CAC
                        Asp Asp Leu Cys Gln Glu Cys Glu Asp Ile Val His

       2104               2119                           2141       2151
ATC CTT AAC AAG ATG GCC AAG GAG GCC ATT TTC CAG GTAATGATGC CCAGATCCTG
Ile Leu Asn Lys MET Ala Lys Glu Ala Ile Phe Gln

      2161       2171       2181       2191       2201       2211       2221
GATGAAGGTT GGGGCCCAAG AGATGAGGGA CAGAGCAGGG AAGAGCTGAG CCCCCTAAAG GGGCCATTTC
```

FIG. 8B-6

```
      2231      2241      2251      2261      2271      2281      2291
CAGGCTGAGG AGGAGGCCTG GGTGCCTGGG AAGTCCCAGC TCCTCCTGGC TGGGAGCAGG TCATGGCCCT

      2301      2311      2321      2331      2341      2351      2361
GAGCTCAATA GCACAGCCAG AGATGGTCTT CCCTGAGGGG AAGGGCCCCT ACATGTGCCC AACTACTTAA

      2371      2381      2391      2401      2411      2421      2431
CTCCTTGGCA CTCGTGAACT CCAGCACCCT GGGGGATTAG GGGTCAGTCT GCCCTGGTGG GGCCTTGTGT

      2441      2451      2461      2471      2481      2491
CCAGGGACTT GGGCGGGGTA GACCTCAGAG AGGCCCAGCT GACGGCCCCC TCTGGCCTCC CAG

                    2509                2524                2539
GAC ACG ATG AGG AAG TTC CTG GAG CAG GAG TGC AAC GTC CTC CCC TTG AAG CTG
Asp Thr MET Arg Lys Phe Leu Glu Gln Glu Cys Asn Val Leu Pro Leu Lys Leu

    2554                2569                2584                2599
CTC ATG CCC CAG TGC AAC CAA GTG CTT GAC GAC TAC TTC CCC CTG GTC ATC GAC
Leu MET Pro Gln Cys Asn Gln Val Leu Asp Asp Tyr Phe Pro Leu Val Ile Asp
```

FIG. 8B-7

```
                  2614                     2630         2640         2650         2660         267
TAC TTC CAG AAC CAG ATT GTGAGGGCTG CAAGCTCACC TCCTGCCTGC CTCCCCACGC AGGCCCCTG
Tyr Phe Gln Asn Gln Ile

      2680       2690       2700       2710       2720       2730       2740
GCCCACCCAT GGGGGAGCCA CACACACAGC ACCCCAGCCA GCCAGACACA CACACACACA CACACACACA

      2750       2760       2770       2780       2790       2800       2810
CAGCACCCAA GCCGGCCAGA CACAAACACA CAGCACCCCA GCCAGCCGGA CACACACACA CACACACACA

      2820       2830       2840       2850       2860       2870       2880
CACAACACCC CAGCTGGCCG GACACACACA CACACAGTAC CCCAGCTGGC CGGACACACA CACACACAGC

      2890       2900       2910       2920       2930       2940       2950
ACCCTATCCA GACACATACA CACACACAGT ACCCCAGCCA GCTGGAAACA CACACACACA CAGCACTCCA

      2960       2970       2980       2990       3000       3010       3020
TCCAGACACA TACCCACACA GTACCCCAGC CAGCCAGACA CACACACACA CACACACACA CACACACACA

      3030       3040       3050       3060       3070       3080       3090
CAGAGCACAC ACACAGCACC CCAGCTGGCC ACACACACAC ACACACACAC CCTGTCCACA AAGGGCCTAG
```

FIG. 8B-8

```
      3100       3110       3120       3130       3140       3150       3160
GAAACTACGT GCCCTTCAGC CATGCACCCG ACCATGGGCC CCCAGGTTCA GGTGCACACG GTGGGCCTGT

      3170       3180       3190       3200       3210       3220       3230
ACGCTCACAC ACCCTTACAC CCTCACTCTC ACACACATGC TTACACACTT ATTCATTCTC ACATATATGC

      3240       3250       3260       3270       3280       3290       3300
TCATGCTCAT TCACACACAA TCCCGGGCCA CCTGCCCTAA AGTCCCCACA CAGCCCTATC TTTGCCTTTT

      3310       3320       3330       3340       3350       3360       3370
GTCCCCCCAC ATAGAGTTCT AAACCACAGC ACCCCCACTA GGCCTGCTTC CTCCCATTCC AGTGGTCCCT

      3380       3390       3400       3410       3420       3430       3440
GAGCCCTTGG GCCGGCCTGA ATAGGGGTGG GCTTCCCTCC CAGACCCTAA CACTCCCACC CTGTGCTGTG

                         3461                         3476                         3491
CCCCAG GAC TCA AAC GGC ATC TGT ATG CAC CTG GGC CTG TGC AAA TCC CGG CAG
       Asp Ser Asn Gly Ile Cys MET His Leu Gly Leu Cys Lys Ser Arg Gln
```

FIG. 8B-9

```
          3506                     3521                     3536
CCA GAG CCA GAG CAG GAG CCA GGG ATG TCA GAC CCC CTG CCC AAA CCT CTG CGG
Pro Glu Pro Glu Gln Glu Pro Gly MET Ser Asp Pro Leu Pro Lys Pro Leu Arg

3551                3566                     3581                3596
GAC CCT CTG CCA GAC CCT CTG CTG GAC AAG CTC GTC CTC CCT GTG CTG CCC GGG
Asp Pro Leu Pro Asp Pro Leu Leu Asp Lys Leu Val Leu Pro Val Leu Pro Gly

        3611                3626                     3645       3655       3665
GCC CTC CAG GCG AGG CCT GGG CCT CAC ACA CAG GTGAGGGAGG CCCCCACAGC CAGTAAAGTG
Ala Leu Gln Ala Arg Pro Gly Pro His Thr Gln

      3675       3685       3695       3705       3715       3725       3735
GAGATCCAGA GGGCTAGAGC CACCTCCGAA GCCCATGGGC ACTGGGCCCT GGGAGAGGCA GAGCCGGGAA

      3745       3755       3765       3775       3785       3795       3805
GGTGATAGGA AGCTCCAGGC AGGGCCTAAG GGAGGAGGGA GAGAAAGGGA GGAAGAGAGA GGGGAGGAGA

      3815       3825       3835       3845       3855
GCCTGGAGGA CTCTTCTCCC AGCACCCAGC CTGGCCTCCA CCTGATTCTT TCCCCAG GAT CTC
                                                              Asp Leu
```

# FIG. 8B-10

```
      3877                     3892                     3907                     3922
TCC GAG CAG CAA TTC CCC ATT CCT CTC CCC TAT TGC TGG CTC TGC AGG GCT CTG
Ser Glu Gln Gln Phe Pro Ile Pro Leu Pro Tyr Cys Trp Leu Cys Arg Ala Leu

                3937                     3952         3962          3972          3982
ATC AAG CGG ATC CAA GCC ATG ATT CCC AAG GTGAGGCATC CAGGGCCTCA AGAGCCCAGG
Ile Lys Arg Ile Gln Ala MET Ile Pro Lys

      3992        4002        4012        4022        4032        4042        4052
AGCACACGCA TACCTGTAGC TCCCTGCAGC TCCCACCTCT CTCCCAACTC ACACCCCCGT CAGACCCAGC

      4062        4072        4082        4092        4102        4112        4122
TGGCTGCCAG AAGTTAGGAG GGGAGAGAGC CGCTTGTGCA TTGCCCCCAC CCAGGGACCC TGGGCTCAGG

      4132        4142        4152        4162        4172        4182        4192
CTCAGGCCTG GTAGGTGCCA GGTACAGTTC ATGCAACAAA CATTAAGCCC CCACTGTATG GAGGTGCCAG

      4202        4212        4222        4232        4242        4252        4262
CCAGGAGCCA AAGTACAAAA ACGGACAAGA CGCAGCTTTG TCCTCCAGCA GCTCACCATC TGATGGAGAA

      4272        4282        4292        4302        4312        4322        4332
AGATCCCCAG AGGTCTCTGT AGAAAGGTTG CTTTGATCTT TCAAGAGGGG AATTTCCACA GATAGATTCC
```

FIG. 8B-11

| | | | | | | |
|---|---|---|---|---|---|---|
| 4342 | 4352 | 4362 | 4372 | 4382 | 4392 | 4402 |
| CCATCCTTGC | CTGAGTCCAA | CTTGGAGTCT | TCCAGACCTG | CAGTGGCTAT | TGTCCAATGG | CCCCGCCAGC |
| 4412 | 4422 | 4432 | 4442 | 4452 | 4462 | 4472 |
| CCAGGGCTAC | CTTGCCCAAA | TTGGGGCCCA | AATGAGGAAA | GGCCCTGCCC | CCTCAGCCTT | TCCCAGATTG |
| 4482 | 4492 | 4502 | 4512 | 4522 | 4532 | 4542 |
| GGTTGCGTGG | GCCACCAGGG | GCACAAGGCA | GCAGGTGAGG | TTCCTGCTGA | GGCAGGTGGT | TCACTTGAGC |
| 4552 | 4562 | 4572 | 4582 | 4592 | 4602 | 4612 |
| CCAGGAGTTC | AAGACCAGCT | TGGGCAACAT | GGCGAAACCC | CGTCTCTACT | AAGAATACAA | AAATTAGCCA |
| 4622 | 4632 | 4642 | 4652 | 4662 | 4672 | 4682 |
| GATGTGACAG | GTGCCTGTAG | TCCCAGCTAC | TCGGGAGGCT | GAGGCAGGAG | AATCACTTGA | ACCCAGGAGG |
| 4692 | 4702 | 4712 | 4722 | 4732 | 4742 | 4752 |
| CGGAGGTTGC | AGTGAGCCGA | CATCACGCCA | CTGTACTCTA | GCCTGGGTGA | CAGAGCAAGA | CTCTGTCTCA |

# FIG. 8B-12

```
      4762       4772       4782       4792       4802       4812       4822
AAAAAAAAGA AAGAAGGAAA GATCACTGCA GAGATTGCAG TGAGAGGTGA TGGGACAGGG ACGGAGCTGA

      4832       4842       4852       4862       4872       4882       4892
GGGCTGGCCT GGGGATGCAT TTGGGAGGTG GGCCCACTGC TATGGGCATG GATGGGCCTG GAGCGTGAGG

      4902       4912       4922       4932       4942       4952       4962
ACCAGGGAGG ACTCCAAAGT GACTTTTACA CACTGGCCAG AGCAACCAGC CCTCTGTAAT GCCAGCAGCT

      4972       4982       4992       5002       5012       5022       5032
GAGATGGGGA GACTAAAGAA GAAAACAGGT TTGAGCAAAA AAACAGAGAG CTCCCTCCTG GCCATGTTGA

      5042       5052       5062       5072       5082       5092       5102
GTTCAAGATG CCTGTGTGAA GTGCAGGAGA GGAGAGTCAG GCAAGCAGCT GAATCCCAAG CATTGGGGGA

      5112       5122       5132       5142       5152       5162       5172
AGGTCAGGTC CACCATGTCA GTCTGAGAGT CACTAGCTGT GGGCCAGAGC CTTTGGGGCC AGACGTAGGT

      5182       5192       5202       5212       5222       5232       5242
CTGAAGCTGG CTCCTACACT CAGTGACCCT GTGTGAGTCC CCTGCATCCC CTGGACTCTC TGATCCCCAG
```

FIG. 8B-13

```
       5252      5262      5272      5282      5292      5302      5312
TGTCCTTATT TGTGAATAGC CTTGCCCTCC CTTCTAGAAG AGAATGAGGG AATGCGTAGG AAGTGCCCAG

       5322      5332      5342      5352      5362      5372      5382
CTGGGTGCTG GGCAGAGAGT GGAGGCTTGC CAAGTGAAGG TCCCATGCTG GCCTCTCTCC GCCCCCGCCC

                   5400                          5415                          5430
CAG GGT GCG CTA CGT GTG GCA GTG GCC CAG GTG TGC CGC GTG GTA CCT CTG GTG
    Gly Ala Leu Arg Val Ala Val Ala Gln Val Cys Arg Val Val Pro Leu Val

        5445                          5460                          5475                          5490
GCG GGC GGC ATC TGC CAG TGC CTG GCT GAG CGC TAC TCC GTC ATC CTG CTC GAC
Ala Gly Gly Ile Cys Gln Cys Leu Ala Glu Arg Tyr Ser Val Ile Leu Leu Asp

                5505                          5520                          5535
ACG CTG CTG GGC CGC ATG CTG CCC CAG CTG GTC TGC CGC CTC GTC CTC CGG TGC
Thr Leu Leu Gly Arg MET Leu Pro Gln Leu Val Cys Arg Leu Val Leu Arg Cys

    5550                          5565                    5578      5588      5598      5608
TCC ATG GAT GAC AGC GCT GGC CCA A GTGAGCCCA CTGCCCCCTC CTTAGCCCAA TGCCCGCTCT
Ser MET Asp Asp Ser Ala Gly Pro A
```

# FIG. 8B-14

```
      5618       5628       5638       5648       5658       5668       5678
CCTCCTCCCC CTACCCTGCC ACTGCATGAC CCTCTCCCTC TGTGGTCCCA CTGCAATGCA CCAAGGAGGA

      5688       5698       5708       5718       5728       5738
CAGAAACCAA ACACCTCTGT AGGGTGGCCT TGCCTGCTTT CCCCCTAATG CTCACATCTC CAG GG
                                                                       rg

            5755                 5770                 5785
TCG CCG ACA GGA GAA TGG CTG CCG CGA GAC TCT GAG TGC CAC CTC TGC ATG TCC
Ser Pro Thr Gly Glu Trp Leu Pro Arg Asp Ser Glu Cys His Leu Cys MET Ser

5800                5815                 5830                 5845
GTG ACC ACC CAG GCC GGG AAC AGC AGC GAG CAG GCC ATA CCA CAG GCA ATG CTC
Val Thr Thr Gln Ala Gly Asn Ser Ser Glu Gln Ala Ile Pro Gln Ala MET Leu

        5860                 5875                        5897       5907
CAG GCC TGT GTT GGC TCC TGG CTG GAC AGG GAA AAG GTATGGGCTG GGCACATGGG
Gln Ala Cys Val Gly Ser Trp Leu Asp Arg Glu Lys

      5917       5927       5937       5947       5957       5967       5977
GACTCATGGT CAGGGCCCGT TCAAGGCAGA AGGCTGAGCC CAGGAAAGGC TTTGCAGCCA GAGACACCTA

      5987       5997       6007       6017       6027       6037       6047
GGATGGGCCA GAATGGAGCA CAGACAGGCA GACAGGATGT GGGGCAGACA ATGGTGGGAC TGTAAGTTAG
```

FIG. 8B-15

| | | | | | | |
|---|---|---|---|---|---|---|
| 6057 | 6067 | 6077 | 6087 | 6097 | 6107 | 6117 |
| GGCAGAGCCT | GCTAAGGGTT | AGGAGTCGCC | TCTGGACAAA | GGGCTGTGGG | CTCCAGAGGA | CCAGCAGGCC |
| 6127 | 6137 | 6147 | 6157 | 6167 | 6177 | 6187 |
| CTCTTCACGG | GCTGAGTGAG | CACCAGGCAA | GCCTTCAGAG | GCCTGGTTAT | CTACCAGGAG | ATGAGTAATG |
| 6197 | 6207 | 6217 | 6227 | 6237 | 6247 | 6257 |
| CTAGGGCCAG | TTCAAGCCAG | GAAAGGGACT | AGCCTTCTCT | CCAGGGTCCT | GATCCCTTTA | CTGCCCCCAC |
| 6267 | 6277 | 6287 | 6297 | 6307 | 6317 | 6327 |
| ACTCCTCAAG | GTGTGACTCA | CTCAGGACAA | ACCCATTGGC | AAAAGGAGAG | GGCTGGACTT | GAAGGTCCTA |
| 6337 | 6347 | 6357 | 6367 | 6377 | 6387 | 6397 |
| GGGCCCTTGC | CAATACTCAG | TCAATGACAG | GAAATTCCCT | TTTTTTTTTT | TTTTTTTTTT | TTTTTGAGAT |
| 6407 | 6417 | 6427 | 6437 | 6447 | 6457 | 6467 |
| GGAGTTTTGC | TCTTGTTGCC | CAGGCTGGAG | TGCAATGGCA | CAATCTTGGC | TCACTGCAAC | CTCTGCCTCC |

FIG. 8B-16

```
      6477       6487       6497       6507       6517       6527       6537
GGGTTCAGGC GATTCTCCTG CCTCAGCCTC TTGAGTAGCT GGGATTACAG GCATGTGCTA CCAGGCCCGG

      6547       6557       6567       6577       6587       6597       6607
CTAATTTTTG TATTTTTAGT AGAGACAAGG TTTCACCATA TTGGTCAGGC TGGTCTCGAA CCCCTGACCT

      6617       6627       6637       6647       6657       6667       6677
GAAGTGATCT GCCCGCCTTG GCCTCCCAAA GTGCTGGGAT TACAGGCATA AGCCACTGCA CCCGGACAGG

      6687       6697       6707       6717       6727       6737       6747
AAATTCCCTT CTTAAAGCGA GATCCTGTCC TGAGGAAAGC CAGCTGATGC TCTTCCCAGG AGGCAGCTGT

      6757       6767       6777       6787       6797       6807       6817
CCACACTGTG CTCCCTGCTC AGCAACTCCC AAGCCTCCCG ACTGCCCATC ACATCTGGTC TCAAGGACCA

      6827       6837       6847       6857       6867       6877       6887
GATGAACGTT AAGGTTCCTT CTAGAACTGA AATGGAGGTG GAGGGAGGGG AGGGTGGTGG CTGAGATTCC

      6897       6907       6917       6927       6937       6947       6957
ACCCCTCTGC CTGAGTCCTC CGTCTCCAGT GTCGCCTGCT TTTCTGATGG AAGTCCTCCA TTTCAGCCTG
```

FIG. 8B-17

```
      6967       6977       6987       6997       7007       7017       7027
GCTCCAGTTT GTTAAGGGTT TCAACTGCAG CCAGAGGTGT TCCGTGAGGG CTGATGGAGG AGTCGGGAGG

      7037       7047       7057       7067       7077       7087       7097
GAGCCCTAGA GTGATCCAGA GATGTGGAGA GGCCCAGGAC CACACGACAG GAGAGTCCTG CAAAGGGACC

      7107       7117                         7136                 7151
TCCACAGCTG TGTGTCTCCC TCAG TGC AAG CAA TTT GTG GAG CAG CAC ACG CCC CAG
                           Cys Lys Gln Phe Val Glu Gln His Thr Pro Gln

            7166                7181                7196                7212
CTG CTG ACC CTG GTG CCC AGG GGC TGG GAT GCC CAC ACC ACC TGC CAG GTACACCCAA
Leu Leu Thr Leu Val Pro Arg Gly Trp Asp Ala His Thr Thr Cys Gln

      7222       7232       7242       7252       7262       7272       7282
CCCCTCCCAA GTTGGTCCTA GGACTTCCCT TGGCTCCCAG AGCCCCCACC CTTTGGGCCC GTGATCCTCA

      7292       7302       7312       7322       7332       7342       7352
GAGGCCTCAC TCCCCTGGGT CCAAGGTGGT CCCAGGTGCA CGGGCCAGGG ACTGGGAGGC ACCCCTCTCT
```

FIG. 8B-18

```
      7362      7372      7382      7392      7402      7412      7422
GTTTCAGTGT AAAAAATCAT GAGAGCATGG AAAAGGGGGA TGGGAAGGGA GGGATGGCCT GAGGAGTGCG

      7432      7442      7452      7462      7472      7482      7492
GCTGGATGTC CATTATAGGA TGGGGCTGTG TTCCCTGGCC AGTGTGTGCT GGTGGGGTGG GGGTACAAAG

      7502      7512      7522      7532      7542      7552      7562
TGGGTGTTCT GGAGTGAACA TCTCACCTCC TCAGGCTCTA AACCCTAAGG CCTGTGGCTC AGGGAGTGGC

      7572      7582      7592      7602      7612      7622      7632
CGAGGGGTCT ACAGAGTCAC ACTGGTAGCA CCCACTAGGC GGGAGGTGGA GTGAGTGCTG TTCTTTCCCG

      7642      7652      7662      7672      7682      7692
GAAGAGCTGG GTGTGGGGAG CTGAGGGGGC CCAGGCCTCA GCCCTGGTGC TGTCCCTGTG ACAG

                    7711                7726                7741
GCC CTC GGG GTG TGT GGG ACC ATG TCC AGC CCT CTC CAG TGT ATC CAC AGC CCC
Ala Leu Gly Val Cys Gly Thr MET Ser Ser Pro Leu Gln Cys Ile His Ser Pro

   7756           7769      7779      7789      7799      7809      7819
GAC CTT TGA TGAGAACTCA GCTGTCCAGG TGAGTCCAGG CCCCCAGTTG CGGGGAGGTA AGGGGGCAGG
Asp Leu
```

FIG. 8B-19

| | | | | | | |
|---|---|---|---|---|---|---|
| 7829 | 7839 | 7849 | 7859 | 7869 | 7879 | 7889 |
| TCCTGACCAT | CAGGGCATGG | GAGGCCCTTC | TGCTCCCCAA | GCAGGAAGAG | GCGGCCACTC | CTGCCGGCTG |
| 7899 | 7909 | 7919 | 7929 | 7939 | 7949 | 7959 |
| CTCCATCCTC | CCTCTCACCG | CACAGCTGGA | GGCTCCTGAG | GGCTTCTGGC | TGGCCATCAG | GAAAACACCC |
| 7969 | 7979 | 7989 | 7999 | 8009 | 8019 | 8029 |
| TTTCCGGACC | CCGAGCACTG | CCCCGCCCAG | AACCCCAGTC | ACTGAGTGCC | CAACCCCCAG | CTTCCCCCCC |
| 8039 | 8049 | 8059 | 8069 | 8079 | 8089 | 8099 |
| AACCCCCCGC | CCTGCCCTGT | CCCAGGCCTC | CCTCTCAGAG | CTTGCCCCAG | GGACTCTCTG | GCCCTCAGGG |
| 8109 | 8119 | 8129 | 8139 | 8149 | 8159 | 8169 |
| TTCAATGTAT | TCTGACCAAG | GCCAAGCTTT | CCTGGGGCTC | AGGGAAAATC | ACACTTTGCT | ACCCGAAGCT |
| 8179 | 8189 | 8199 | 8209 | 8219 | 8229 | 8239 |
| GTATCCCCTC | AGATGCCAGG | AAGGCCGTGA | TCATCTGACT | CCACCCTCCT | GAGACACATT | CTCTCCCTGA |

# FIG. 8B-20

| | | | | | | |
|---|---|---|---|---|---|---|
| 8249 | 8259 | 8269 | 8279 | 8289 | 8299 | 8309 |
| CTGTCCTGTT | CTAAGTCAGC | GGAGCACCTT | AGGATGGAGG | GGTGGAGGCG | AGGCCAGATG | CAGCCTCTGT |
| 8319 | 8329 | 8339 | 8349 | 8359 | 8369 | 8379 |
| GAACAGGTGC | CTGGAGGCTG | GGAAATGACC | CTGAGAGGGC | AGGACACAGC | AACCGTGGGC | TTAAGGTGAC |
| 8389 | 8399 | 8409 | 8419 | 8429 | 8439 | 8449 |
| CTTGAGAGCA | AGCTTGGCCC | ACTTTACAAT | TCTGTTCAGA | GCCAGCCCCT | AACATGGTGG | TCATTTATTC |
| 8459 | 8469 | 8479 | 8489 | 8499 | 8509 | 8519 |
| ATTTGTTCCC | TCATTTTAAA | AAATGTAAGG | CCAGGCATGG | TGGCTCACGC | CGGTAATCCC | AGCACTTTGG |
| 8529 | 8539 | 8549 | 8559 | 8569 | 8579 | 8589 |
| GAGGCCGAGG | CAGGCAGATC | ACCTGAGGTC | AGGAGTTCGA | GACTAGCCTG | GCCAACATGG | CGAAACCCTG |
| 8599 | 8609 | 8619 | 8629 | 8639 | 8649 | 8659 |
| TCTCTACTAA | AAATATTTTT | TAAAAATTAG | CTGAGCATGG | TGGCAGGTGC | CTGTAATCCC | AGCTACTCAG |
| 8669 | 8679 | 8689 | 8699 | 8709 | 8719 | 8729 |
| GACGCTTAGG | CAGGAGAATC | ACTTGAACCT | GGGAGGCGAA | GGTTGCGGTG | TGCTGAGATC | GTGCCACTGC |

FIG. 8B-21

```
      8739       8749       8759       8769       8779       8789       8799
ACTCTAGCCT AGGCAACAGA GCACAACTCT GTCTCAGGAA AAAAAAAAAA AAAAAAAAAG GTATTTCTTT

      8809       8819       8829       8839       8849       8859       8869
GCTGGGCGCA GTGGCTCACA CCTGTAATCC CAGCACTTTG GGAGACCGAG GCGAGTGGAT CACTTGAGGT

      8879       8889       8899       8909       8919       8929       8939
CAGGAGTTCA AGACCAGCCT TACCAACATG ATGAAACCCC GTATCTACTA AAAAAAAAAA AAAAAAAAAA

      8949       8959       8969       8979       8989       8999       9009
AAAAAAATTA GCCAGATGTG GTGGCACACA CCTGTAATCC CAGCTACTTG GGAGGCTGAG GAGGAGAATT

      9019       9029       9039       9049       9059       9069       9079
GCTTGAACCT GGGAGGCGGA GATTGCAGCG AGCCAAGATT GCGCCTCTGC ACTCCAGCCT GGGTGACAGA

      9089       9099       9109       9119       9129       9139       9149
GTGAGACTCC GTCTCAAAAA AAAAAAAAAA AAAGTAGTGG GTGCCTGTGG CCAGGCCACA TCCTAGGGTA
```

FIG. 8B-22

```
      9159       9169       9179       9189       9199       9209       9219
GGGGCTATGG CTGAGCCCTG CCCTCCTGGA GCTCACAGCC AAGTCCACTT CTTCCATCTG AGGCGGGGAA

      9229       9239       9249       9259       9269       9279       9289
GCCAGCCCTG TTCCTGAAAC CCTGCATCAC AAGCCCCTGT GGGAGGCAGT GGGGAGGGGA GGTCCTCCCC

      9299       9309       9319       9329       9339       9349       9359
CACTCAGACC TGACCCACAG GGACCAGTTT AATGTGTCCT TGCCCCAGTG ATGACAGCTG GGGATCTGGG

      9369       9379       9389       9399       9409       9419       9429
GGTGGGGAGT CACCCAGGAC CCGGGCAGTC GCCTTTCCCC AGCTCCTAGG GCTCCCGGCC TTCCCTGCTG

      9439       9449       9459       9469       9479       9489       9499
AAACAGCAAG ACCAGTGGGT TGGCGTGGGA GGCCTGGGCT TCAAACCACC TCTGCTATCA CCTGGCTGTG

      9509       9519       9529       9539       9549       9559       9569
GGTCCCCAGG CAGGACATAC ACACAGTCCC TCTCTGGCCC TCATCCTCCT CAGCTGCAAA GGAAAAGCCA

      9579       9589       9599       9609       9619       9629       9639
AGTGAGACGG GCTCTGGGAC CATGGTGACC AGGCTCTTCC CCTGCTCCCT GGCCCTCGCC AGCTGCCAGG
```

# FIG. 8B-23

```
      9649       9659       9669       9679       9689       9699       9709
CTGAAAAGAA GCCTCAGCTC CCACACCGCC CTCCTCACCG CCCTTCCTCG GGAGTCACTT CCACTGGTGG

      9719       9729       9739       9749       9759       9769       9779
ACCACGGGCC CCCAGCCCTG TGTCGGCCTT GTCTGTCTCA GCTCAACCAC AGTCTGACAC CAGAGCCCAC

      9789       9799       9809       9819       9829       9839       9849
TTCCATCCTC TCTGGTGTGA GGCACAGCGA GGGCAGCATC TGGAGGAGCT CTGCAGCCTC CACACCTACC

      9859       9869       9879       9889       9899       9909       9919
ACGACCTCCC AGGGCTGGGC TCAGGAAAAA CCAGCCACTG CTTTACAGGA CAGGGGGTTG AAGCTGAGCC

      9929       9939       9949       9959       9969       9979       9989
CCGCCTCACA CCCACCCCCA TGCACTCAAA GATTGGATTT TACAGCTACT TGCAATTCAA AATTCAGAAG

      9999      10009      10019      10029      10039      10049      10059
AATAAAAAAT GGGAACATAC AGAACTCTAA AAGATAGACA TCAGAAATTG TTAAGTTAAG CTTTTTCAAA
```

# FIG. 8B-24

```
     10069      10079      10089      10099      10109      10119      10129
AAATCAGCAA TTCCCCAGCG TAGTCAAGGG TGGACACTGC ACGCTCTGGC ATGATGGGAT GGCGACCGGG

     10139      10149      10159      10169      10179      10189      10199
CAAGCTTTCT TCCTCGAGAT GCTCTGCTGC TTGAGAGCTA TTGCTTTGTT AAGATATAAA AAGGGGTTTC

     10209      10219      10229      10239      10249      10259      10269
TTTTTGTCTT TCTGTAAGGT GGACTTCCAG CTTTTGATTG AAAGTCCTAG GGTGATTCTA TTTCTGCTGT

     10279      10289      10299      10309      10319      10329      10339
GATTTATCTG CTGAAAGCTC AGCTGGGGTT GTGCAAGCTA GGGACCCATT CCTGTGTAAT ACAATGTCTG

     10349      10359      10369      10379      10389      10399      10409
CACCAGTGCT AATAAAGTCC TATTCTCTTT TATGAGAAAG AAAAAGACAC CAGTCCTTTA AAGTGCTGCA

     10419      10429      10439      10449      10459      10469
GTATGGCCAG ACGTGGTGGC TCACACCTGC AATCCCAGCA CCTTAGGAGG CCGAGGCAGG AGGATCC
```

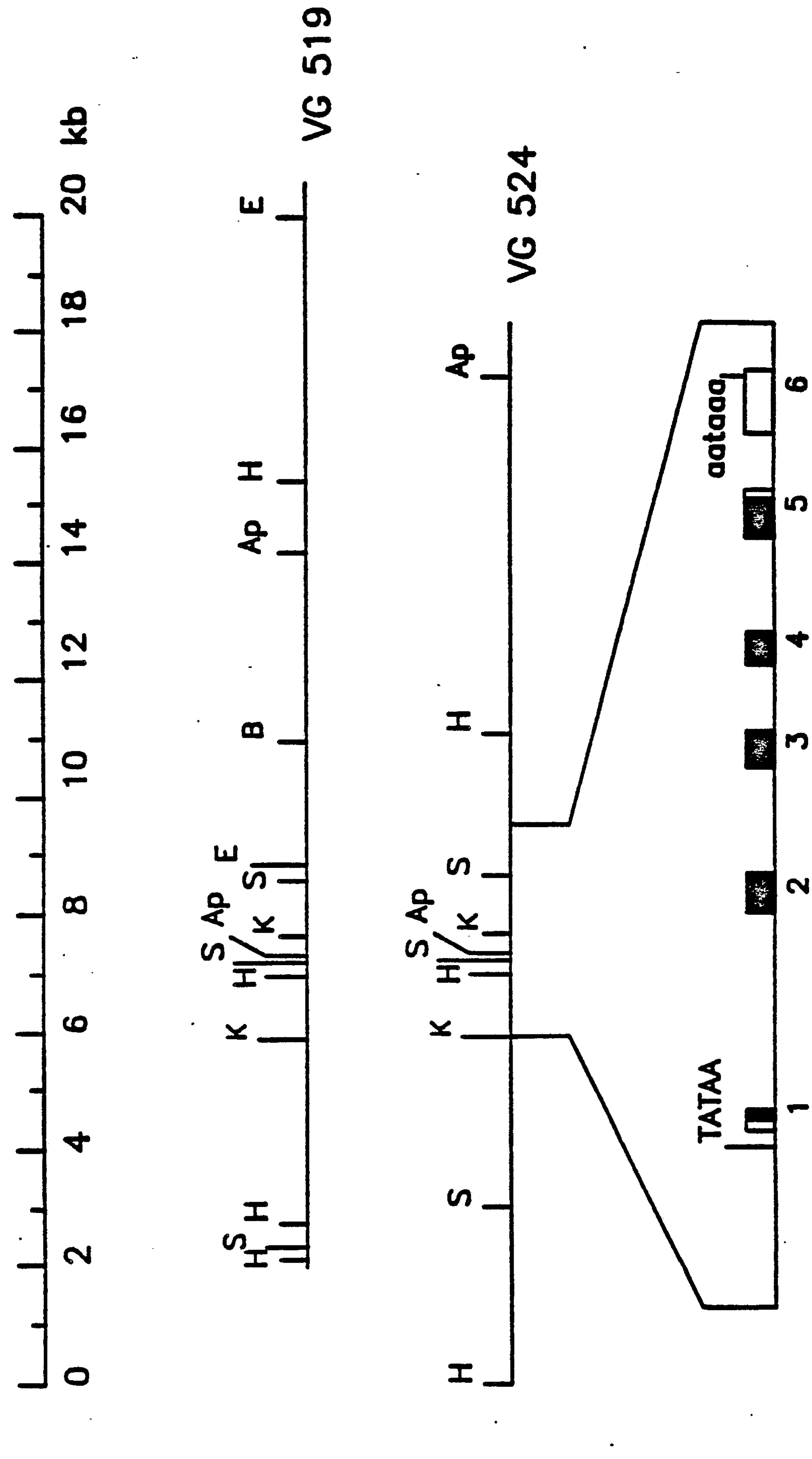
0
2
4
6
8
10
12
14
16
18
20 kb
VG 519
H S H
K
H S Ap K S E
B
Ap H
E
VG 524
H
S
K
H S Ap K
S
H
Ap
TATAA
aataaa
1
2
3
4
5
6

FIG.9

```
GGTACCAGATATGTGGGAGGAGGCAAGGTAAGGGAAAGAGTACTTGAAGTTGGAACTGGTCCTTGCAGGGAAATGCACATTTATGAAACCCC    -499

GAAAACTGATGTCAAAGCACCTCCTGCCTTGGGCAGTCCTCTCAGAGTCTACAGGTGCTGCCTCCAGAACCCTCTTCCTGGAGCGCATCCCT    -407

ATGTATCTAGAAATTCTGCTGGGAAATATGATGGTCAGACCCTTGGCCACCTGAAAGTTCAGGGTGGTAGAAGAAAAAGGAAAGCCACAGGG    -315

CAGCAGGGGCAGGTGCAGCAAGGAAGGCAGGCACGCCAGGAAGACACCCATGGGTAGAAGTGCAGATGGCCCGAGGGCACAGTTTGCTCAAC    -223

TCACCCAGGTTTGCTCTTGCTGGGGCCAAGAGGACTCATGTGCCAGGGCCAAGGGCTCTGGGGGCTCTCACAGGGGGCTTATCTGGGCTTCG    -131

                                                                      *     -          -
GTTCTGGAGGGCCAGGAACAAACAGGCTTCAAAGCAAGGGCTTGGCTGGCACACAGGGGCTTGGTCCTTCACCTCTGTCCCTCTCCTACGGA    -39

                            +1
CACATATAAGACCCTGGTCACACCTGGGAGAGGAGGAGAGGAGAGCATAGCACCTGCAGCAAGATGGATGTGGGCAGCAAAGAGGTCCTG        52
                                                                 MetAspValGlySerLysGluValLeu

              -                         -
ATGGAGAGCCCGCCGGTGAGTGTGGTTGCGTGTGTGTATGTATGTGCGCGCACATGTGTGTGATGGCCCTGCCTCCTCTATCCTCCCTGG    144
MetGluSerProPro

CCTGTTTCCTTATCCAGATCCATTCACTCAACTAACCTAGGACTGTGATAAGTCAGGATGGGGACACCAAGACCACTAAGCCAGGGACCCTT    236
```

FIG.10A

```
                                                  *  A
GGGGAGCTGTTTGTGGCCAAGAGCCACTATAGGGGTCCGTAGAACTGGAGTGCGCGTAGACAGCCCTGAGTCAGAAGCCATGAGAAACTTCA    328

                                                                             *
GAAGTCAGGGGACACTTCTCAGAGAAAAACCACATACGAGCTGGAGCCAGAATAAGGAGGAGCTCGCCCGGTGGAGAAGGAGGAAGGCATTC    420

                                                                          *
CAGGAAGGAGGGAGACTCTGTATCACCGCATGGAGGTGATCACTTGGGGAGAGAGAGGGGCTGACCATGGCTGGGGGAAGCAGCAGGGAGAG    512

                                                  *
ACAGGTGAAGCAGGCTCTCTTGGGTCCCTCAAAACTAGACCCTGCTTCTAAGCTTCTATGTATCTATGGGTTTGTTAGAATCCAGGCCACCT    604

CCTCCAAGAAGCCTTCTCTGATCTCCTCAGCCCTTCCCTGTCCATCCATCGCATCGGCTGTCCAGCCTAGGAGCCGTGGGAGGGTGTTCAGC    696

TTGTATAGGGAGAAGAGGGGACAGCCTCATGACCTCATGCCTGTCTCCTTGCCTGCCCCACCGTGTCAGGACTACTCCGCAGCTCCCCGG      786
                                                                          AspTyrSerAlaAlaProArg

GGCCGATTTGGCATTCCCTGCTGCCCAGTGCACCTGAAACGCCTTCTTATCGTGGTGGTGGTGGTGGTCCTCATCGTCGTGGTGATTGTG      876
GlyArgPheGlyIleProCysCysProValHisLeuLysArgLeuLeuIleValValValValValValLeuIleValValValIleVal

GGAGCCCTGCTCATGGGTCTCCACATGAGCCAGAAACACACGGAGATGGTGAGAGGTGTGGGATGCACAGCAGTGGGCACAGGACATGCC      966
GlyAlaLeuLeuMetGlyLeuHisMetSerGlnLysHisThrGluMet
```

FIG.10B

```
AGACAGAGGGGCTAGGTGGGATGGGCGATAGGAAACTGTCCAAGGGGAGTGGAGGGGAGGAGGCAAGGGGCACAGCTAGAAGGAAAGAGG   1056

CACGAACCAGGCAGCAACCCAGCTCAGGCTTTTCCACAAGGCCCCTGCCCGCGACAGGACAGCCAGCTCCCTCCAGCACCTGGTTCCACT   1146

CAGCCTCCCTGAACTCTTGGGAAAGAGGGAAGCGCATTTGAGTACAGAGGCCTGAGTATGGGGATGGGTACCACTGGCTGAGTAGGAAAG   1236

GGGAAGACCAGGTGGCTCCATGCCTTTCCCCAGGTTCTGGAGATGAGCATTGGGGCGCCGGAAGCCCAGCAACGCCTGGCCCTGAGTGAG   1326
                                        ValLeuGluMetSerIleGlyAlaProGluAlaGlnGlnArgLeuAlaLeuSerGlu

                                                                                    -
CACCTGGTTACCACTGCCACCTTCTCCATCGGCTCCACTGGCCTCGTGGTGTATGACTACCAGCAGGTGGGTATGCCAGACCTCCTGACC   1416
HisLeuValThrThrAlaThrPheSerIleGlySerThrGlyLeuValValTyrAspTyrGlnGln

                **                                      *    *                 -        -    -
TGGACCAATGACAACTGGGCTCTGCTAGAGCGCCCAGCTGGCCACTTTCATTCCACATCCATCTCTCCTCTCTCAGACTTTTTGCTGAGC   1506

 -              -A                                                                 -     -
CCAGATTCTAGTAGTCTCCCGTGCCCAACCTAGAGGGAGGTGGCTAAGGACCTGGGTCAGGGAGAGAGCAGGGCAGGACCCCGAATGATC   1596

TCCAGCATTCTGTGCCTAGCTGCTGATCGCCTACAAGCCAGCCCCTGGCACCTGCTGCTACATCATGAAGATAGCTCCAGAGAGCATCCCC  1687
                       LeuLeuIleAlaTyrLysProAlaProGlyThrCysCysTyrIleMetLysIleAlaProGluSerIlePro
```

FIG.10C

```
AGTCTTGAGGCTCTCACTAGAAAAGTCCACAACTTCCAGGTGTGTGTGTGTGGGTGAAAAGAGTGGGCTGTCTCCCTCCCAGGCTGCTGG   1777
SerLeuGluAlaLeuAsnArgLysValHisAsnPheGln

  A          *
AGGAGTGTCCGAATGGTGGCTATTTGTCACCTGTAAAGCACTGTTCCTCATTGGCTGCCAGCTGACTGCCCCTCTCCTATTCCCCTGCAC   1867

                                                                                         T
GACTCCTTTCCTTCCCACCCCACTGCCAAGCTGCTGGGCTCAGCTGAGTCCACTCACTACCTGGTGGCTTCTGACTCTAGCACAGCCCCT   1957

CTTTACTGATGAGAAAACTGAGGCTCAGAGAGATTGCCTGATATACCTGAAGTCCCACAATAAGGGCTGCACATGGGATAGAAACTCACT   2047

    G
TCCTACATTCCAGATGGAATGCTCTCTGCAGGCCAAGCCCGCAGTGCCTACGTCTAAGCTGGGCCAGGCAGAGGGGCGAGATGCAGGCTCA   2138
            MetGluCysSerLeuGlnAlaLysProAlaValProThrSerLysLeuGlyGlnAlaGluGlyArgAspAlaGlySer

GCACCCTCCGGAGGGGACCCGGCCTTCCTGGGCATGGCCGTGAACACCCTGTGTGGCGAGGTGCCGCTCTACTACATCTAGGACGCCTCC   2228
AlaProSerGlyGlyAspProAlaPheLeuGlyMetAlaValAsnThrLeuCysGlyGluValProLeuTyrTyrIleEnd

                                                    C      A*  T  CG          -
GGTGAGCAGGTGTGATCCCAGGGCCCCTGATCAGCAGCGGAGGAGCGCTGGCCACCTGCCCGGGCTGTGGAGGAGGCTCGCTGACCAGGC   2318
```

FIG.10D

```
C T  G *                                                                        *
TGGGGCGTCCACTGAAGCGGGGTCATCCAGGCAACTCGGGGGAGGGGAAGCTCACAGACCGGTACTTCCCACTCCCCTGAATTCTCTCTG    2408

                          C
TCCATCCTCAACATTCCTTTGCTTCATAGGGTCAGTGGAAGCCCCAACGGAAAGGAAACGCCCCGGGCAAAGGGTCTTTTGCAGCTTTTG    2498

                                          G
CAGACGGGCAAGAAGCTGCTTCTGCCCACACCGCAGGGACAAACCCTGGAGAAATGGGAGCTTGGGGAGAGGATGGGAGTGGGCAGAGGT    2588

   G                                                -
GGCACCCAGGGGCCCGGGAACTCCTGCCACAACAGAATAAAGCAGCCTGATTTGAAAAGCAAAGGGTCTGCTTCTGTCTTCCTGCAGGGC    2678

                        T              **         CG        T
GCAGTCCTCGCTGGCGGGGCCGGCCAAGAAGGGAAGGGCCTTGGGAGAGCAAAGTGGGGTTTCCATTCGCCCTCTGTCCCAGGGCGCTGG    2768

CACTGTCCACCTCGGCGGGGAGAGGGGCTCGCAGGGAGCATCCACGGGCTTT                                          2820
```

FIG.10E

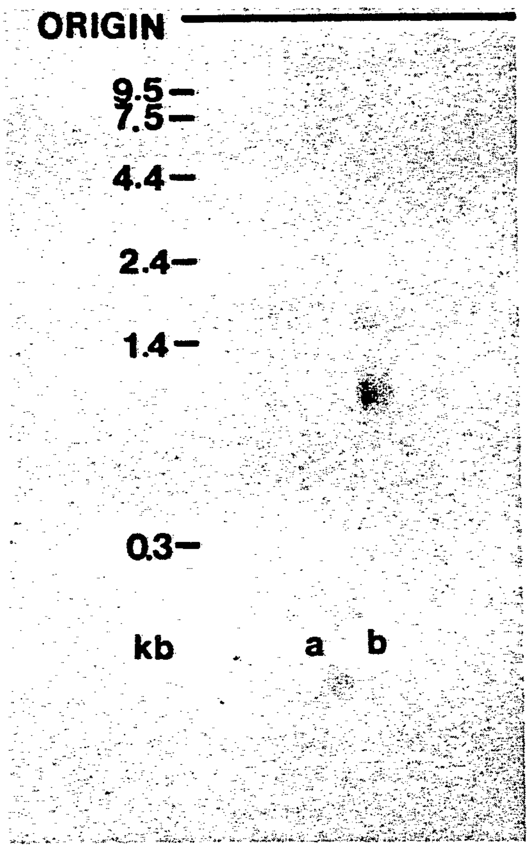


FIG.11

POOR QUALITY

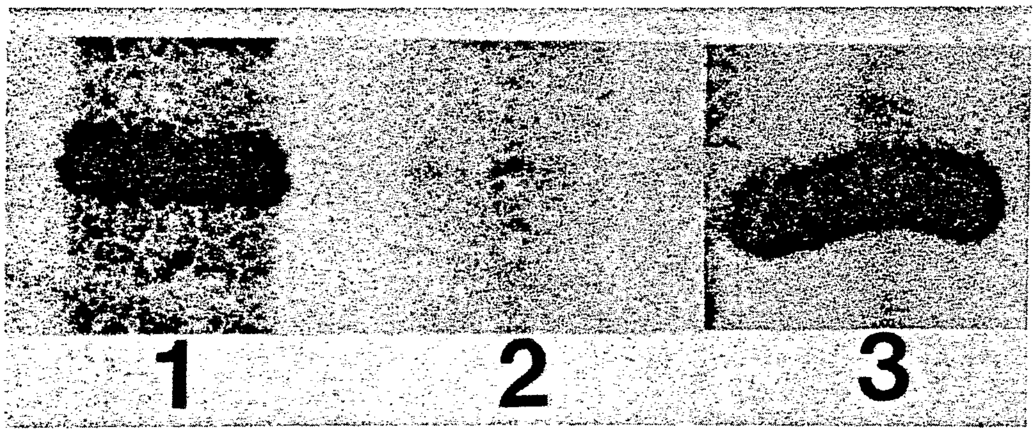


FIG.12

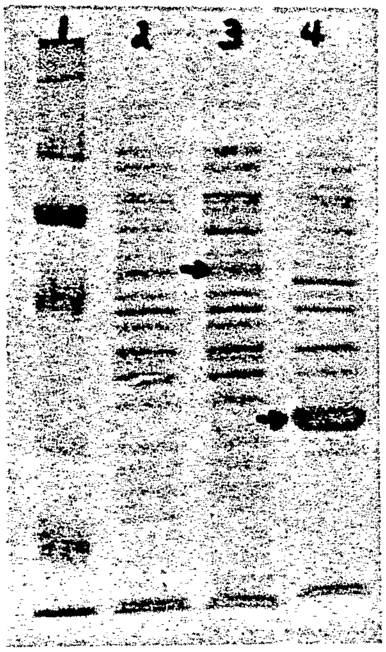


FIG.13

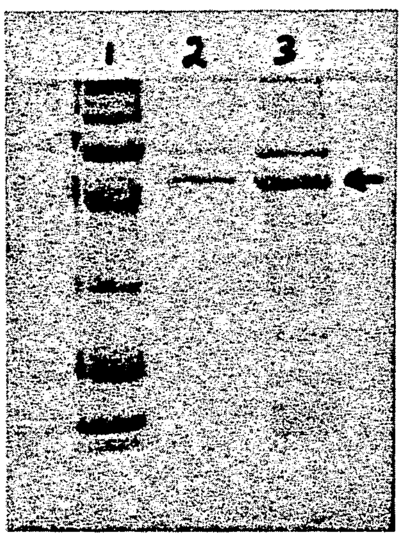


FIG.14

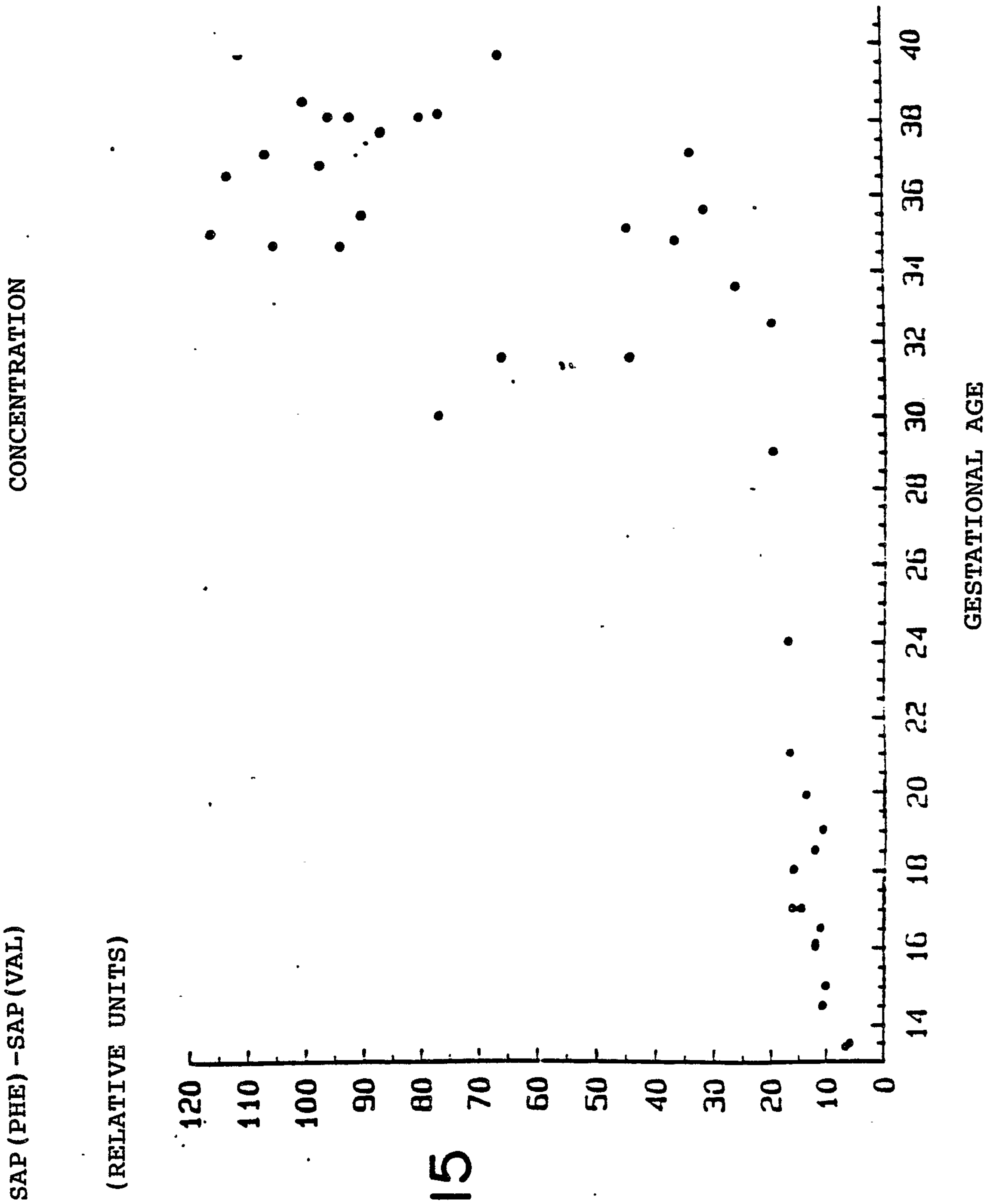

SAP (PHE) -SAP (VAL)
CONCENTRATION
(RELATIVE UNITS)
120
110
100
90
80
70
60
50
40
30
20
10
0
14
16
18
20
22
24
26
28
30
32
34
36
38
40
GESTATIONAL AGE


FIG.15

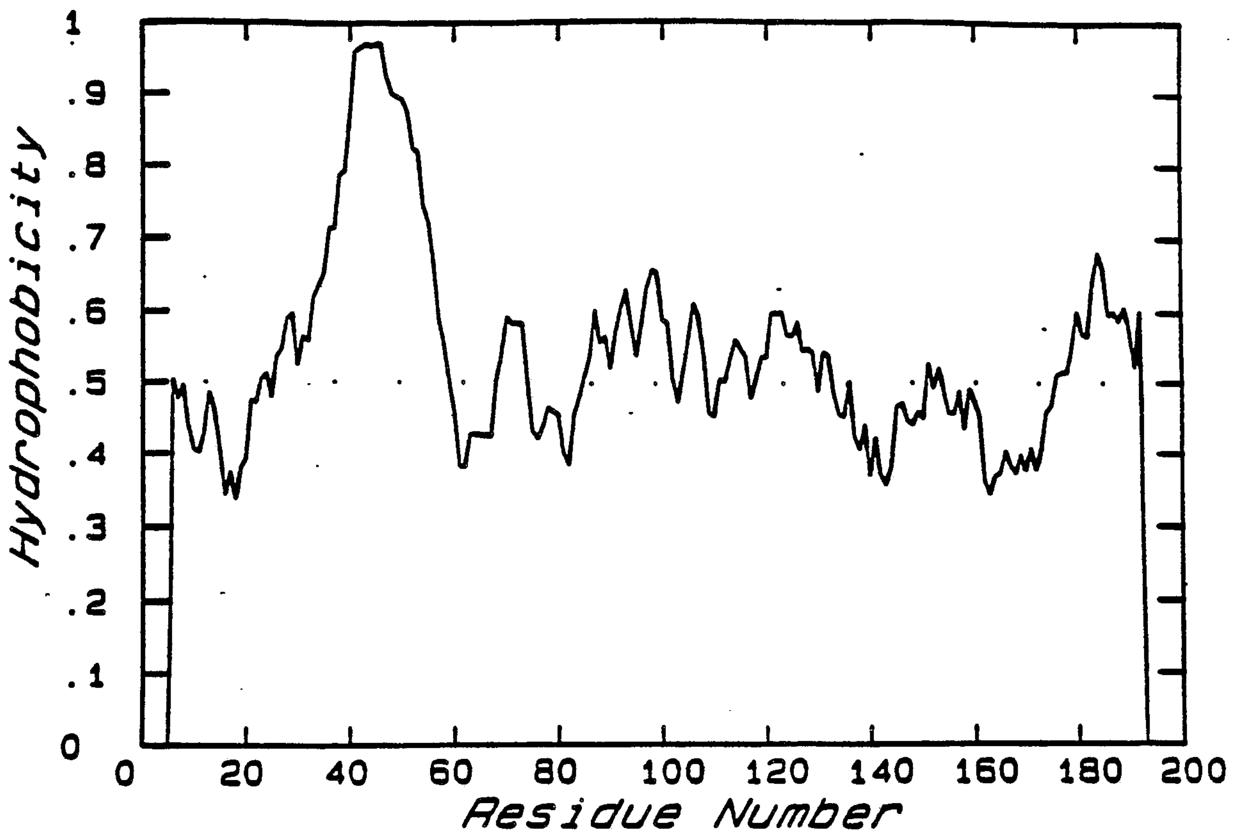


FIG.16

**POOR QUALITY**